# EUROPEAN PATENT SPECIFICATION

(11) **EP 2 943 222 B1**
(45) Date of publication and mention of the grant of the patent: **26.08.2020**
(21) Application number: 14701930.1
(22) Date of filing: 14.01.2014
(51) Int. Cl.: A61P 1/00, A61P 1/02, A61P 1/04, A61P 1/16, A61P 1/18, A61K 9/14, A61K 31/12, A61K 31/167, A61K 31/137

(54) **SOLID SOLUTION COMPOSITIONS AND USE IN SEVERE PAIN**
FESTE LÖSUNGSZUSAMMENSETZUNGEN UND VERWENDUNG BEI SCHWEREN SCHMERZEN
COMPOSITIONS DE SOLUTIONS SOLIDES ET UTILISATION DANS LA DOULEUR AIGUË

(30) Priority: 14.01.2013 US 201361752356 P; 04.02.2013 US 201361752309 P
(43) Date of publication of application: 18.11.2015
(73) Proprietor: InFirst Healthcare Limited, London EC2Y 8AD (GB)
(72) Inventor: BANNISTER, Robin Mark, London Greater London EC2Y 8AD (GB); BREW, John, London Greater London EC2Y 8AD (GB); REILEY, Richard Robert, London Greater London EC2Y 8AD (GB); CAPARRÓS-WANDERLEY, Wilson, London Greater London EC2Y 8AD (GB)
(74) Representative: Clements, Andrew Russell Niel
(86) International application number: PCT/EP2014/050638
(87) International publication number: WO 2014/108574

(56) References cited:
- WO-A1-92/09272
- WO-A2-00/67728
- CN-A- 102 793 628
- US-A- 3 800 038

## Description

This application claims priority from U.S. Non-Provisional Patent Application 13/365,824, filed February 3, 2012, a continuation-in-part application that claims priority to patent application PCT/GB2011/052115, filed October 31, 2011, an international patent application that claims priority to GB 1018289.7, filed October 29, 2010, and claims priority to patent application US 13/365,828, filed February 3, 2012, and claims priority to GB 1113730.4, filed August 10, 2011, GB 1113729.6, filed August 10, 2011, GB 1113728.8, filed August 10, 2011, and GB 1101937.9, filed February 4, 2011 and this application claims priority from U.S. Provisional Patent Application 61/752,309, filed January 14, 2013, and U.S. Provisional Patent Application 61/752,356, filed January 14, 2013.

Pain is a subjective and very complex perception that signals to an individual that tissue damage has occurred or may be occurring. Pain may be transitory, lasting only until the noxious stimulus causing the pain is removed or the underling damage or pathology has healed or may last beyond the healing of an injury, continuing for a period of several months or longer. A pain response has both physiological as well as psychological components. A pain response evokes a wide range of sensations that may be described as a dull, aching sensation, a sharp stabbing sensation, a hot, cold, or icy-hot sensation, a tingling or itchy sensation or a numbness. Pain can be felt in one area of the body, such as your back, abdomen or chest or throughout the body, such as when all the muscles in a body ache from the flu.

Severe pain is the most common reason for an individual to consult a healthcare provider in the United States. It is a major symptom in many medical conditions, and can significantly interfere with a person's quality of life and general functioning. As such, severe pain is a significant and costly healthcare problem.

Current therapies for treating severe pain are limited, often involving the administration of multiple medications with the understanding that pain relief will not be complete and the quality of life may not be restored. These therapies may require frequent dosing, can be associated with undesirable systemic side effects, and typically provide unsatisfactory relief. Therefore, there remains a need for a therapeutic option developed specifically for severe pain that provides sustained relief while minimizing the potential for systemic side effects and drug-drug interactions.

WO 92/09272 describes controlled delivery of biologically active agents such as pharmaceuticals to body sites using a composition of a monoglyceride and a vegetable oil. Upon contact with an aqueous liquid, the composition forms a reverse hexagonal liquid crystalline phase matrix which releases the active agent in a controlled fashion.

WO 2000/067728 describes lipid particles which do or do not carry active agents and comprise a mixed matrix consisting of solid and liquid lipid (so-called solid/liquid particles). The particles have a disordered structure (semicrystalline, mostly non-crystalline to amorphous) in the semisolid to solid condition of matter. WO 2000/067728 also describes a method for producing these dispersions and a method for producing highly concentrated lipid particle dispersions with a lipid content of 30 % to 95 % or a solids content of 30 % to 95 % (lipid and stabiliser).

The present specification discloses solid solution pharmaceutical compositions and methods for treating an individual suffering from a severe pain condition. The pharmaceutical compositions disclosed herein are formulated in a manner that essentially produces a lipid-adjuvant delivery system that enables a therapeutic compound having anti-pain activity to be delivered in a manner that more effectively inhibits a pain response. The end result is an improved treatment for a severe pain condition.

The Invention is set out in the appended set of claims. Aspects of the present invention disclose, a solid solution pharmaceutical composition comprising: a) one or more therapeutic compounds having an anti-pain activity comprising 5% to 55% by weight of the pharmaceutical composition, wherein when the one or more therapeutic compounds is a therapeutic compound having a free acid or base with a logP of 2.2 to 3.0, one or more stabilizing agents comprising a liquid glycol polymer are present, wherein when the one or more therapeutic compounds is a therapeutic compound salt with a logP of 2.2 or less, one or more neutralizing agents are present; b) one or more lipids that are solid at 20 °C comprising 30% to 75% by weight of the pharmaceutical composition wherein the one or more lipids have a melting point of about 40°C to about 50°C, and wherein the lipids comprise a triglyceride with one saturated or unsaturated fatty acid having a carbon length of C₁₂-C₂₄, two saturated or unsaturated fatty acids each having a carbon length of C₁₂-C₂₄, or three saturated or unsaturated fatty acids each having a carbon length of C₁₂-C₂₄; c) one or more lipids that are liquid at 20°C comprising 1% to 20% by weight of the pharmaceutical composition, wherein the solid solution pharmaceutical composition has a melting point of 30°C or higher. The solid solution pharmaceutical composition disclosed herein may further comprise one or more stabilizing agents, one or more neutralizing agents, or any combination thereof.

Other aspects of the present specification, which do not form part of the claimed invention, disclose, in part, a method of preparing a solid solution pharmaceutical composition disclosed herein. A method disclosed herein, which is not part of the claimed invention, comprises the steps of a) contacting a therapeutic compound disclosed herein with one or more room temperature liquid lipids under conditions which allow the therapeutic compound to dissolve in the lipids; and b) contacting the compound/lipid solution with one or more room temperature solid lipids under conditions which allow the formation of a solid solution composition. In aspects of this method, heat is applied to dissolve the therapeutic compound into the one or more room temperature liquid lipids to create a solution. In other aspects of this method, step (a) comprises contacting a therapeutic compound disclosed herein with one or more room temperature liquid lipids and/or one or more stabilizing agents, and/or one or more neutralizing agents under conditions which allow the therapeutic compound to dissolve in the lipids.

Other aspects of the present specification disclose pharmaceutical compositions of the invention for treating an individual with a severe pain condition. Treatment with the pharmaceutical compositions of the invention comprises the step of administering to the individual in need thereof the pharmaceutical composition, wherein administration results in a reduction in a symptom associated with the severe pain condition, thereby treating the individual.

Also disclosed herein is a solid solution pharmaceutical composition of the invention for use in the treatment of a severe pain.

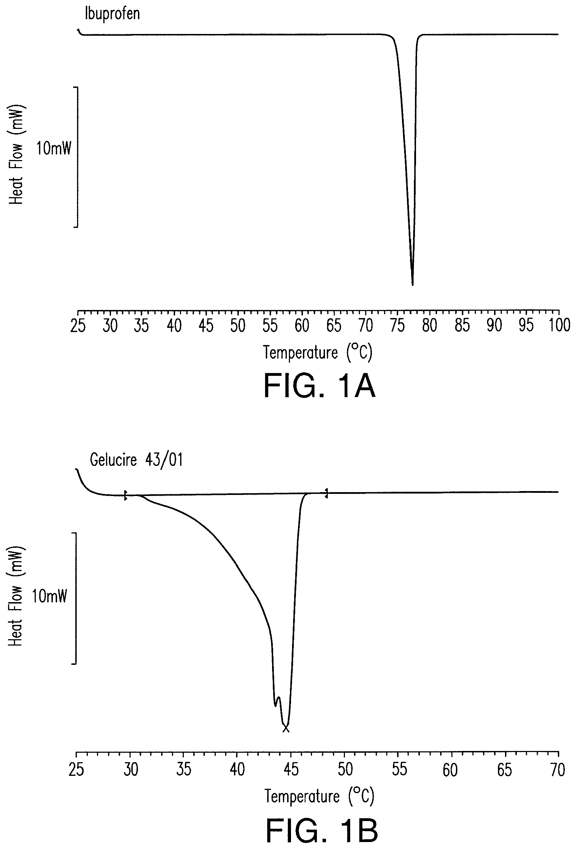

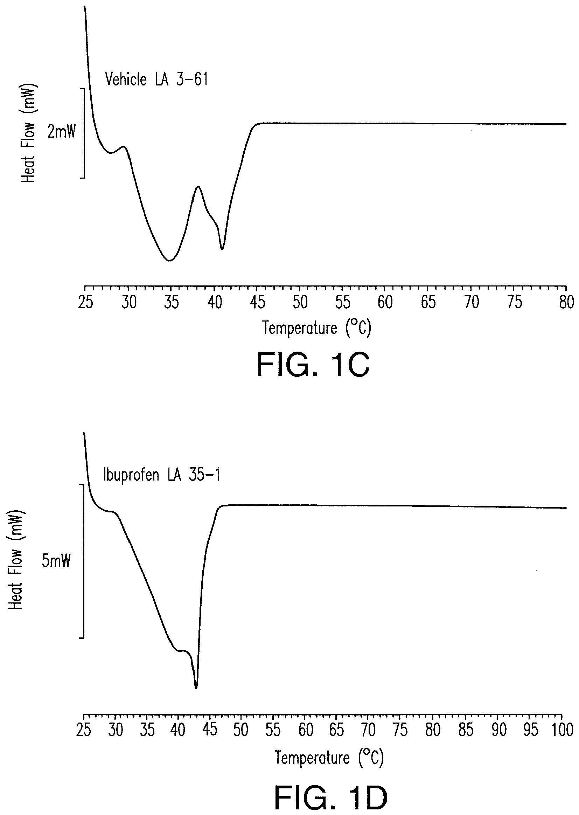

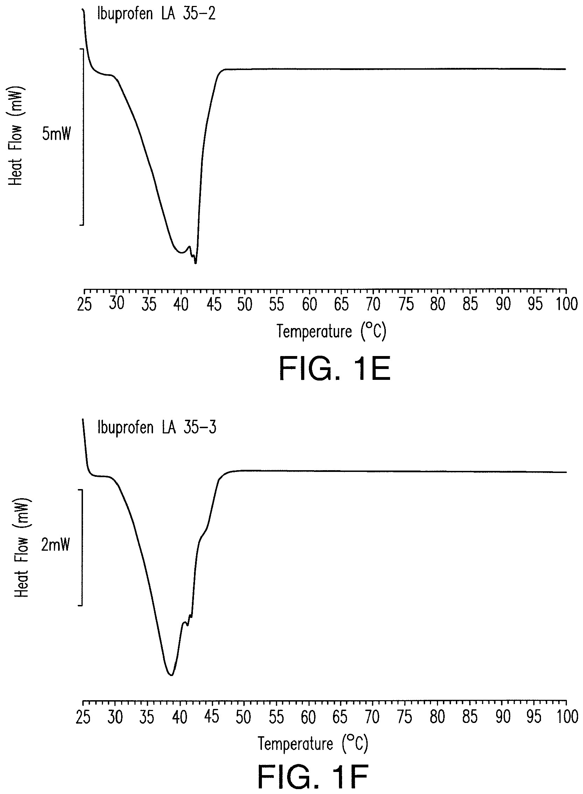

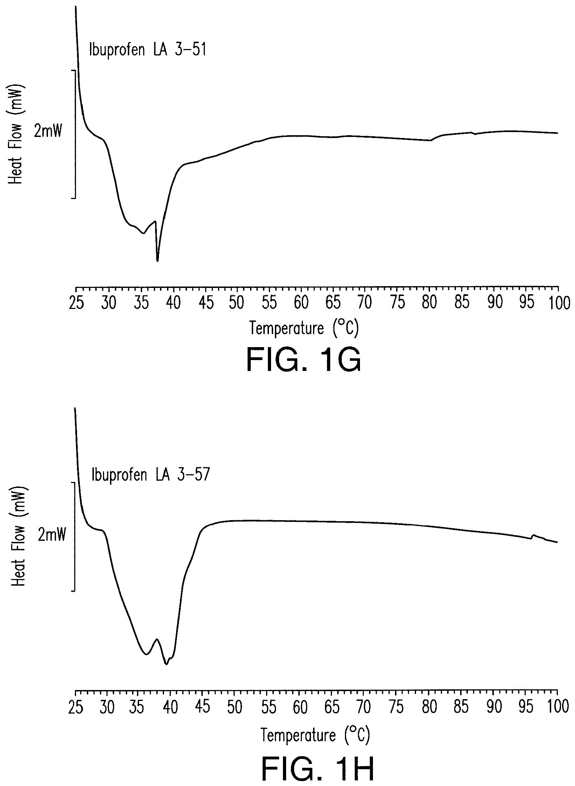

**FIG. 1** shows a differential scanning calorimetry (DSC) graph of solid solution compositions disclosed herein comprising ibuprofen. **FIG. 1A** is a DSC graph of ibuprofen alone exhibiting a melting point range of 75°C to 78°C; **FIG. 1B** is a DSC graph of GELUCIE® 43/01 alone exhibiting a melting point range of 41°C to 45°C; **FIG. 1C** is a DSC graph of a vehicle comprising GELUCIE® 43/01, MAISINE® 35-1, and PEG 400 exhibiting a melting point ranges of 32°C to 38°C and 41°C to 45°C; **FIG. 1D** is a DSC graph of Ibuprofen composition LA 35-1 exhibiting a melting point range of 32°C to 44°C; **FIG. 1E** is a DSC graph of Ibuprofen composition LA 35-2 exhibiting a melting point range of 32°C to 43°C; **FIG. 1F** is a DSC graph of Ibuprofen composition LA 35-1 exhibiting a melting point range of 32°C to 42°C; **FIG. 1G** is a DSC graph of Ibuprofen composition LA 35-1 exhibiting a melting point range of 32°C to 38°C; **FIG. 1H** is a DSC graph of Ibuprofen composition LA 35-1 exhibiting a melting point range of 32°C to 42°C.
**FIG. 2** shows a DSC graph of Artemether composition LA 2-15-1 exhibiting a melting point range of 35°C to 40°C.
**FIG. 3** shows a DSC graph of Aspirin composition LA 3-86-3 exhibiting a melting point range of 35°C to 40°C.
**FIG. 4** shows a DSC graph of Dantrolene composition LA 3-104-2 exhibiting a melting point range of 34°C to 39°C.
**FIG. 5** shows a DSC graph of Diclofenac composition LA 3-103 exhibiting a melting point range of 35°C to 40°C.
**FIG. 6** shows a DSC graph of Fenofibrate composition LA 2-19 exhibiting a melting point range of 34°C to 39°C.
**FIG. 7** shows a DSC graph of Lidocaine composition LA 3-101-2 exhibiting a melting point range of 34°C to 40°C.
**FIG. 8** shows a DSC graph of Nabumetone composition LA 3-105-1 exhibiting a melting point range of 35°C to 40°C.
**FIG. 9** shows a DSC graph of Naproxen composition LA 1-23-5 exhibiting a melting point range of 30°C to 39°C.
**FIG. 10** shows a DSC graph of Salbutamol composition LA 1 exhibiting a melting point range of 32°C to 40°C.
**FIG. 11** shows a DSC graph of Salmeterol composition LA 1-23-7 exhibiting a melting point range of 34°C to 43°C.
**FIG. 12** shows a DSC graph of Simvastatin composition LA 3-83-3 exhibiting a melting point range of 32°C to 43°C.
**FIG. 13** shows a DSC graph of Telmisartan composition LA 1 exhibiting a melting point range of 34°C to 43°C.

The present specification discloses solid solution compositions useful to formulate a wide variety of therapeutic compounds having an anti-pain activity. Solid solution compositions are crystalline solids comprising a matrix of a solvent material (which may be solid at normal temperatures) and solutes where the molecules in the solid solution are arranged in a random fashion and not in an ordered alignment. The solid solution pharmaceutical compositions disclosed herein act as a delivery system that enable a therapeutic compound disclosed herein to be more effectively delivered or targeted to a cell type, tissue, organ, or region of the body in a manner that more effectively inhibits a pain response. This inhibition results in an improved treatment of a severe pain.

For example, a pharmaceutical composition disclosed herein may facilitate the delivery of a therapeutic compound disclosed herein into macrophages. Macrophages exist at the crossroads of two fundamental pathways being the principle cells in the immune system and also lipid metabolism. With regards to the immune system, most pathogens have a lipid-containing surface component that macrophage recognize and then engulf the pathogen. One possible mechanism that achieves this selective biodistribution is that the pharmaceutical compositions disclosed herein may be designed to take advantage of the activity of chylomicrons. Chylomicrons are relatively large lipoprotein particles having a diameter of 75 nm to 1,200 nm. Comprising triglycerides (85-92%), phospholipids (6-12%), cholesterol (1-3%) and apolipoproteins (1-2%), chylomicrons transport dietary lipids from the intestines to other locations in the body. Chylomicrons are one of the five major groups of lipoproteins, the others being VLDL, IDL, low-density lipoproteins (LDL), high-density lipoproteins (HDL), that enable fats and cholesterol to move within the water-based solution of the bloodstream.

During digestion, fatty acids and cholesterol undergo processing in the gastrointestinal tract by the action of pancreatic juices including lipases and emulsification with bile salts to generate micelles. These micelles allow the absorption of lipid as free fatty acids by the absorptive cells of the small intestine, known as enterocytes. Once in the enterocytes, triglycerides and cholesterol are assembled into nascent chylomicrons. Nascent chylomicrons are primarily composed of triglycerides (85%) and contain some cholesterol and cholesteryl esters. The main apolipoprotein component is apolipoprotein B-48 (APOB48). These nascent chylomicrons are released by exocytosis from enterocytes into lacteals, lymphatic vessels originating in the villi of the small intestine, and are then secreted into the bloodstream at the thoracic duct's connection with the left subclavian vein.

While circulating in lymph and blood, chylomicrons exchange components with HDL. The HDL donates apolipoprotein C-II (APOC2) and apolipoprotein E (APOE) to the nascent chylomicron and thus converts it to a mature chylomicron (often referred to simply as "chylomicron"). APOC2 is the cofactor for lipoprotein lipase (LPL) activity. Once triglyceride stores are distributed, the chylomicron returns APOC2 to the HDL (but keeps APOE), and, thus, becomes a chylomicron remnant, now only 30-50 nm. APOB48 and APOE are important to identify the chylomicron remnant in the liver for endocytosis and breakdown into lipoproteins (VLDL, LDL and HDL). These lipoproteins are processed and stored by competent cells, including, *e.g.,* hepatocytes, adipocytes and macrophages. Thus, without wishing to be limited by any theory, upon oral administration, a pharmaceutical composition disclosed herein can be processed into micelles while in the gastrointestinal tract, absorbed by enterocytes and assembled into nascent chylomicrons, remain associated with chylomicron remnants taken up by the liver, and ultimately loaded into macrophages which are present in inflamed tissues.

As another example, a pharmaceutical composition disclosed herein may facilitate the delivery of a therapeutic compound disclosed herein into dentritic cells. One possible mechanism to achieve selective biodistribution of the pharmaceutical compositions disclosed herein may be to take advantage of the endocytotic/phagocytotic activity of dentritic cells. Dendritic cells are immune cells forming part of the mammalian immune system. The main function of dendritic cells is to process antigen material and present it on the surface to other cells of the immune system. Thus, dendritic cells function as antigen-presenting cells that act as messengers between innate and adaptive immunity. Dendritic cells are present in tissues in contact with the external environment, such as, *e.g.,* the skin (where there is a specialized dendritic cell type called Langerhans cells) and the inner lining of the nose, lungs, stomach and intestines. These cells can also be found in an immature state in the blood. Once activated, they migrate to the lymph nodes where they interact with T cells and B cells to initiate and shape the adaptive immune response. Dendritic cells are known to endocytose and phagocytose lipid particles as part of their environmental monitoring and antigen presentation processes. Without wishing to be limited by any theory, upon topical or inhalatory administration, a pharmaceutical composition disclosed herein can penetrate into the skin or inner lining of the nose, lungs, stomach and intestines, be endocytosed/phagocytosed by dendritic cells, and ultimately loaded into T cells and/or B cells which are present in inflamed tissues.

In addition to the targeted delivery of the therapeutic compound disclosed herein, a solid solution pharmaceutical composition disclosed herein take advantage of the different melting point temperatures of the various lipids used. By controlling the types and amounts of the lipids added, a pharmaceutical composition disclosed herein can be made that is substantially solid at room temperature, but melts when it reaches body temperature, such as, *e.g.,* after being ingested. The resulting melted composition readily forms micelles which are absorbed by the intestine, assembled into chylomicrons, and ultimately absorbed by macrophages or taken up by dentritic cells as described above.

Aspects of the present specification disclose, in part, a solid solution composition. A solid solution composition disclosed herein is generally administered as a pharmaceutical acceptable composition. As used herein, the term "pharmaceutically acceptable" refers any molecular entity or composition that does not produce an adverse, allergic or other untoward or unwanted reaction when administered to an individual. As used herein, the term "pharmaceutically acceptable composition" is synonymous with "pharmaceutical composition" and means a therapeutically effective concentration of an active ingredient, such as, e.g., any of the therapeutic compounds disclosed herein. A pharmaceutical composition disclosed herein is useful for medical and veterinary applications. A pharmaceutical composition may be administered to an individual alone, or in combination with other supplementary active ingredients, agents, drugs or hormones.

To influence the pharmacodynamics of a therapeutic compound disclosed herein three features of the solid solution composition must be present. First, at least one lipid used in the solid solution composition must be made up of at least one fatty acid in which the carbon chain length is above 12 and below 24 and therefore suitable for absorption through the enterocyte pathways. Fatty acids below this C₁₂-C₂₄ length do not form a lipid-drug matrix, and thus the drug is taken up by the body by the normal absorption process. Fatty acids above this C₁₂-C₂₄ length, although forming lipid-drug matrices, cannot be absorbed and the drug leaches out of the solid solution composition and is eliminated by the body via the gastrointestinal tract.

Second, the therapeutic compound itself should have a lipophilicity that allows it to form a solid solution matric with the C₁₂-C₂₄ lipid. As discussed below, this lipophilicity can be inherent to the therapeutic compound (lipid-soluble drug formulations), or certain additives may be used that facilitate a wider range of lipid soluble drugs in the matrix (free acid/free base drug formulations, salt drug formulations, and combination drug formulations).

Third, the therapeutic compound itself should influence the biology of certain cell types that are contacted by the lipid-adjuvant nature of a solid solution composition that ultimately circulate in the body. Such constructs include chylomicron, LDL particles and HDL particles. The cell types contacted may include macrophages, dendritic cells and adipose cells and cancer cells. Tissues that have a high surface lipid content may also be preferentially targeted. These include nerve tissues and the brain.

The present specification discloses four general types of solid solution compositions, namely lipid-soluble drug formulations, free acid/free base drug formulations, salt drug formulations, and combination drug formulations. Solid solution compositions formulated using a lipid-soluble drug formulation only require a lipid component to formulate a therapeutic compound disclosed herein into a solid solution composition. Without wishing to be limited by a theory, lipid-soluble drugs can typically be dissolved in a lipid under heat. Upon cooling it is believed that the lipid component and drug form lipid-drug matrices organized in a manner where the lipids encase the drug. Since only hydrophobic interactions are present, there is no organized alignment of these lipid-drug matrices resulting in a solid solution composition (i.e., there is no crystallization into a classic solid form).

Generally, therapeutic compounds having a logP of 3.0 or greater are useful in a lipid-soluble drug formulation. Non-limiting examples include an Artemisinin like Arteether, Artemether, Artemisinin, Artesunate, and Dihydroartemisinin; a Fibrate like Bezafibrate, Ciprofibrate, Clofibrate, Fenofibrate, and Gemfibrozil; and a Statin like Atorvastatin, Fluvastatin, Lovastatin, Pitavastatin, Pravastatin, Rosuvastatin, and Simvastatin.

A lipid-soluble drug formulation does not use or require a surfactant. In addition, a lipid-soluble drug formulation does not use or require non-lipid based solvent.

Solid solution compositions formulated using a free acid/free base drug formulation require a stabilizing agent in addition to a lipid component to formulate a therapeutic compound disclosed herein into a solid solution composition. A therapeutic compound having a free acid or free base can dissolve in a lipid under heat, but upon cooling to room temperature will crystalize to form a classic solid composition. This occurs because the thermodynamic properties of these mixtures favor the lower energy solid phase. In order to produce a solid solution composition, a stability agent must be added to stabilize the drug and prevent its transition into a classic solid phase upon cooling. Without wishing to be limited by a theory, it is believed that the stability agent coats lipid-drug matrices upon their formation. This coating impedes interactions between matrices thereby preventing the alignments necessary to form a crystalline matrix of a solid phase composition. As such, the transition to a solid phase does not occur and a solid solution composition is formed. Thus, a stability agents is a compound that provide a barrier to the thermodynamic transition to a classic solid phase or prolongs this transition to such an extent that it does not occur. Examples of stability agents include liquid polyethylene glycols, isosorbide dimethyl ether, diethylene glycol monoethyl ether (2-(2-ethoxyethoxy)ethanol), monohydrate alcohols

Generally, therapeutic compounds having a logP of 2.2 to 3.0 are useful in a free acid/free base drug formulation. Non-limiting examples include a non-steroidal anti-inflammatory drug (NSAID) and an ester of aminobenzoic acid. A NSAID includes a salicylate derivative NSAID, a p-amino phenol derivative NSAID, a propionic acid derivative NSAID, an acetic acid derivative NSAID, an enolic acid (Oxicam) derivative NSAID, a fenamic acid derivative NSAID, a non-selective cyclo-oxygenase (COX) inhibitor, a selective cyclooxygenase 1 (COX 1) inhibitor, a selective cyclooxygenase 2 (COX 2) inhibitor. An ester of aminobenzoic acid includes Amylocaine, Benzocaine, Butacaine, Butamben, Chloroprocaine, Dimethocaine, Lidocaine, Meprylcaine, Metabutethamine, Metabutoxycaine, Orthocaine, Prilocaine, Propoxycaine, Procaine (Novocaine), Proxymetacaine, Risocaine, and Tetracaine.

Solid solution compositions formulated using a salt drug formulation require a neutralizing agent in addition to a lipid component to formulate a therapeutic compound disclosed herein into a solid solution composition. A therapeutic compound salt can dissolve in a lipid under heat, but upon cooling to room temperature will crystalize to form a classic solid composition. This occurs because the thermodynamic properties of these mixtures favor the lower energy solid phase. In order to produce a solid solution composition, a neutralizing agent must be added to neutralize a therapeutic compound salt and prevent its transition into a classic solid phase upon cooling. Without wishing to be limited by a theory, it is believed that the neutralizing agent eliminates that charges presence in the salt drugs. This neutralization impedes the ionic interactions between matrices thereby preventing the alignments necessary to form a crystalline matrix of a solid phase composition. As such, the transition to a solid phase does not occur and a solid solution composition is formed. Thus, a neutralizing agent is a compound that provide a barrier to the thermodynamic transition to a classic solid phase or prolongs this transition to such an extent that it does not occur.

Neutralizing agents include fatty acids for base-salt drugs and triethanolamine for acid-salt drugs. The degree of neutralization depends on the amount of neutralizing agent added to the formulation. For complete neutralization, one molar equivalent of neutralizing agent is added to the formulation. For partial neutralization, less than one molar equivalent is added. Partial neutralization is advantageous in producing a sustained release formulation. Upon administration, a portion of the drug is immediately made available to the body (instant bioavailability) while the bioavailability of another portion is delayed until the neutralized by the neutralizing agent. A neutralizing agent may also be added in an excessive amount, i.e., more than one molar equivalent. Besides neutralizing the salt-drug, excessive amounts of neutralizing agent can also enable adjustments to the melting point of the solid solution composition.

Generally, therapeutic compounds having a logP of 2.2 or less are useful in a salt drug formulation. Non-limiting examples include ryanodine receptor antagonists like Azumolene and Dantrolene; and Angiotensin II receptor antagonists like Azilsartan, Candesartan, Eprosartan, Irbesartan, Losartan, Olmesartan, Telmisartan, and Valsartan.

Solid solution compositions may also comprises different combinations of lipid-soluble drugs, free-acid/free-base drugs, and salt drugs. Depending of the drugs used, such formulations, besides the lipid component and the drug, can also include a stabilizing agent, a neutralizing agent, or both.

Aspects of the present specification disclose, in part, a therapeutic compound. A therapeutic compound is a compound that provides pharmacological activity or other direct effect in the diagnosis, cure, mitigation, treatment, or prevention of disease, or to affect the structure or any function of the body of man or animals. A therapeutic compound disclosed herein may be used in the form of a pharmaceutically acceptable salt, solvate, or solvate of a salt, e.g. the hydrochloride. Additionally, therapeutic compound disclosed herein may be provided as racemates, or as individual enantiomers, including the R- or S-enantiomer. Thus, the therapeutic compound disclosed herein may comprise a R-enantiomer only, a S-enantiomer only, or a combination of both a R-enantiomer and a S-enantiomer of a therapeutic compound. A therapeutic compound disclosed herein may have anti-inflammatory activity.

The therapeutic compound disclosed herein has an anti-pain activity capable of reducing a severe pain response. In aspects of this embodiment, a therapeutic compound disclosed herein has an anti-pain activity capable of reducing a severe pain response by, *e.g.,* at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95%. In other aspects of this embodiment, a therapeutic compound disclosed herein has an anti-pain activity capable of reducing a severe pain response in a range from, *e.g.,* 10% to 100%, 20% to 100%, 30% to 100%, 40% to 100%, 50% to 100%, 60% to 100%, 70% to 100%, 80% to 100%, 10% to 90%, 20% to 90%, 30% to 90%, 40% to 90%, 50% to 90%, 60% to 90%, 70% to 90%, 10% to 80%, 20% to 80%, 30% to 80%, 40% to 80%, 50% to 80%, or 60% to 80%, 10% to 70%, 20% to 70%, 30% to 70%, 40% to 70%, or 50% to 70%.

In an embodiment, a therapeutic compound disclosed herein has an anti-pain activity capable of reducing a nociceptive pain response. In aspects of this embodiment, a therapeutic compound disclosed herein has an anti-pain activity capable of reducing a nociceptive pain response by, *e.g.,* at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95%. In other aspects of this embodiment, a therapeutic compound disclosed herein has an anti-pain activity capable of reducing a nociceptive pain response in a range from, e.g., 10% to 100%, 20% to 100%, 30% to 100%, 40% to 100%, 50% to 100%, 60% to 100%, 70% to 100%, 80% to 100%, 10% to 90%, 20% to 90%, 30% to 90%, 40% to 90%, 50% to 90%, 60% to 90%, 70% to 90%, 10% to 80%, 20% to 80%, 30% to 80%, 40% to 80%, 50% to 80%, or 60% to 80%, 10% to 70%, 20% to 70%, 30% to 70%, 40% to 70%, or 50% to 70%.

In an embodiment, a therapeutic compound disclosed herein has an anti-pain activity capable of reducing a pain response mediated by a nociceptive receptor. In aspects of this embodiment, a therapeutic compound disclosed herein has an anti-pain activity capable of reducing a pain response mediated by a nociceptive receptor by, *e.g.,* at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95%. In other aspects of this embodiment, a therapeutic compound disclosed herein has an anti-pain activity capable of reducing a pain response mediated by a nociceptive receptor in a range from, *e.g.,* 10% to 100%, 20% to 100%, 30% to 100%, 40% to 100%, 50% to 100%, 60% to 100%, 70% to 100%, 80% to 100%, 10% to 90%, 20% to 90%, 30% to 90%, 40% to 90%, 50% to 90%, 60% to 90%, 70% to 90%, 10% to 80%, 20% to 80%, 30% to 80%, 40% to 80%, 50% to 80%, or 60% to 80%, 10% to 70%, 20% to 70%, 30% to 70%, 40% to 70%, or 50% to 70%.

In an embodiment, a therapeutic compound disclosed herein has an anti-pain activity capable of reducing a somatic pain response. In aspects of this embodiment, a therapeutic compound disclosed herein has an anti-pain activity capable of reducing a somatic pain response by, *e.g.,* at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95%. In other aspects of this embodiment, a therapeutic compound disclosed herein has an anti-pain activity capable of reducing a somatic pain response in a range from, e.g., 10% to 100%, 20% to 100%, 30% to 100%, 40% to 100%, 50% to 100%, 60% to 100%, 70% to 100%, 80% to 100%, 10% to 90%, 20% to 90%, 30% to 90%, 40% to 90%, 50% to 90%, 60% to 90%, 70% to 90%, 10% to 80%, 20% to 80%, 30% to 80%, 40% to 80%, 50% to 80%, or 60% to 80%, 10% to 70%, 20% to 70%, 30% to 70%, 40% to 70%, or 50% to 70%.

In an embodiment, a therapeutic compound disclosed herein has an anti-pain activity capable of reducing a visceral pain response. In aspects of this embodiment, a therapeutic compound disclosed herein has an anti-pain activity capable of reducing a visceral pain response by, *e.g.,* at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95%. In other aspects of this embodiment, a therapeutic compound disclosed herein has an anti-pain activity capable of reducing a visceral pain response in a range from, *e.g.,* 10% to 100%, 20% to 100%, 30% to 100%, 40% to 100%, 50% to 100%, 60% to 100%, 70% to 100%, 80% to 100%, 10% to 90%, 20% to 90%, 30% to 90%, 40% to 90%, 50% to 90%, 60% to 90%, 70% to 90%, 10% to 80%, 20% to 80%, 30% to 80%, 40% to 80%, 50% to 80%, or 60% to 80%, 10% to 70%, 20% to 70%, 30% to 70%, 40% to 70%, or 50% to 70%.

In an embodiment, a therapeutic compound disclosed herein has an anti-pain activity capable of reducing a pathological pain response. In aspects of this embodiment, a therapeutic compound disclosed herein has an anti-pain activity capable of reducing a pathological pain response by, *e.g.,* at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95%. In other aspects of this embodiment, a therapeutic compound disclosed herein has an anti-pain activity capable of reducing a pathological pain response in a range from, *e.g.,* 10% to 100%, 20% to 100%, 30% to 100%, 40% to 100%, 50% to 100%, 60% to 100%, 70% to 100%, 80% to 100%, 10% to 90%, 20% to 90%, 30% to 90%, 40% to 90%, 50% to 90%, 60% to 90%, 70% to 90%, 10% to 80%, 20% to 80%, 30% to 80%, 40% to 80%, 50% to 80%, or 60% to 80%, 10% to 70%, 20% to 70%, 30% to 70%, 40% to 70%, or 50% to 70%.

In an embodiment, a therapeutic compound disclosed herein has an anti-pain activity capable of reducing a neuropathic pain response. In aspects of this embodiment, a therapeutic compound disclosed herein has an anti-pain activity capable of reducing a neuropathic pain response by, *e.g.,* at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95%. In other aspects of this embodiment, a therapeutic compound disclosed herein has an anti-pain activity capable of reducing a neuropathic pain response in a range from, e.g., 10% to 100%, 20% to 100%, 30% to 100%, 40% to 100%, 50% to 100%, 60% to 100%, 70% to 100%, 80% to 100%, 10% to 90%, 20% to 90%, 30% to 90%, 40% to 90%, 50% to 90%, 60% to 90%, 70% to 90%, 10% to 80%, 20% to 80%, 30% to 80%, 40% to 80%, 50% to 80%, or 60% to 80%, 10% to 70%, 20% to 70%, 30% to 70%, 40% to 70%, or 50% to 70%.

In an embodiment, a therapeutic compound disclosed herein has an anti-pain activity capable of reducing a central neuropathic pain response. In aspects of this embodiment, a therapeutic compound disclosed herein has an anti-pain activity capable of reducing a central neuropathic pain response by, *e.g.,* at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95%. In other aspects of this embodiment, a therapeutic compound disclosed herein has an anti-pain activity capable of reducing a central neuropathic pain response in a range from, *e.g.,* 10% to 100%, 20% to 100%, 30% to 100%, 40% to 100%, 50% to 100%, 60% to 100%, 70% to 100%, 80% to 100%, 10% to 90%, 20% to 90%, 30% to 90%, 40% to 90%, 50% to 90%, 60% to 90%, 70% to 90%, 10% to 80%, 20% to 80%, 30% to 80%, 40% to 80%, 50% to 80%, or 60% to 80%, 10% to 70%, 20% to 70%, 30% to 70%, 40% to 70%, or 50% to 70%.

In an embodiment, a therapeutic compound disclosed herein has an anti-pain activity capable of reducing a peripheral neuropathic pain response. In aspects of this embodiment, a therapeutic compound disclosed herein has an anti-pain activity capable of reducing a peripheral neuropathic pain response by, *e.g.,* at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95%. In other aspects of this embodiment, a therapeutic compound disclosed herein has an anti-pain activity capable of reducing a peripheral neuropathic pain response in a range from, e.g., 10% to 100%, 20% to 100%, 30% to 100%, 40% to 100%, 50% to 100%, 60% to 100%, 70% to 100%, 80% to 100%, 10% to 90%, 20% to 90%, 30% to 90%, 40% to 90%, 50% to 90%, 60% to 90%, 70% to 90%, 10% to 80%, 20% to 80%, 30% to 80%, 40% to 80%, 50% to 80%, or 60% to 80%, 10% to 70%, 20% to 70%, 30% to 70%, 40% to 70%, or 50% to 70%.

In an embodiment, a therapeutic compound disclosed herein has an anti-pain activity capable of reducing a mononeuropathic pain response. In aspects of this embodiment, a therapeutic compound disclosed herein has an anti-pain activity capable of reducing a mononeuropathic pain response by, *e.g.,* at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95%. In other aspects of this embodiment, a therapeutic compound disclosed herein has an anti-pain activity capable of reducing a mononeuropathic pain response in a range from, *e.g.,* 10% to 100%, 20% to 100%, 30% to 100%, 40% to 100%, 50% to 100%, 60% to 100%, 70% to 100%, 80% to 100%, 10% to 90%, 20% to 90%, 30% to 90%, 40% to 90%, 50% to 90%, 60% to 90%, 70% to 90%, 10% to 80%, 20% to 80%, 30% to 80%, 40% to 80%, 50% to 80%, or 60% to 80%, 10% to 70%, 20% to 70%, 30% to 70%, 40% to 70%, or 50% to 70%.

In an embodiment, a therapeutic compound disclosed herein has an anti-pain activity capable of reducing a mononeuropathic multiplex pain response. In aspects of this embodiment, a therapeutic compound disclosed herein has an anti-pain activity capable of reducing a mononeuropathic multiplex pain response by, *e.g.,* at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95%. In other aspects of this embodiment, a therapeutic compound disclosed herein has an anti-pain activity capable of reducing a mononeuropathic multiplex pain response in a range from, e.g., 10% to 100%, 20% to 100%, 30% to 100%, 40% to 100%, 50% to 100%, 60% to 100%, 70% to 100%, 80% to 100%, 10% to 90%, 20% to 90%, 30% to 90%, 40% to 90%, 50% to 90%, 60% to 90%, 70% to 90%, 10% to 80%, 20% to 80%, 30% to 80%, 40% to 80%, 50% to 80%, or 60% to 80%, 10% to 70%, 20% to 70%, 30% to 70%, 40% to 70%, or 50% to 70%.

In an embodiment, a therapeutic compound disclosed herein has an anti-pain activity capable of reducing a polyneuropathic pain response. In aspects of this embodiment, a therapeutic compound disclosed herein has an anti-pain activity capable of reducing a polyneuropathic pain response by, *e.g.,* at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95%. In other aspects of this embodiment, a therapeutic compound disclosed herein has an anti-pain activity capable of reducing a polyneuropathic pain response in a range from, e.g., 10% to 100%, 20% to 100%, 30% to 100%, 40% to 100%, 50% to 100%, 60% to 100%, 70% to 100%, 80% to 100%, 10% to 90%, 20% to 90%, 30% to 90%, 40% to 90%, 50% to 90%, 60% to 90%, 70% to 90%, 10% to 80%, 20% to 80%, 30% to 80%, 40% to 80%, 50% to 80%, or 60% to 80%, 10% to 70%, 20% to 70%, 30% to 70%, 40% to 70%, or 50% to 70%.

In an embodiment, a therapeutic compound disclosed herein has an anti-pain activity capable of reducing an autonomic neuropathic pain response. In aspects of this embodiment, a therapeutic compound disclosed herein has an anti-pain activity capable of reducing an autonomic neuropathic pain response by, *e.g.,* at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95%. In other aspects of this embodiment, a therapeutic compound disclosed herein has an anti-pain activity capable of reducing an autonomic neuropathic pain response in a range from, *e.g.,* 10% to 100%, 20% to 100%, 30% to 100%, 40% to 100%, 50% to 100%, 60% to 100%, 70% to 100%, 80% to 100%, 10% to 90%, 20% to 90%, 30% to 90%, 40% to 90%, 50% to 90%, 60% to 90%, 70% to 90%, 10% to 80%, 20% to 80%, 30% to 80%, 40% to 80%, 50% to 80%, or 60% to 80%, 10% to 70%, 20% to 70%, 30% to 70%, 40% to 70%, or 50% to 70%.

In an embodiment, a therapeutic compound disclosed herein has an anti-pain activity capable of reducing a neuralgia pain response. In aspects of this embodiment, a therapeutic compound disclosed herein has an anti-pain activity capable of reducing a neuralgia pain response by, *e.g.,* at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95%. In other aspects of this embodiment, a therapeutic compound disclosed herein has an anti-pain activity capable of reducing a neuralgia pain response in a range from, *e.g.,* 10% to 100%, 20% to 100%, 30% to 100%, 40% to 100%, 50% to 100%, 60% to 100%, 70% to 100%, 80% to 100%, 10% to 90%, 20% to 90%, 30% to 90%, 40% to 90%, 50% to 90%, 60% to 90%, 70% to 90%, 10% to 80%, 20% to 80%, 30% to 80%, 40% to 80%, 50% to 80%, or 60% to 80%, 10% to 70%, 20% to 70%, 30% to 70%, 40% to 70%, or 50% to 70%.

In an embodiment, a therapeutic compound disclosed herein has an anti-pain activity capable of reducing a complex regional pain syndrome pain response. In aspects of this embodiment, a therapeutic compound disclosed herein has an anti-pain activity capable of reducing a complex regional pain syndrome pain response by, *e.g.,* at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95%. In other aspects of this embodiment, a therapeutic compound disclosed herein has an anti-pain activity capable of reducing a complex regional pain syndrome pain response in a range from, e.g., 10% to 100%, 20% to 100%, 30% to 100%, 40% to 100%, 50% to 100%, 60% to 100%, 70% to 100%, 80% to 100%, 10% to 90%, 20% to 90%, 30% to 90%, 40% to 90%, 50% to 90%, 60% to 90%, 70% to 90%, 10% to 80%, 20% to 80%, 30% to 80%, 40% to 80%, 50% to 80%, or 60% to 80%, 10% to 70%, 20% to 70%, 30% to 70%, 40% to 70%, or 50% to 70%.

In an embodiment, a therapeutic compound disclosed herein has an anti-pain activity capable of reducing a referred pain response. In aspects of this embodiment, a therapeutic compound disclosed herein has an anti-pain activity capable of reducing a referred pain response by, *e.g.,* at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95%. In other aspects of this embodiment, a therapeutic compound disclosed herein has an anti-pain activity capable of reducing a referred pain response in a range from, *e.g.,* 10% to 100%, 20% to 100%, 30% to 100%, 40% to 100%, 50% to 100%, 60% to 100%, 70% to 100%, 80% to 100%, 10% to 90%, 20% to 90%, 30% to 90%, 40% to 90%, 50% to 90%, 60% to 90%, 70% to 90%, 10% to 80%, 20% to 80%, 30% to 80%, 40% to 80%, 50% to 80%, or 60% to 80%, 10% to 70%, 20% to 70%, 30% to 70%, 40% to 70%, or 50% to 70%.

In an embodiment, a therapeutic compound disclosed herein has an anti-pain activity capable of reducing a deafferentation pain response. In aspects of this embodiment, a therapeutic compound disclosed herein has an anti-pain activity capable of reducing a deafferentation pain response by, *e.g.,* at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95%. In other aspects of this embodiment, a therapeutic compound disclosed herein has an anti-pain activity capable of reducing a deafferentation pain response in a range from, *e.g.,* 10% to 100%, 20% to 100%, 30% to 100%, 40% to 100%, 50% to 100%, 60% to 100%, 70% to 100%, 80% to 100%, 10% to 90%, 20% to 90%, 30% to 90%, 40% to 90%, 50% to 90%, 60% to 90%, 70% to 90%, 10% to 80%, 20% to 80%, 30% to 80%, 40% to 80%, 50% to 80%, or 60% to 80%, 10% to 70%, 20% to 70%, 30% to 70%, 40% to 70%, or 50% to 70%.

In an embodiment, a therapeutic compound disclosed herein has an anti-pain activity capable of reducing a dysfunctional pain response. In aspects of this embodiment, a therapeutic compound disclosed herein has an anti-pain activity capable of reducing a dysfunctional pain response by, *e.g.*, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95%. In other aspects of this embodiment, a therapeutic compound disclosed herein has an anti-pain activity capable of reducing a dysfunctional pain response in a range from, *e.g.,* 10% to 100%, 20% to 100%, 30% to 100%, 40% to 100%, 50% to 100%, 60% to 100%, 70% to 100%, 80% to 100%, 10% to 90%, 20% to 90%, 30% to 90%, 40% to 90%, 50% to 90%, 60% to 90%, 70% to 90%, 10% to 80%, 20% to 80%, 30% to 80%, 40% to 80%, 50% to 80%, or 60% to 80%, 10% to 70%, 20% to 70%, 30% to 70%, 40% to 70%, or 50% to 70%.

In an embodiment, a therapeutic compound disclosed herein has an anti-pain activity capable of reducing a headache pain response. In aspects of this embodiment, a therapeutic compound disclosed herein has an anti-pain activity capable of reducing a headache pain response by, *e.g.,* at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95%. In other aspects of this embodiment, a therapeutic compound disclosed herein has an anti-pain activity capable of reducing a headache pain response in a range from, *e.g.,* 10% to 100%, 20% to 100%, 30% to 100%, 40% to 100%, 50% to 100%, 60% to 100%, 70% to 100%, 80% to 100%, 10% to 90%, 20% to 90%, 30% to 90%, 40% to 90%, 50% to 90%, 60% to 90%, 70% to 90%, 10% to 80%, 20% to 80%, 30% to 80%, 40% to 80%, 50% to 80%, or 60% to 80%, 10% to 70%, 20% to 70%, 30% to 70%, 40% to 70%, or 50% to 70%.

In an embodiment, a therapeutic compound disclosed herein has an anti-pain activity capable of reducing a migraine pain response. In aspects of this embodiment, a therapeutic compound disclosed herein has an anti-pain activity capable of reducing a migraine pain response by, *e.g.,* at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95%. In other aspects of this embodiment, a therapeutic compound disclosed herein has an anti-pain activity capable of reducing a migraine pain response in a range from, *e.g.,* 10% to 100%, 20% to 100%, 30% to 100%, 40% to 100%, 50% to 100%, 60% to 100%, 70% to 100%, 80% to 100%, 10% to 90%, 20% to 90%, 30% to 90%, 40% to 90%, 50% to 90%, 60% to 90%, 70% to 90%, 10% to 80%, 20% to 80%, 30% to 80%, 40% to 80%, 50% to 80%, or 60% to 80%, 10% to 70%, 20% to 70%, 30% to 70%, 40% to 70%, or 50% to 70%.

A therapeutic compound disclosed herein may have a log P value indicating that the compound is soluble in an organic solvent. As used herein, the term "log P value" refers to the logarithm (base 10) of the partition coefficient (P) for a compound and is a measure of lipophilicity. Typically, P is defined as the ratio of concentrations of a unionized compound in the two phases of a mixture of two immiscible solvents at equilibrium. Thus, log P = Log 10 (P), where P = [solute in immiscible solvent 1] / [solute in immiscible solvent 2]. With regard to organic and aqueous phases, the log P value of a compound is constant for any given pair of aqueous and organic solvents, and its value can be determined empirically by one of several phase-partitioning methods known to one skilled in the art including, *e.g.*, a shake flask assay, a HPLC assay, and an interface between two immiscible electrolyte solutions (ITIES) assay.

In aspects of this embodiment, a therapeutic compound disclosed herein may have a log P value indicating that the compound is substantially soluble in an organic solvent. In aspects of this embodiment, a therapeutic compound disclosed herein may have a log P value indicating that the compound is, *e.g.,* at least 50% soluble in an organic solvent, at least 60% soluble in an organic solvent, at least 70% soluble in an organic solvent, at least 80% soluble in an organic solvent, or at least 90% soluble in an organic solvent. In aspects of this embodiment, a therapeutic compound disclosed herein may have a log P value indicating that the compound is between, *e.g.,* 50% to 100% soluble in an organic solvent, 60% to 100% soluble in an organic solvent, 70% to 100% soluble in an organic solvent, 80% to 100% soluble in an organic solvent, or 90% to 100% soluble in an organic solvent.

In aspects of this embodiment, a therapeutic compound disclosed herein may have a log P value of, *e.g.,* more than 1.1, more than 1.2, more than 1.4, more than 1.6, more than 1.8, more than 2.0, more than 2.2, more than 2.4, more than 2.6, more than 2.8, more than 3.0, more than 3.2, more than 3.4, or more than 3.6. In other aspects of this embodiment, a therapeutic compound disclosed herein may have a log P value in the range of, e.g., between 1.8 and 4.0, between 2.0 and 4.0, between 2.1 and 4.0, between 2.2 and 4.0, or between 2.3 and 4.0, between 2.4 and 4.0, between 2.5 and 4.0, between 2.6 and 4.0, or between 2.8 and 4.0. In other aspects of this embodiment, a therapeutic compound disclosed herein may have a log P value in the range of, *e.g.,* between 3.0 and 4.0, or between 3.1 and 4.0, between 3.2 and 4.0, between 3.3 and 4.0, between 3.4 and 4.0, between 3.5 and 4.0, or between 3.6 and 4.0. In still other aspects of this embodiment, a therapeutic compound disclosed herein may have a log P value in the range of, e.g., between 2.0 and 2.5, between 2.0 and 2.7, between 2.0 and 3.0, or between 2.2 and 2.5.

A therapeutic compound disclosed herein may have a polar surface area that is hydrophobic. As used herein, the term "polar surface area" refers to the surface sum over all of the polar atoms in the structure of a compound and is a measure of hydrophobicity. Typically, these polar atoms include, *e.g.,* oxygen, nitrogen, and their attached hydrogens. In aspects of this embodiment, a therapeutic compound disclosed herein may have a polar surface area of, *e.g.,* less than 8.0 nm², less than 7.0 nm², less than 6.0 nm², less than 5.0 nm², less than 4.0 nm², or less than 3.0 nm². In other aspects of this embodiment, a therapeutic compound disclosed herein may have a polar surface area in the range of, *e.g.,* between 3.0 nm² and 6.5 nm², between 3.0 nm² and 6.0 nm², between 3.0 nm² and 5.5 nm², between 3.0 nm² and 5.0 nm², between 3.0 nm² and 4.5 nm², between 3.5 nm² and 6.5 nm², between 3.5 nm² and 6.0 nm², between 3.5 nm² and 5.5 nm², between 3.5 nm² and 5.0 nm², between 3.5 nm² and 4.5 nm², between 4.0 nm² and 6.5 nm², between 4.0 nm² and 6.0 nm², between 4.0 nm² and 5.5 nm², or between 4.0 nm² and 5.0 nm², between 4.0 nm² and 4.5 nm², or between 4.5 nm² and 5.5 nm². In yet other aspects of this embodiment, a therapeutic compound disclosed herein may have a polar surface area in the range of, *e.g.,* between 2.0 nm² and 6.5 nm², between 2.0 nm² and 6.0 nm², between 2.0 nm² and 5.5 nm², between 2.0 nm² and 5.0 nm², between 2.0 nm² and 4.5 nm², between 2.5 nm² and 6.5 nm², between 2.5 nm² and 6.0 nm², between 2.5 nm² and 5.5 nm², between 2.5 nm² and 5.0 nm², or between 2.5 nm² and 4.5 nm².

A therapeutic compound disclosed herein may be a non-steroidal anti-inflammatory drug (NSAID). NSAIDs are a large group of therapeutic compounds with analgesic, anti-inflammatory, and anti-pyretic properties. NSAIDs reduce inflammation by blocking cyclooxygenase. NSAIDs include, without limitation, Aceclofenac, Acemetacin, Actarit, Alcofenac, Alminoprofen, Amfenac, Aloxipirin, Aminophenazone, Antraphenine, Aspirin, Azapropazone, Benorilate, Benoxaprofen, Benzydamine, Butibufen, Celecoxib, Chlorthenoxacin, Choline Salicylate, Clometacin, Dexketoprofen, Diclofenac, Diflunisal, Emorfazone, Epirizole; Etodolac, Etoricoxib, Feclobuzone, Felbinac, Fenbufen, Fenclofenac, Flurbiprofen, Glafenine, Hydroxylethyl salicylate, Ibuprofen, Indometacin, Indoprofen, Ketoprofen, Ketorolac, Lactyl phenetidin, Loxoprofen, Lumiracoxib, Mefenamic acid, Meloxicam, Metamizole, Metiazinic acid, Mofebutazone, Mofezolac, Nabumetone, Naproxen, Nifenazone, Niflumic acid, Oxametacin, Phenacetin, Pipebuzone, Pranoprofen, Propyphenazone, Proquazone, Protizinic acid, Rofecoxib, Salicylamide, Salsalate, Sulindac, Suprofen, Tiaramide, Tinoridine, Tolfenamic acid, Valdecoxib, and Zomepirac.

NSAIDs may be classified based on their chemical structure or mechanism of action. Non-limiting examples of NSAIDs include a salicylate derivative NSAID, a p-amino phenol derivative NSAID, a propionic acid derivative NSAID, an acetic acid derivative NSAID, an enolic acid derivative NSAID, a fenamic acid derivative NSAID, a non-selective cyclo-oxygenase (COX) inhibitor, a selective cyclooxygenase 1 (COX 1) inhibitor, and a selective cyclooxygenase 2 (COX 2) inhibitor. A NSAID may be a profen. Examples of a suitable salicylate derivative NSAID include, without limitation, Acetylsalicylic acid (asprin), Diflunisal, and Salsalate. Examples of a suitable p-amino phenol derivative NSAID include, without limitation, Paracetamol and Phenacetin. Examples of a suitable propionic acid derivative NSAID include, without limitation, Alminoprofen, Benoxaprofen, Dexketoprofen, Fenoprofen, Flurbiprofen, Ibuprofen, Indoprofen, Ketoprofen, Loxoprofen, Naproxen, Oxaprozin, Pranoprofen, and Suprofen. Examples of a suitable acetic acid derivative NSAID include, without limitation, Aceclofenac, Acemetacin, Actarit, Alcofenac, Amfenac, Clometacin, Diclofenac, Etodolac, Felbinac, Fenclofenac, Indometacin, Ketorolac, Metiazinic acid, Mofezolac, Nabumetone, Naproxen, Oxametacin, Sulindac, and Zomepirac. Examples of a suitable enolic acid (Oxicam) derivative NSAID include, without limitation, Droxicam, Isoxicam, Lornoxicam, Meloxicam, Piroxicam, and Tenoxicam. Examples of a suitable fenamic acid derivative NSAID include, without limitation, Flufenamic acid, Mefenamic acid, Meclofenamic acid, and Tolfenamic acid. Examples of a suitable selective COX-2 inhibitors include, without limitation, Celecoxib, Etoricoxib, Firocoxib, Lumiracoxib, Meloxicam, Paracetamol (Acetaminophen), Parecoxib, Rofecoxib, and Valdecoxib. Only therapeutic compounds having an anti-pain activity are part of the invention.

A therapeutic compound disclosed herein may be a PPARα agonist. Examples of a suitable PPARα agonist include, without limitation, Pirinixic (WY 14643), GW6471, and a Fibrate.

A therapeutic compound disclosed herein may be a PPARβ/δ agonist. Examples of a suitable PPARβ/δ agonist include, without limitation, Tetradecylthioacetic acid (TTA). GSK0660, GSK3787, GW501516 (GW-501,516, GW1516, GSK-516 and Endurobol), GW0742, and GW610742X.

A therapeutic compound disclosed herein may be a PPARγ agonist. Examples of a suitable PPARγ agonist include, without limitation, Monascin, a Thiazolidinediones like Rosiglitazone, Pioglitazone, and Troglitazone and T0070907. Other suitable PPARγ agonists are described in Masson and Caumont-Bertrand, *PPAR Agonist Compounds, Preparation and Uses,* US 2011/0195993.

A therapeutic compound disclosed herein may be a Glitazar (a duel α and γ PPAR agonist). Examples of a suitable Glitazar include, without limitation, Aleglitazar, Muraglitazar, Saroglitazar, and Tesaglitazar.

A therapeutic compound disclosed herein may be an immunosupressive drug. Examples of a suitable immunosupressive drug include, without limitation, Azathioprine and Mycophenolic acid.

A therapeutic compound disclosed herein may be an uricosuric drug. Examples of a suitable uricosuric drug include, without limitation, Benzbromarone.

A therapeutic compound disclosed herein may be an Aglycone. Examples of a suitable Aglycone drug include, without limitation, Piceatannol, Pinosylvin, Pterostilbene, and Resveratrol

A therapeutic compound disclosed herein may be a Cannabidiol. Examples of a suitable uricosuric drug include, without limitation, a Phytocannabinoid, an Endocannabinoid, and a synthetic cannabinoid. A Phytocannabinoid includes a Tetrahydrocannabinol (such as, *e.g.,* Delta-9-tetrahydrocannabinol (Δ9-THC, THC), and Delta-8-tetrahydrocannabinol (Δ8-THC)), a Cannabidiol, a Cannabinol, a Cannabigerol, a Tetrahydrocannabivarin, a Cannabidivarin, and a Cannabichromene. An Endocannabinoid includes Arachidonoylethanolamine (Anandamide or AEA), 2-arachidonoyl glycerol (2-AG), 2-arachidonyl glyceryl ether (noladin ether), N-arachidonoyl-dopamine (NADA), Virodhamine (OAE), and Lysophosphatidylinositol (LPI). A synthetic cannabinoid includes Dronabinol (Marinol), Nabilone (Cesamet), Sativex, Rimonabant (SR141716), JWH-018, JWH-073, CP-55940, Dimethylheptylpyran, HU-210, HU-331, SR144528, WIN 55,212-2, JWH-133, Levonantradol (Nantrodolum), and AM-2201.

A therapeutic compound disclosed herein may be a nuclear receptor binding agent. Examples of a suitable nuclear receptor binding agent include, without limitation, a Retinoic Acid Receptor (RAR) binding agent, a Retinoid X Receptor (RXR) binding agent, a Liver X Receptor (LXR) binding agent and a Vitamin D binding agent.

A therapeutic compound disclosed herein may be an Angiotensin II receptor antagonist. Examples of a suitable Angiotensin II receptor antagonist include, without limitation, Azilsartan, Candesartan, Eprosartan, Irbesartan, Losartan, Olmesartan, Telmisartan, and Valsartan.

A therapeutic compound disclosed herein may be an Acetylcholinesterase (ACE) inhibitor. Examples of a suitable ACE inhibitor include, without limitation, a Sulfhydryl-containing agent, a Dicarboxylate-containing agent, a Phosphonate-containing agent, a Casokinin, and a Lactokinin. A Sulfhydryl-containing agent includes Captopril (Capoten) and Zofenopril. A Dicarboxylate-containing agent includes Enalapril (Vasotec/Renitec), Ramipril (Altace/Prilace/Ramace/Ramiwin/Triatec/Tritace), Quinapril (Accupril), Perindopril (Coversyl/Aceon), Lisinopril (Listril/Lopril/Novatec/Prinivil/Zestril), Benazepril (Lotensin), Imidapril (Tanatril), Zofenopril (Zofecard), and Trandolapril (Mavik/Odrik/Gopten). A Phosphonate-containing agent includes Fosinopril (Fositen/Monopril).

A therapeutic compound disclosed herein may be a Phosphodiesterase inhibitor. Examples of a suitable Phosphodiesterase inhibitor include, without limitation, a PDE 1 selective inhibitor, a PDE 2 selective inhibitor, a PDE 3 selective inhibitor, a PDE 4 selective inhibitor, a PDE 5 selective inhibitor, and a PDE 10 selective inhibitor. A PDE1 selective inhibitor includes Vinpocetine. A PDE2 selective inhibitor includes BAY 60-7550 (2-[(3,4-dimethoxyphenyl)methyl]-7-[(1R)-1-hydroxyethyl]-4-phenylbutyl]-5-methyl-imidazo[5,1-f][1,2,4]triazin-4(1H)-one), EHNA (erythro-9-(2-hydroxy-3-nonyl)adenine), Oxindole, and PDP (9-(6-Phenyl-2-oxohex-3-yl)-2-(3,4-dimethoxybenzyl)-purin-6-one). A PDE3 selective inhibitor includes Anagrelide, Cilostazol, Enoximone, Inamrinone, and Milrinone. A PDE4 selective inhibitor includes Drotaverine, Ibudilast, Luteolin, Mesembrine, Piclamilast, Roflumilast, and Rolipram. A PDE5 selective inhibitor includes Avanafil, Dipyridamole, Icariin, 4-Methylpiperazine, Pyrazolo Pyrimidin-7-1, Sildenafil, Tadalafil, Udenafil, and Vardenafil. A PDE10 selective inhibitor includes Papaverine.

A therapeutic compound disclosed herein may be a fibrate. Fibrates are a class of amphipathic carboxylic acids with lipid level modifying properties. These therapeutic compounds are used for a range of metabolic disorders. One non-limiting use is as an anti-hyperlipidemic agent where it may lower levels of, e.g., triglycerides and LDL as well as increase levels of HDL. Examples of a suitable fibrate include, without limitation, Bezafibrate, Ciprofibrate, Clofibrate, Gemfibrozil, and Fenofibrate.

A therapeutic compound disclosed herein may be a statin. Statins (or HMG-CoA reductase inhibitors) are a class of therapeutic compounds used to lower LDL and/or cholesterol levels by inhibiting the enzyme HMG-CoA reductase, which plays a central role in the production of cholesterol in the liver. To compensate for the decreased cholesterol availability, synthesis of hepatic LDL receptors is increased, resulting in an increased clearance of LDL particles from the blood. Examples of a suitable statin include, without limitation, Atorvastatin, Fluvastatin, Lovastatin, Pitavastatin, Pravastatin, Rosuvastatin, and Simvastatin.

A therapeutic compound disclosed herein may be a tocotrienol. Tocotrienols are another class of HMG-CoA reductase inhibitors and may be used to lower LDL and/or cholesterol levels by inducing hepatic LDL receptor up-regulation and/or decreasing plasma LDL levels. Examples of a suitable tocotrienol include, without limitation, a γ-tocotrienol and a δ-tocotrienol.

A therapeutic compound disclosed herein may be a niacin. Niacins are a class of therapeutic compounds with lipid level modifying properties. For example, a niacin may lower LDL by selectively inhibiting hepatic diacyglycerol acyltransferase 2, reduce triglyceride synthesis, and VLDL secretion through a receptor HM74 and HM74A or GPR109A. These therapeutic compounds are used for a range of metabolic disorders. One non-limiting use is as an anti-hyperlipidemic agent where it may inhibit the breakdown of fats in adipose tissue. Because a niacin blocks the breakdown of fats, it causes a decrease in free fatty acids in the blood and, as a consequence, decreases the secretion of very-low-density lipoproteins (VLDL) and cholesterol by the liver. By lowering VLDL levels, a niacin may also increase the level of HDL in blood. Examples of a suitable niacin include, without limitation, Acipimox, Niacin, Nicotinamide, and Vitamin B3.

A therapeutic compound disclosed herein may be a bile acid sequestrant. Bile acid sequestrants (also known as resins) are a class of therapeutic compounds used to bind certain components of bile in the gastrointestinal tract. They disrupt the enterohepatic circulation of bile acids by sequestering them and preventing their reabsorption from the gut. Bile acid sequestrants are particularly effective for lowering LDL and cholesterol by sequestering the cholesterol-containing bile acids released into the intestine and preventing their reabsorption from the intestine. In addition, a bile acid sequestrant may also raise HDL levels. Examples of a suitable bile acid sequestrant include, without limitation, Cholestyramine, Colesevelam, and Colestipol.

A therapeutic compound disclosed herein may be a cholesterol absorption inhibitor. Cholesterol absorption inhibitors are a class of therapeutic compounds that inhibits the absorption of cholesterol from the intestine. Decreased cholesterol absorption leads to an upregulation of LDL-receptors on the surface of cells and an increased LDL-cholesterol uptake into these cells, thus decreasing levels of LDL in the blood plasma. Examples of a suitable cholesterol absorption inhibitor include, without limitation, Ezetimibe, a phytosterol, a sterol and a stanol.

A therapeutic compound disclosed herein may be a fat absorption inhibitor. Fat absorption inhibitors are a class of therapeutic compounds that inhibits the absorption of fat from the intestine. Decreased fat absorption reduces caloric intake. In one aspect, a fat absorption inhibitor inhibits pancreatic lipase, an enzyme that breaks down triglycerides in the intestine. Examples of a suitable fat absorption inhibitor include, without limitation, Orlistat.

A therapeutic compound disclosed herein may be a sympathomimetic amine. Sympathomimetic amines are a class of therapeutic compounds that mimic the effects of transmitter substances of the sympathetic nervous system such as catecholamines, epinephrine (adrenaline), norepinephrine (noradrenaline), and/or dopamine. A sympathomimetic amine may act as an α-adrenergic agonist, a β-adrenergic agonist, a dopaminergic agonist, a monoamine oxidase (MAO) inhibitor, and a COMT inhibitor. Such therapeutic compounds, among other things, are used to treat cardiac arrest, low blood pressure, or even delay premature labor. Examples of a suitable sympathomimetic amine include, without limitation, Clenbuterol, Salbutamol, ephedrine, pseudoephedrine, methamphetamine, amphetamine, phenylephrine, isoproterenol, dobutamine, methylphenidate, lisdexamfetamine, cathine, cathinone, methcathinone, cocaine, benzylpiperazine (BZP), methylenedioxypyrovalerone (MDPV), 4-methylaminorex, pemoline, phenmetrazine, and propylhexedrine. An α-adrenergic agonist includes Phenylephrine, Propylhexedrine, and Pseudoephedrine. A β-adrenergic agonist includes Clenbuterol, Dobutamine, Eephedrine, Isoproterenol, and Salbutamol. A Dopaminergic/Norepinephrinergic agonist includes Cocaine (DA/NE reuptake inhibitor), Lisdexamfetamine (5HT/DA/NE reuptake inhibitor), Methylphenidate (DA/NE reuptake inhibitor), and Methylenedioxypyrovalerone (DA/NE reuptake inhibitor). A Neurotransmitter releasing agent includes Amphetamine (DA/NE releasing agent), Benzylpiperazine (DA/NE releasing agent), Cathine (DA/NE releasing agent), Cathinone (DA/NE releasing agent), Methamphetamine (DA/NE releasing agent), Methcathinone (DA/NE releasing agent), 4-methylaminorex (DA/NE releasing agent), Pemoline, Phenmetrazine (DA/NE releasing agent), and Phenethylamine (DA/NE releasing agent).

A therapeutic compound disclosed herein may be a Ryanodine receptor antagonist. Examples of a Ryanodine receptor antagonist include, without limitation, Azumolene and Dantrolene.

A therapeutic compound disclosed herein may be a cancer drug. Examples of a suitable cancer drug include, without limitation, an alkylating agent, an anti-metabolite, a plant alkaloid and terpenoid, a topoisomerase inhibitor and a cytotoxic antibiotic. An alkylating agent incudes Carboplatin, Chlorambucil, Cisplatin, Cyclophosphamide, Ifosfamide, Oxaliplatin, and Mechlorethamine. An anti-metabolite includes Azathioprine and Mercaptopurine. A plant alkaloid and terpenoid include a Vinca alkaloid like Vincristine, Vinblastine, Vinorelbine, and Vindesine, a Podophyllotoxin like Etoposide and Teniposide, and a Taxane like Docetaxel and Ortataxel. A Topoisomerase inhibitor includes a Type I topoisomerase inhibitor like a Camptothecins, such as, *e.g.,* Exatecan, Irinotecan, Lurtotecan, Topotecan, BNP 1350, CKD 602, DB 67 (AR67), and ST 1481, and a Type II inhibitor like an Epipodophyllotoxin such as, *e.g.,* Amsacrine, Etoposid, Etoposide phosphate, and Teniposide. A Cytotoxic antibiotic includes an Actinomycin like Actinomycin D, Bacitracin, Colistin (polymyxin E), and Polymyxin B, an Anthracenedione like Mitoxantrone and Pixantrone, and a Anthracycline like Bleomycin, Doxorubicin (Adriamycin), Daunorubicin (Daunomycin), Epirubicin, Idarubicin, Mitomycin, Plicamycin, and Valrubicin.

A therapeutic compound disclosed herein may be Metformin, Curcumin, glycyrrhetinic acid, or 6-shogaol.

A therapeutic compound disclosed herein may be an antibiotic. Examples of a suitable sympathomimetic amine include, without limitation, Isoniazid, Rifampicin, Pyrazinamide, and Ethambutol.

A therapeutic compound disclosed herein may be an anti-helmintic drug. Examples of a suitable anti-helmintic drug include, without limitation, Abamectin, an Aminoacetonitrile like Monepantel, a Benzimidazole, Diethylcarbamazine, Ivermectin, Levamisole, Niclosamide, an Octadepsipeptide like Emodepside, Phosphonic acid (Metrifonate), Praziquantel, a Spiroindole like Derquantel, and Suramin Pyrantel pamoate. A Benzimidazole includes Albendazole, Fenbendazole, Flubendazole, Mebendazole, Thiabendazole, and Triclabendazole.

A therapeutic compound disclosed herein may be an anti-malaria drug. Examples of a suitable anti-malaria drug include, without limitation, Amodiaquine, an Artemisinin, Atovaquone, Chloroquine, Clindamycin, Doxycycline, Halofantrine, Mefloquine, Primaquine, Proguanil, Pyrimethamine, a Quinine and related agent like Quinimax and Quinidine, Rufigallol, and a Sulfonamide like Sulfadoxine and Sulfamethoxypyridazine. An Artemisinin incudes Arteether, Artemether, Artemisinin, Artesunate, and Dihydroartemisinin.

A therapeutic compound disclosed herein may be an anti-hyperlipidemic agent. There are several classes of anti-hyperlipidemic agents (also known as hypolipidemic agents). They may differ in both their impact on the cholesterol profile and adverse effects. For example, some may lower low density lipoprotein (LDL), while others may preferentially increase high density lipoprotein (HDL). Clinically, the choice of an agent will depend on the cholesterol profile of an individual, cardiovascular risk of an individual, and/or the liver and kidney functions of an individual. Examples of a suitable anti-hyperlipidemic agent include, without limitation, an Angiotensin II receptor antagonist, an ACE inhibitor, a Phosphodiesterase inhibitor, a Fibrate, a Statin, a Tocotrienol, a Niacin, a bile acid sequestrants (resin), a cholesterol absorption inhibitor, a pancreatic lipase inhibitor, and a sympathomimetic amine.

A therapeutic compound disclosed herein may be an ester of a therapeutic compound. An ester of a therapeutic compound increases the logP value relative to the same therapeutic compound, but without the ester modification. An ester group may be attached to a therapeutic compound by, *e.g.,* a carboxylic acid or hydroxyl functional group present of the therapeutic compound. An ester of a therapeutic compound may have an increased hydrophobicity, and as such, may be dissolved in a reduced volume of solvent disclosed herein. In some instances, an ester of a therapeutic compound may be combined directly with an adjuvant disclosed herein, thereby eliminating the need of a solvent. An ester of a therapeutic compound may enable the making of a pharmaceutical composition disclosed herein, in situations where a non-esterified form of the same therapeutic compound is otherwise immiscible in a solvent disclosed herein. An ester of a therapeutic compound may still be delivered in a manner that more effectively inhibits a pro-inflammatory response as long as the compound is combined with an adjuvant disclosed herein. In one embodiment, a therapeutic compound may be reacted with ethyl ester in order to form an ethyl ester of the therapeutic compound.

A solid solution pharmaceutical composition disclosed herein may comprise a therapeutic compound in an amount sufficient to allow customary administration to an individual. In aspects of this embodiment, a pharmaceutical composition disclosed herein may comprise a therapeutic compound in an amount of, *e.g.,* at least 5 mg, at least 10 mg, at least 15 mg, at least 20 mg, at least 25 mg, at least 30 mg, at least 35 mg, at least 40 mg, at least 45 mg, at least 50 mg, at least 55 mg, at least 60 mg, at least 65 mg, at least 70 mg, at least 75 mg, at least 80 mg, at least 85 mg, at least 90 mg, at least 95 mg, or at least 100 mg. In other aspects of this embodiment, a pharmaceutical composition disclosed herein may comprise a therapeutic compound in an amount of, *e.g.,* at least 5 mg, at least 10 mg, at least 20 mg, at least 25 mg, at least 50 mg, at least 75 mg, at least 100 mg, at least 200 mg, at least 300 mg, at least 400 mg, at least 500 mg, at least 600 mg, at least 700 mg, at least 800 mg, at least 900 mg, at least 1,000 mg, at least 1,100 mg, at least 1,200 mg, at least 1,300 mg, at least 1,400 mg, or at least 1,500 mg. In yet other aspects of this embodiment, a pharmaceutical composition disclosed herein may comprise a therapeutic compound in an amount of, e.g., 5 mg to 100 mg, 10 mg to 100 mg, 50 mg to 150 mg, 100 mg to 250 mg, 150 mg to 350 mg, 250 mg to 500 mg, 350 mg to 600 mg, 500 mg to 750 mg, 600 mg to 900 mg, 750 mg to 1,000 mg, 850 mg to 1,200 mg, or 1,000 mg to 1,500 mg. In still other aspects of this embodiment, a pharmaceutical composition disclosed herein may comprise a therapeutic compound in an amount of, *e.g.,* 10 mg to 250 mg, 10 mg to 500 mg, 10 mg to 750 mg, 10 mg to 1,000 mg, 10 mg to 1,500 mg, 50 mg to 250 mg, 50 mg to 500 mg, 50 mg to 750 mg, 50 mg to 1,000 mg, 50 mg to 1,500 mg, 100 mg to 250 mg, 100 mg to 500 mg, 100 mg to 750 mg, 100 mg to 1,000 mg, 100 mg to 1,500 mg, 200 mg to 500 mg, 200 mg to 750 mg, 200 mg to 1,000 mg, 200 mg to 1,500 mg, 5 mg to 1,500 mg, 5 mg to 1,000 mg, or 5 mg to 250 mg.

The pharmaceutical composition disclosed herein comprises a therapeutic compound in an amount of 5% to 55% by weight.

In other aspects of this embodiment, a pharmaceutical composition disclosed herein may comprise a therapeutic compound in an amount of, *e.g.,* 5% to 45% by weight, 5% to 40% by weight, 5% to 35% by weight, 5% to 30% by weight, 5% to 25% by weight, 5% to 20% by weight, 5% to 15% by weight, 5% to 10% by weight, 10% to 45% by weight, 10% to 40% by weight, 10% to 35% by weight, 10% to 30% by weight, 10% to 25% by weight, 10% to 20% by weight, 10% to 15% by weight, 15% to 45% by weight, 15% to 40% by weight, 15% to 35% by weight, 15% to 30% by weight, 15% to 25% by weight, 15% to 20% by weight, 20% to 45% by weight, 20% to 40% by weight, 20% to 35% by weight, 20% to 30% by weight, 20% to 25% by weight, 25% to 45% by weight, 25% to 40% by weight, 25% to 35% by weight, or 25% to 30% by weight.

The final concentration of a therapeutic compound disclosed herein in a pharmaceutical composition disclosed herein may be of any concentration desired. In an aspect of this embodiment, the final concentration of a therapeutic compound in a pharmaceutical composition may be a therapeutically effective amount. In other aspects of this embodiment, the final concentration of a therapeutic compound in a pharmaceutical composition may be, *e.g.,* at least 0.00001 mg/mL, at least 0.0001 mg/mL, at least 0.001 mg/mL, at least 0.01 mg/mL, at least 0.1 mg/mL, at least 1 mg/mL, at least 10 mg/mL, at least 25 mg/mL, at least 50 mg/mL, at least 100 mg/mL, at least 200 mg/mL, at least 500 mg/mL, at least 700 mg/mL, at least 1,000 mg/mL, or at least 1,200 mg/mL. In other aspects of this embodiment, the concentration of a therapeutic compound disclosed herein in the solution may be, *e.g.,* at most 1,000 mg/mL, at most 1,100 mg/mL, at most 1,200 mg/mL, at most 1,300 mg/mL, at most 1,400 mg/mL, at most 1,500 mg/mL, at most 2,000 mg/mL, at most 2,000 mg/mL, or at most 3,000 mg/mL. In other aspects of this embodiment, the final concentration of a therapeutic compound in a pharmaceutical composition may be in a range of, *e.g.,* 0.00001 mg/mL to 3,000 mg/mL, 0.0001 mg/mL to 3,000 mg/mL, 0.01 mg/mL to 3,000 mg/mL, 0.1 mg/mL to 3,000 mg/mL, 1 mg/mL to 3,000 mg/mL, 250 mg/mL to 3,000 mg/mL, 500 mg/mL to 3,000 mg/mL, 750 mg/mL to 3,000 mg/mL, 1,000 mg/mL to 3,000 mg/mL, 100 mg/mL to 2,000 mg/mL, 250 mg/mL to 2,000 mg/mL, 500 mg/mL to 2,000 mg/mL, 750 mg/mL to 2,000 mg/mL, 1,000 mg/mL to 2,000 mg/mL, 100 mg/mL to 1,500 mg/mL, 250 mg/mL to 1,500 mg/mL, 500 mg/mL to 1,500 mg/mL, 750 mg/mL to 1,500 mg/mL, 1,000 mg/mL to 1,500 mg/mL, 100 mg/mL to 1,200 mg/mL, 250 mg/mL to 1,200 mg/mL, 500 mg/mL to 1,200 mg/mL, 750 mg/mL to 1,200 mg/mL, 1,000 mg/mL to 1,200 mg/mL, 100 mg/mL to 1,000 mg/mL, 250 mg/mL to 1,000 mg/mL, 500 mg/mL to 1,000 mg/mL, 750 mg/mL to 1,000 mg/mL, 100 mg/mL to 750 mg/mL, 250 mg/mL to 750 mg/mL, 500 mg/mL to 750 mg/mL, 100 mg/mL to 500 mg/mL, 250 mg/mL to 500 mg/mL, 0.00001 mg/mL to 0.0001 mg/mL, 0.00001 mg/mL to 0.001 mg/mL, 0.00001 mg/mL to 0.01 mg/mL, 0.00001 mg/mL to 0.1 mg/mL, 0.00001 mg/mL to 1 mg/mL, 0.001 mg/mL to 0.01 mg/mL, 0.001 mg/mL to 0.1 mg/mL, 0.001 mg/mL to 1 mg/mL, 0.001 mg/mL to 10 mg/mL, or 0.001 mg/mL to 100 mg/mL.

Aspects of the present specification disclose, in part, one or more lipids. A lipid may be broadly defined as a hydrophobic or amphiphilic small molecule. The amphiphilic nature of some lipids allows them to form structures such as vesicles, liposomes, or membranes in an aqueous environment. Non-limiting examples, of lipids include fatty acids, glycerolipids, phospholipids, sphingolipids, sterol lipids, prenol lipids, saccharolipids, and polyketides.

A lipid useful in the pharmaceutical compositions disclosed herein may be a pharmaceutically-acceptable fatty acid. A fatty acid comprises a carboxylic acid with a long unbranched hydrocarbon chain which may be either saturated or unsaturated. Thus arrangement confers a fatty acid with a polar, hydrophilic end, and a nonpolar, hydrophobic end that is insoluble in water. Most naturally occurring fatty acids have a hydrocarbon chain of an even number of carbon atoms, typically between 4 and 24 carbons, and may be attached to functional groups containing oxygen, halogens, nitrogen, and sulfur. Synthetic or non-natural fatty acids may have a hydrocarbon chain of any number of carbon atoms from between 3 and 40 carbons. Where a double bond exists, there is the possibility of either a cis or a trans geometric isomerism, which significantly affects the molecule's molecular configuration. Cis-double bonds cause the fatty acid chain to bend, an effect that is more pronounced the more double bonds there are in a chain. Most naturally occurring fatty acids are of the cis configuration, although the trans form does exist in some natural and partially hydrogenated fats and oils. Examples of fatty acids include, without limitation, Capryllic acid (8:0), Pelargonic acid (9:0), Capric acid (10:0), Undecylic acid (11:0), Lauric acid (12:0), Tridecylic acid (13:0), Myristic acid (14:0), Myristoleic acid (14:1), Pentadecyclic acid (15:0), Palmitic acid (16:0), Palmitoleic acid (16:1), Sapienic acid (16:1), Margaric acid (17:0), Stearic acid (18:0), Oleic acid (18:1), Elaidic acid (18:1), Vaccenic acid (18:1), Linoleic acid (18:2), Linoelaidic acid (18:2), α-Linotenic acid (18:3), γ-Linolenic acid (18:3), Stearidonic acid (18:4), Nonadecylic acid (19:0), Arachidic acid (20:0), Eicosenoic acid (20:1), Dihomo-γ-linolenic acid (20:3), Mead acid (20:3), Arachidonic acid (20:4), Eicosapentaenoic acid (20:5), Heneicosylic acid (21:0), Behenic acid (22:0), Erucic acid (22:1), Docosahexaenoic acid (22:6), Tricosylic acid (23:0), Lignoceric acid (24:0), Nervonic acid (24:1), Pentacosylic acid (25:0), Cerotic acid (26:0), Heptacosylic acid (27:0), Montanic acid (28:0), Nonacosylic acid (29:0), Melissic acid (30:0), Henatriacontylic acid (31:0), Lacceroic acid (32:0), Psyllic acid (33:0), Geddic acid (34:0), Ceroplastic acid (35:0), and Hexatriacontylic acid (36:0).

In an embodiment, a lipid may be a pharmaceutically-acceptable saturated or unsaturated fatty acid. In aspects of this embodiment, a saturated or unsaturated fatty acid comprises, *e.g.,* at least 8, at least 10, at least 12, at least 14, at least 16, at least 18, at least 20, at least 22, at least 24, at least 26, at least 28, or at least 30 carbon atoms, In other aspects of this embodiment, a saturated or unsaturated fatty acid comprises, *e.g.,* between 4 and 24 carbon atoms, between 6 and 24 carbon atoms, between 8 and 24 carbon atoms, between 10 and 24 carbon atoms, between 12 and 24 carbon atoms, between 14 and 24 carbon atoms, or between 16 and 24 carbon atoms, between 4 and 22 carbon atoms, between 6 and 22 carbon atoms, between 8 and 22 carbon atoms, between 10 and 22 carbon atoms, between 12 and 22 carbon atoms, between 14 and 22 carbon atoms, or between 16 and 22 carbon atoms, between 4 and 20 carbon atoms, between 6 and 20 carbon atoms, between 8 and 20 carbon atoms, between 10 and 20 carbon atoms, between 12 and 20 carbon atoms, between 14 and 20 carbon atoms, or between 16 and 20 carbon atoms. If unsaturated, the fatty acid may have, *e.g.,* 1 or more, 2 or more, 3 or more, 4 or more, 5 or more, or 6 or more double bonds.

A lipid useful in the pharmaceutical compositions disclosed herein may be a pharmaceutically-acceptable hard fat. Also known as a "solid fat", room temperature solid lipid", or simply "fat", a hard fat includes any fatty acid that is solid at normal room temperature, such as, *e.g.* 20°C. Fats consist of a wide group of compounds that are generally soluble in organic solvents and generally insoluble in water. Examples of mixtures of pharmaceutically-acceptable hard fats include, without limitation, a mixture of one or more glycerolipids disclosed herein, a mixture of one or more glycol fatty acid esters disclosed herein, a mixture of more polyether fatty acid esters disclosed herein, a mixture of more glycerides disclosed herein.

A hard fat used in the pharmaceutical compositions disclosed is a pharmaceutically-acceptable triglyceride which may include one saturated or unsaturated fatty acid having a carbon length of C₁₂-C₂₄, two saturated or unsaturated fatty acids each having a carbon length of C₁₂-C₂₄, or three saturated or unsaturated fatty acids each having a carbon length of C₁₂-C₂₄.

Solid lipids which form part of the invention are triglycerides having a melting point of 40°C to 50°C, such as 40°C to 44°C, 40°C to 45°C, 40°C to 46°C, 40°C to 47°C, 40°C to 48°C, 40°C to 49°C, 40°C to 50°C, 42°C to 44°C, 42°C to 45°C, 42°C to 46°C, 42°C to 47°C, 42°C to 48°C, 42°C to 49°C, or 42°C to 50°C.

In other aspects of this embodiment, only the compositions defined by the claim scope are part of the invention, a mixture of pharmaceutically-acceptable lipids includes a mixture PEG fatty acid esters having a melting point of, *e.g.,* 33°C, 34°C, 35°C, 36°C, 37°C, 38°C, 39°C, 40°C, 41°C, 43°C, 43°C, 44°C, 45°C, 45°C, 47°C, 48°C, 49°C, 50°C. In aspects of this embodiment, a mixture of pharmaceutically-acceptable lipids includes a mixture PEG fatty acid esters having a melting point of, e.g., 30°C to 44°C, 30°C to 45°C, 30°C to 46°C, 30°C to 47°C, 30°C to 48°C, 30°C to 49°C, 30°C to 50°C, 32°C to 44°C, 32°C to 45°C, 32°C to 46°C, 32°C to 47°C, 32°C to 48°C, 32°C to 49°C, 32°C to 50°C, 34°C to 44°C, 34°C to 45°C, 34°C to 46°C, 34°C to 47°C, 34°C to 48°C, 34°C to 49°C, 34°C to 50°C, 36°C to 44°C, 36°C to 45°C, 36°C to 46°C, 36°C to 47°C, 36°C to 48°C, 36°C to 49°C, 36°C to 50°C, 38°C to 44°C, 38°C to 45°C, 38°C to 46°C, 38°C to 47°C, 38°C to 48°C, 38°C to 49°C, 38°C to 50°C, 40°C to 44°C, 40°C to 45°C, 40°C to 46°C, 40°C to 47°C, 40°C to 48°C, 40°C to 49°C, 40°C to 50°C, 42°C to 44°C, 42°C to 45°C, 42°C to 46°C, 42°C to 47°C, 42°C to 48°C, 42°C to 49°C, or 42°C to 50°C.

In other aspects of this embodiment, a mixture of pharmaceutically-acceptable lipids includes a mixture of mono-, di-, and/or triglycerides and PEG fatty acid esters having a melting point of, *e.g.,* 33°C, 34°C, 35°C, 36°C, 37°C, 38°C, 39°C, 40°C, 41°C, 43°C, 43°C, 44°C, 45°C, 45°C, 47°C, 48°C, 49°C, 50°C. In aspects of this embodiment, a mixture of pharmaceutically-acceptable lipids includes a mixture of mono-, di-, and/or triglycerides and PEG fatty acid esters having a melting point of, *e.g.,* 30°C to 44°C, 30°C to 45°C, 30°C to 46°C, 30°C to 47°C, 30°C to 48°C, 30°C to 49°C, 30°C to 50°C, 32°C to 44°C, 32°C to 45°C, 32°C to 46°C, 32°C to 47°C, 32°C to 48°C, 32°C to 49°C, 32°C to 50°C, 34°C to 44°C, 34°C to 45°C, 34°C to 46°C, 34°C to 47°C, 34°C to 48°C, 34°C to 49°C, 34°C to 50°C, 36°C to 44°C, 36°C to 45°C, 36°C to 46°C, 36°C to 47°C, 36°C to 48°C, 36°C to 49°C, 36°C to 50°C, 38°C to 44°C, 38°C to 45°C, 38°C to 46°C, 38°C to 47°C, 38°C to 48°C, 38°C to 49°C, 38°C to 50°C, 40°C to 44°C, 40°C to 45°C, 40°C to 46°C, 40°C to 47°C, 40°C to 48°C, 40°C to 49°C, 40°C to 50°C, 42°C to 44°C, 42°C to 45°C, 42°C to 46°C, 42°C to 47°C, 42°C to 48°C, 42°C to 49°C, or 42°C to 50°C.

A solid solution pharmaceutical composition disclosed herein may comprise a pharmaceutically-acceptable room temperature solid lipid (hard fat) in an amount of 30% to 75% by weight.

Commercially available mixtures of pharmaceutically-acceptable glycerolipids include, without limitation, Cocoa butter, mixtures of PEG-6 sterate and ethylene glycol palmitostearate and PEG-32 stearate (TEFOSE® 1500; TEFOSE® 63), mixtures of triceteareth-4 phosphate and ethylene glycol palmitostearate and diethylene glycol palmitostearate (SEDEFOS® 75), mixtures of glycerol monostearate and PEG-75 stearate (GELOT®), mixtures of cetyl alcohol and ethoxylated fatty alcohols (seteth-2-, steareth-20) (EMULCIRE®), mixtures of saturated C₁₀-C₁₈ triglycerides having a melting point around 33°C (GELUCIRE® 33/01), mixtures of saturated C₁₀-C₁₈ triglycerides having a melting point around 39°C (GELUCIRE® 39/01), mixtures of saturated C₁₀-C₁₈ triglycerides having a melting point around 43°C (GELUCIRE® 43/01), mixtures of glycerol monostearate 40-55 (type I) and diglycerides (GELEOL® Mono and Diglycerides), and mixtures of medium-chain triglycerides (LABRAFAC® Lipophile WL 1349). Only room temperature solid lipids comprising a triglyceride defined by the claim scope are part of the invention.

A hard fat useful in the pharmaceutical compositions disclosed herein may be a pharmaceutically-acceptable glycol fatty acid ester. A pharmaceutically-acceptable glycol fatty acid ester can be a monoester of a glycol, a diester of a glycol, or a triester of a glycol. A glycol fatty acid ester include, without limitation, an ethylene glycol fatty acid ester, a diethylene glycol fatty acid ester, a propylene glycol fatty acid ester, and a dipropylene fatty acid ester. Non-limiting examples of glycol fatty acid esters include, *e.g.,* ethelene glycol caprylate, ethelene glycol pelargonate, ethelene glycol caprate, ethelene glycol undecylate, ethelene glycol laurate, ethelene glycol tridecylate, ethelene glycol myristate, ethelene glycol myristolate, ethelene glycol pentadecyclate, ethelene glycol palmitate, ethelene glycol palmitoleate, ethelene glycol sapienate, ethelene glycol margarate, ethelene glycol stearate, ethelene glycol palmitostearate, ethelene glycol oleate, ethelene glycol elaidate, ethelene glycol vaccinate, ethelene glycol linoleate, ethelene glycol linoelaidate, ethelene glycol α-linolenate, ethelene glycol γ-linolenate, ethelene glycol stearidonate, ethelene glycol capprylocaprate, ethelene glycol dicapprylocaprate, diethelene glycol caprylate, diethelene glycol pelargonate, diethelene glycol caprate, diethelene glycol undecylate, diethelene glycol laurate, diethelene glycol tridecylate, diethelene glycol myristate, diethelene glycol myristolate, diethelene glycol pentadecyclate, diethelene glycol palmitate, diethelene glycol palmitoleate, diethelene glycol sapienate, diethelene glycol margarate, diethelene glycol stearate, diethelene glycol palmitostearate, diethelene glycol oleate, diethelene glycol elaidate, diethelene glycol vaccinate, diethelene glycol linoleate, diethelene glycol linoelaidate, diethelene glycol α-linolenate, diethelene glycol γ-linolenate, diethelene glycol stearidonate, diethelene glycol capprylocaprate, diethelene glycol dicapprylocaprate, propylene glycol caprylate, propylene glycol pelargonate, propylene glycol caprate, propylene glycol undecylate, propylene glycol laurate, propylene glycol tridecylate, propylene glycol myristate, propylene glycol myristolate, propylene glycol pentadecyclate, propylene glycol palmitate, propylene glycol palmitoleate, propylene glycol sapienate, propylene glycol margarate, propylene glycol stearate, propylene glycol palmitostearate, propylene glycol oleate, propylene glycol elaidate, propylene glycol vaccinate, propylene glycol linoleate, propylene glycol linoelaidate, propylene glycol α-linolenate, propylene glycol γ-linolenate, propylene glycol stearidonate, propylene glycol capprylocaprate, propylene glycol dicapprylocaprate, dipropylene glycol caprylate, dipropylene glycol pelargonate, dipropylene glycol caprate, dipropylene glycol undecylate, dipropylene glycol laurate, dipropylene glycol tridecylate, dipropylene glycol myristate, dipropylene glycol myristolate, dipropylene glycol pentadecyclate, dipropylene glycol palmitate, dipropylene glycol palmitoleate, dipropylene glycol sapienate, dipropylene glycol margarate, dipropylene glycol stearate, dipropylene glycol palmitostearate, dipropylene glycol oleate, dipropylene glycol elaidate, dipropylene glycol vaccinate, dipropylene glycol linoleate, dipropylene glycol linoelaidate, dipropylene glycol α-linolenate, dipropylene glycol γ-linolenate, dipropylene glycol stearidonate, dipropylene glycol capprylocaprate, dipropylene glycol dicapprylocaprate, or any combination thereof. Only room temperature solid lipids comprising a triglyceride defined by the claim scope are part of the invention.

Commercially available pharmaceutically-acceptable glycol fatty acid esters include, without limitation, propylene glycol monopalmitostearate (MONOSTEOL®), propylene glycol dicaprylocaprate (LABRAFAC® PG), propylene glycol monolaurate (type I) (LAUROGLYCOL® FCC), propylene glycol monolaurate (type II) (LAUROGLYCOL® 90), propylene glycol monocaprylate (type I) (CAPRYOL® PGMC), and propylene glycol monocaprylate (type II) (CAPRYOL® 90).

Another lipid used in the pharmaceutical compositions disclosed herein is a pharmaceutically-acceptable room temperature liquid lipid. Also known as a "liquid fat", a room temperature liquid lipid includes any fatty acid that is liquid at normal room temperature, i.e. 20°C. Room temperature liquid lipid comprise a wide group of compounds that are generally soluble in organic solvents and generally insoluble in water. Examples of mixtures of pharmaceutically-acceptable room temperature liquid lipids include, without limitation, a mixture of one or more fatty acids disclosed herein, a mixture of one or more partially hydrolyzed fat, and a mixture of one or more partially hydrogenated fat.

A pharmaceutically-acceptable room temperature liquid lipid includes a pharmaceutically-acceptable partially hydrogenated fat. The process of hydrogenation adds hydrogen atoms to unsaturated lipid, eliminating double bonds and making them into partially or completely saturated lipid. Partial hydrogenation is a chemical rather than enzymatic, that converts a part of cis-isomers into trans-unsaturated lipids instead of hydrogenating them completely. In the first reaction step, one hydrogen is added, with the other, coordinatively unsaturated, carbon being attached to the catalyst. The second step is the addition of hydrogen to the remaining carbon, producing a saturated fatty acid. The first step is reversible, such that the hydrogen is readsorbed on the catalyst and the double bond is re-formed. The intermediate with only one hydrogen added contains no double bond and can freely rotate. Thus, the double bond can re-form as either cis or trans, of which trans is favored, regardless the starting material.

A solid solution pharmaceutical composition disclosed herein comprises a room temperature liquid lipid in an amount in a range of 1% to 20% by weight, such as e.g., 1% to 10% by weight, 2% to 20% by weight, 2% to 10% by weight, 4% to 20% by weight, 4% to 10% by weight, 6% to 20% by weight, 6% to 10% by weight, 8% to 20% by weight, 8% to 15% by weight, or 8% to 12% by weight.

Examples of a pharmaceutically-acceptable room temperature liquid lipids include monoglycerides including, without limitation, glycerol monomyristoleate, glycerol monopalmitoleate, glycerol monosapienate, glycerol monooleate, glycerol monoelaidate, glycerol monovaccenate, glycerol monolinoleate, glycerol monolinoelaidate, glycerol monolinolenate, glycerol monostearidonate, glycerol monoeicosenoate, glycerol monomeadate, glycerol monoarachidonate, glycerol monoeicosapentaenoate, glycerol monoerucate, glycerol monodocosahexaenoate, and glycerol mononervonate.

Commercially available pharmaceutically-acceptable room temperature liquid lipids include, without limitation, glyceryl dibehenate (COMPRITOL® 888), glycerol behenate (COMPRITOL® E ATO), glycerol dipalmitostearate (Biogapress Vegetal BM297ATO), glycerol distearate (type I) (PRECIROL® ATO 5), and glycerol monolinoleate (MAISINE™ 35-1).

Aspects of the present specification disclose, in part, a stabilizing agent. A stability agent is a compound that interacts with a free acid or base present on a therapeutic compound disclosed herein to shield the charges, thereby impeding ionic interactions between therapeutic compound/lipid matrices preventing the alignments necessary to form a crystalline matrix of a solid phase composition. Thus, a stabilizing agent prevent the thermodynamic transition of a composition into a classic solid phase or prolongs this transition to such an extent that it does not occur. Examples of stability agents include a liquid glycol polymer, a monohydric alcohol, isosorbide dimethyl ether, and diethylene glycol monoethyl ether (2-(2-ethoxyethoxy)ethanol) (TRANSCUTOL®). In the claims, the stabilizing agent is defined to comprise a liquid glycol polymer.

A pharmaceutical composition disclosed herein may comprise a stabilizing agent in an amount sufficient to stabilize the free acid or base present in a therapeutic compound disclosed herein. In other aspects of this embodiment, a pharmaceutical composition disclosed herein may comprise a stabilizing agent in an amount of, *e.g.,* less than 40% by weight, less than 35% by weight, less than 30% by weight, less than 25% by weight, less than 20% by weight, less than 19% by weight, less than 18% by weight, less than 17% by weight, less than 16% by weight, less than 15% by weight, less than 14% by weight, less than 13% by weight, less than 12% by weight, less than 11% by weight, less than 10% by weight, less than 9% by weight, less than 8% by weight, less than 7% by weight, less than 6% by weight, less than 5% by weight, less than 4% by weight, less than 3% by weight, less than 2% by weight, or less than 1%. In other aspects of this embodiment, a pharmaceutical composition disclosed herein may comprise a stabilizing agent in an amount of, *e.g.,* 1% to 5% by weight, 1% to 7% by weight, 1% to 10% by weight, 1% to 12% by weight, 1% to 15% by weight, 1% to 18% by weight, 1% to 20% by weight, 2% to 5% by weight, 2% to 7% by weight, 2% to 10% by weight, 2% to 12% by weight, 2% to 15% by weight, 2% to 18% by weight, 2% to 20% by weight, 3% to 5% by weight, 3% to 7% by weight, 3% to 10% by weight, 3% to 12% by weight, 3% to 15% by weight, 3% to 18% by weight, 3% to 20% by weight, 4% to 5% by weight, 4% to 7% by weight, 4% to 10% by weight, 4% to 12% by weight, 4% to 15% by weight, 4% to 18% by weight, 4% to 20% by weight, 5% to 7% by weight, 5% to 10% by weight, 5% to 12% by weight, 5% to 15% by weight, 5% to 18% by weight, 5% to 20% by weight, 6% to 7% by weight, 6% to 10% by weight, 6% to 12% by weight, 6% to 15% by weight, 6% to 18% by weight, 6% to 20% by weight, 7% to 10% by weight, 7% to 12% by weight, 7% to 15% by weight, 7% to 18% by weight, 7% to 20% by weight, 8% to 10% by weight, 8% to 12% by weight, 8% to 15% by weight, 8% to 18% by weight, 8% to 20% by weight, 9% to 10% by weight, 9% to 12% by weight, 9% to 15% by weight, 9% to 18% by weight, 9% to 20% by weight, 10% to 12% by weight, 10% to 15% by weight, 10% to 18% by weight, or 10% to 20% by weight.

A stability agent as disclosed herein is not a solvent as it is used in an amount that does not result in substantial dissolving of a solute. As such, the amount stability agent used in a solid solution composition disclosed herein results in no more than 85% dissolution of a therapeutic compound disclosed herein. In aspects of this embodiment, he amount stability agent used in a solid solution composition disclosed herein results in. *e.g.,* no more than 80%, no more than 75%, no more than 70%, no more than 65%, no more than 60%, no more than 55%, no more than 50%, no more than 45%, no more than 40%, no more than 35%, no more than 30%, no more than 25%, no more than 20%, no more than 15%, no more than 10%, or no more than 5% dissolution of a therapeutic compound disclosed herein.

In an embodiment, a glycol polymer may comprise a pharmaceutically-acceptable PEG polymer. PEG polymers, also known as polyethylene oxide (PEO) polymers or polyoxyethylene (POE) polymers, are prepared by polymerization of ethylene oxide and are commercially available over a wide range of molecular weights from 100 g/mol to 10,000,000 g/mol. PEG polymers with a low molecular mass are liquids or low-melting solids, whereas PEG polymers of a higher molecular mass are solids. In an aspect of this embodiment, a PEG polymer used as a stability agent is a liquid PEG polymer. In aspects of this embodiment, a PEG polymer has a molecular weight of, *e.g.*, no more than 100 g/mol, no more than 200 g/mol, no more than 300 g/mol, no more than 400 g/mol, no more than 500 g/mol, no more than 600 g/mol, no more than 700 g/mol, no more than 800 g/mol, no more than 900 g/mol, or no more than 1000 g/mol.

A PEG polymer include, without limitation, PEG 100, PEG 200, PEG 300, PEG 400, PEG 500, PEG 600, PEG 700, PEG 800, PEG 900, PEG 1000, PEG 1100, PEG 1200, PEG 1300, PEG 1400, PEG 1500, PEG 1600, PEG 1700, PEG 1800, PEG 1900, PEG 2000, PEG 2100, PEG 2200, PEG 2300, PEG 2400, PEG 2500, PEG 2600, PEG 2700, PEG 2800, PEG 2900, PEG 3000, PEG 3250, PEG 3350, PEG 3500, PEG 3750, PEG 4000, PEG 4250, PEG 4500, PEG 4750, PEG 5000, PEG 5500, PEG 6000, PEG 6500, PEG 7000, PEG 7500, PEG 8000, PEG 8500, PEG 9000, PEG 9500, PEG 10,000, PEG 11,000, PEG 12,000, PEG 13,000, PEG 14,000, PEG 15,000, PEG 16,000, PEG 17,000, PEG 18,000, PEG 19,000, or PEG 20,000.

In another embodiment, a glycol polymer may comprise a pharmaceutically-acceptable polypropylene glycol (PPG) polymer. PPG polymers, also known as polypropylene oxide (PPO) polymers or polyoxypropylene (POP) polymers, are prepared by polymerization of propylene oxide and are commercially available over a wide range of molecular weights from 100 g/mol to 10,000,000 g/mol. PPG polymers with a low molecular mass are liquids or low-melting solids, whereas PPG polymers of a higher molecular mass are solids. In an aspect of this embodiment, a PPG polymer used as a stability agent is a liquid PPG polymer. In aspects of this embodiment, a PPG polymer has a molecular weight of, *e.g.,* no more than 100 g/mol, no more than 200 g/mol, no more than 300 g/mol, no more than 400 g/mol, no more than 500 g/mol, no more than 600 g/mol, no more than 700 g/mol, no more than 800 g/mol, no more than 900 g/mol, or no more than 1000 g/mol.

A PPG polymer include, without limitation, PPG 100, PPG 200, PPG 300, PPG 400, PPG 500, PPG 600, PPG 700, PPG 800, PPG 900, PPG 1000, PPG 1100, PPG 1200, PPG 1300, PPG 1400, PPG 1500, PPG 1600, PPG 1700, PPG 1800, PPG 1900, PPG 2000, PPG 2100, PPG 2200, PPG 2300, PPG 2400, PPG 2500, PPG 2600, PPG 2700, PPG 2800, PPG 2900, PPG 3000, PPG 3250, PPG 3350, PPG 3500, PPG 3750, PPG 4000, PPG 4250, PPG 4500, PPG 4750, PPG 5000, PPG 5500, PPG 6000, PPG 6500, PPG 7000, PPG 7500, PPG 8000, PPG 8500, PPG 9000, PPG 9500, PPG 10,000, PPG 11,000, PPG 12,000, PPG 13,000, PPG 14,000, PPG 15,000, PPG 16,000, PPG 17,000, PPG 18,000, PPG 19,000, or PPG 20,000.

A monohydric alcohol may also be used as a stability agent. In aspects of this embodiment, the monohydric alcohol may be, *e.g.,* a C₂₋₄ alcohol, a C₁₋₄ alcohol, a C₁₋₅ alcohol, a C₁₋₇ alcohol, a C₁₋₁₀ alcohol, a C₁₋₁₅ alcohol, or a C₁₋₂₀ alcohol. Examples of a monohydric alcohol include, without limitation, methanol, ethanol, propanol, butanol, pentanol, and 1-hexadecanol.

Aspects of the present specification disclose, in part, a neutralizing agent. A neutralizing agent is a compound that interacts with a therapeutic compound disclosed herein that is a salt to neutralized the ionic charges produced when the therapeutic compound dissolves, thereby impeding ionic interactions between therapeutic compound/lipid matrices preventing the alignments necessary to form a crystalline matrix of a solid phase composition. Thus, a neutralizing agent prevent the thermodynamic transition of a composition into a classic solid phase or prolongs this transition to such an extent that it does not occur. Examples of neutralizing agents include fatty acids as disclosed herein for base-salt drugs and sodium acetate or triethanolamine for acid-salt drugs.

The amount of neutralizing agent used is based upon the extent of charge neutralization desired. For complete neutralization, one molar equivalent of neutralizing agent relative to therapeutic compound is added to the formulation. For partial neutralization, less than one molar equivalent is added. Partial neutralization is advantageous in producing a sustained release formulation. Upon administration, a portion of the therapeutic compound is immediately made available to the body (instant bioavailability) while the bioavailability of another portion is delayed until the therapeutic compound is neutralized by the neutralizing agent. A neutralizing agent may also be added in an excessive amount, i.e., more than one molar equivalent relative to therapeutic compound. Besides neutralizing the salt-drug, excessive amounts of neutralizing agent can also enable adjustments to the melting point of the solid solution composition.

In aspects of this embodiment, a pharmaceutical composition disclosed herein may comprise a neutralizing agent in an amount of, *e.g.,* at least 5 mg, at least 10 mg, at least 15 mg, at least 20 mg, at least 25 mg, at least 30 mg, at least 35 mg, at least 40 mg, at least 45 mg, at least 50 mg, at least 55 mg, at least 60 mg, at least 65 mg, at least 70 mg, at least 75 mg, at least 80 mg, at least 85 mg, at least 90 mg, at least 95 mg, or at least 100 mg. In other aspects of this embodiment, a pharmaceutical composition disclosed herein may comprise a neutralizing agent in an amount of, *e.g.,* at least 5 mg, at least 10 mg, at least 20 mg, at least 25 mg, at least 50 mg, at least 75 mg, at least 100 mg, at least 200 mg, at least 300 mg, at least 400 mg, at least 500 mg, at least 600 mg, at least 700 mg, at least 800 mg, at least 900 mg, at least 1,000 mg, at least 1,100 mg, at least 1,200 mg, at least 1,300 mg, at least 1,400 mg, or at least 1,500 mg. In yet other aspects of this embodiment, a pharmaceutical composition disclosed herein may comprise a neutralizing agent in an amount of, *e.g.,* 5 mg to 100 mg, 10 mg to 100 mg, 50 mg to 150 mg, 100 mg to 250 mg, 150 mg to 350 mg, 250 mg to 500 mg, 350 mg to 600 mg, 500 mg to 750 mg, 600 mg to 900 mg, 750 mg to 1,000 mg, 850 mg to 1,200 mg, or 1,000 mg to 1,500 mg. In still other aspects of this embodiment, a pharmaceutical composition disclosed herein may comprise a neutralizing agent in an amount of, *e.g.,* 10 mg to 250 mg, 10 mg to 500 mg, 10 mg to 750 mg, 10 mg to 1,000 mg, 10 mg to 1,500 mg, 50 mg to 250 mg, 50 mg to 500 mg, 50 mg to 750 mg, 50 mg to 1,000 mg, 50 mg to 1,500 mg, 100 mg to 250 mg, 100 mg to 500 mg, 100 mg to 750 mg, 100 mg to 1,000 mg, 100 mg to 1,500 mg, 200 mg to 500 mg, 200 mg to 750 mg, 200 mg to 1,000 mg, 200 mg to 1,500 mg, 5 mg to 1,500 mg, 5 mg to 1,000 mg, or 5 mg to 250 mg.

In other aspects of this embodiment, a pharmaceutical composition disclosed herein may comprise a neutralizing agent in an amount of, *e.g.,* less than 90% by weight, less than 80% by weight, less than 70% by weight, less than 65% by weight, less than 60% by weight, less than 55% by weight, less than 50% by weight, less than 45% by weight, less than 40% by weight, less than 35% by weight, less than 30% by weight, less than 25% by weight, less than 20% by weight, less than 15% by weight, less than 10% by weight, less than 5% by weight, or less than 1% by weight. In other aspects of this embodiment, a pharmaceutical composition disclosed herein may comprise a neutralizing agent in an amount of, *e.g.,* 1% to 90% by weight, 1% to 80% by weight, 1% to 75% by weight, 1% to 70% by weight, 1% to 65% by weight, 1% to 60% by weight, 1% to 55% by weight, 1% to 50% by weight, 1% to 45% by weight, 1% to 40% by weight, 1% to 35% by weight, 1% to 30% by weight, 1% to 25% by weight, 1% to 20% by weight, 1% to 15% by weight, 1% to 10% by weight, 1% to 5% by weight, 2% to 50% by weight, 2% to 40% by weight, 2% to 30% by weight, 2% to 20% by weight, 2% to 10% by weight, 4% to 50% by weight, 4% to 40% by weight, 4% to 30% by weight, 4% to 20% by weight, 4% to 10% by weight, 6% to 50% by weight, 6% to 40% by weight, 6% to 30% by weight, 6% to 20% by weight, 6% to 10% by weight, 8% to 50% by weight, 8% to 40% by weight, 8% to 30% by weight, 8% to 20% by weight, 8% to 15% by weight, or 8% to 12% by weight.

In other aspects of this embodiment, a pharmaceutical composition disclosed herein may comprise a neutralizing agent in an amount of, *e.g.,* 0.1% to 45% by weight, 0.1% to 40% by weight, 0.1% to 35% by weight, 0.1% to 30% by weight, 0.1% to 25% by weight, 0.1% to 20% by weight, 0.1% to 15% by weight, 0.1% to 10% by weight, 0.1% to 5% by weight, 1% to 45% by weight, 1% to 40% by weight, 1% to 35% by weight, 1% to 30% by weight, 1% to 25% by weight, 1% to 20% by weight, 1% to 15% by weight, 1% to 10% by weight, 1% to 5% by weight, 5% to 45% by weight, 5% to 40% by weight, 5% to 35% by weight, 5% to 30% by weight, 5% to 25% by weight, 5% to 20% by weight, 5% to 15% by weight, 5% to 10% by weight, 10% to 45% by weight, 10% to 40% by weight, 10% to 35% by weight, 10% to 30% by weight, 10% to 25% by weight, 10% to 20% by weight, 10% to 15% by weight, 15% to 45% by weight, 15% to 40% by weight, 15% to 35% by weight, 15% to 30% by weight, 15% to 25% by weight, 15% to 20% by weight, 20% to 45% by weight, 20% to 40% by weight, 20% to 35% by weight, 20% to 30% by weight, 20% to 25% by weight, 25% to 45% by weight, 25% to 40% by weight, 25% to 35% by weight, or 25% to 30% by weight.

A pharmaceutical composition disclosed herein may optionally include a pharmaceutically-acceptable carrier that facilitates processing of an active ingredient into pharmaceutically-acceptable compositions. As used herein, the term "pharmacologically-acceptable carrier" is synonymous with "pharmacological carrier" and means any carrier that has substantially no long term or permanent detrimental effect when administered and encompasses terms such as "pharmacologically acceptable vehicle, stabilizer, diluent, additive, auxiliary or excipient." Such a carrier generally is mixed with an active compound or permitted to dilute or enclose the active compound and can be a solid, semi-solid, or liquid agent. It is understood that the active ingredients can be soluble or can be delivered as a suspension in the desired carrier or diluent. Any of a variety of pharmaceutically acceptable carriers can be used including, without limitation, aqueous media such as, e.g., water, saline, glycine, hyaluronic acid and the like; solid carriers such as, e.g., mannitol, lactose, starch, magnesium stearate, sodium saccharin, talcum, cellulose, glucose, sucrose, magnesium carbonate, and the like; solvents; dispersion media; coatings; antibacterial and antifungal agents; isotonic and absorption delaying agents; or any other inactive ingredient. Selection of a pharmacologically acceptable carrier can depend on the mode of administration. Except insofar as any pharmacologically acceptable carrier is incompatible with the active ingredient, its use in pharmaceutically acceptable compositions is contemplated. Non-limiting examples of specific uses of such pharmaceutical carriers can be found in Pharmaceutical Dosage Forms and Drug Delivery Systems (Howard C. Ansel et al., eds., Lippincott Williams & Wilkins Publishers, 7th ed. 1999); REMINGTON: THE SCIENCE AND PRACTICE OF PHARMACY (Alfonso R. Gennaro ed., Lippincott, Williams & Wilkins, 20th ed. 2000); Goodman & Gilman's The Pharmacological Basis of Therapeutics (Joel G. Hardman et al., eds., McGraw-Hill Professional, 10th ed. 2001); and Handbook of Pharmaceutical Excipients (Raymond C. Rowe et al., APhA Publications, 4th edition 2003). These protocols are routine procedures and any modifications are well within the scope of one skilled in the art and from the teaching herein.

A pharmaceutical composition disclosed herein can optionally include, without limitation, other pharmaceutically acceptable components (or pharmaceutical components), including, without limitation, buffers, preservatives, tonicity adjusters, salts, antioxidants, osmolality adjusting agents, physiological substances, pharmacological substances, bulking agents, emulsifying agents, wetting agents, sweetening or flavoring agents, and the like. Various buffers and means for adjusting pH can be used to prepare a pharmaceutical composition disclosed herein, provided that the resulting preparation is pharmaceutically acceptable. Such buffers include, without limitation, acetate buffers, citrate buffers, phosphate buffers, neutral buffered saline, phosphate buffered saline and borate buffers. It is understood that acids or bases can be used to adjust the pH of a composition as needed. Pharmaceutically acceptable antioxidants include, without limitation, sodium metabisulfite, sodium thiosulfate, acetylcysteine, butylated hydroxyanisole and butylated hydroxytoluene. Useful preservatives include, without limitation, benzalkonium chloride, chlorobutanol, thimerosal, phenylmercuric acetate, phenylmercuric nitrate, a stabilized oxy chloro composition and chelants, such as, e.g., DTPA or DTPA-bisamide, calcium DTPA, and CaNaDTPA-bisamide. Tonicity adjustors useful in a pharmaceutical composition include, without limitation, salts such as, e.g., sodium chloride, potassium chloride, mannitol or glycerin and other pharmaceutically acceptable tonicity adjustor. The pharmaceutical composition may be provided as a salt and can be formed with many acids, including but not limited to, hydrochloric, sulfuric, acetic, lactic, tartaric, malic, succinic, etc. Salts tend to be more soluble in aqueous or other protonic solvents than are the corresponding free base forms. It is understood that these and other substances known in the art of pharmacology can be included in a pharmaceutical composition.

The pharmaceutical composition of the invention is formulated to be a solid at a temperature of, 30°C or lower, 29°C or lower, 28°C or lower, 27°C or lower, 26°C or lower, 25°C or lower, 24°C or lower, 23°C or lower, 22°C or lower, 21°C or lower, 20°C or lower, 19°C or lower, 18°C or lower, 17°C or lower, 16°C or lower, 15°C or lower, 14°C or lower, 13°C or lower, 12°C or lower, 11°C or lower, 10°C or lower, 9°C or lower, 8°C or lower, 7°C or lower, 6°C or lower, 5°C or lower, 4°C or lower, 3°C or lower, 2°C or lower, 1°C or lower, or 0°C or lower.

Accordingly, the composition of the invention has a melting point temperature of 30°C or higher, 31°C or higher, 32°C or higher, 33°C or higher, 34°C or higher, 35°C or higher, 36°C or higher, or 37°C or higher, such as being in the range of 30°C to 32°C, 31°C to 33°C, 32°C to 34°C, 33°C to 35°C, 34°C to 36°C, or 35°C to 37°C, or in the range of 30°C to 34°C, 32°C to 36°C, or 34°C to 38°C.

Also disclosed herein, but not part of the invention, is, in part, a method of preparing a solid solution pharmaceutical composition disclosed herein comprises the steps of a) contacting a therapeutic compound disclosed herein with one or more room temperature liquid lipids under conditions which allow the therapeutic compound to dissolve in the one or more lipids; and b) contacting the compound/lipid solution with one or more room temperature solid lipids disclosed herein under conditions which allow the formation of a solid solution composition.

A method disclosed herein comprises the steps of a) contacting a therapeutic compound disclosed herein with one or more room temperature liquid lipids and one or more stabilizing agents under conditions which allow the therapeutic compound to dissolve in the one or more lipids; and b) contacting the compound/lipid solution with one or more room temperature solid lipids disclosed herein under conditions which allow the formation of a solid solution composition.

A method disclosed herein comprises the steps of a) contacting a therapeutic compound disclosed herein with one or more room temperature liquid lipids and one or more neutralizing agents under conditions which allow the therapeutic compound to dissolve in the one or more lipids; and b) contacting the compound/lipid solution with one or more room temperature solid lipids disclosed herein under conditions which allow the formation of a solid solution composition.

In one embodiment, a method disclosed herein comprises the steps of a) contacting a therapeutic compound disclosed herein with one or more room temperature liquid lipids, one or more stabilizing agents, and one or more neutralizing agents under conditions which allow the therapeutic compound to dissolve in the one or more lipids; and b) contacting the compound/lipid solution with one or more room temperature solid lipids disclosed herein under conditions which allow the formation of a solid solution composition.

The method disclosed herein is carried out under conditions which allow the therapeutic compound to dissolve in other components. A method disclosed herein may be carried out at a temperature sufficient to dissolve the therapeutic compound into the one or more room temperature liquid lipids, and/or one or more stabilizing agents, and/or one or more neutralizing agents to create a solution. In addition, a method disclosed herein comprises heating a mixture comprising a therapeutic compound disclosed herein to a temperature of, *e.g.,* 40°C, 45°C, 50°C, 55°C, 60°C, 65°C, 70°C, or 75°C. In addition a method disclosed herein comprises heating a mixture comprising a therapeutic compound disclosed herein to a temperature of, *e.g.,* at least 40°C, at least 45°C, at least 50°C, at least 55°C, at least 60°C, at least 65°C, at least 70°C, or at least 75°C. In addition a method disclosed herein comprises heating a mixture comprising a therapeutic compound disclosed herein to a temperature of, *e.g.*, at most 40°C, at most 45°C, at most 50°C, at most 55°C, at most 60°C, at most 65°C, at most 70°C, or at most 75°C. In addition a method disclosed herein comprises heating a mixture comprising a therapeutic compound disclosed herein to a temperature of, *e.g.,* 40°C to 45°C, 40°C to 50°C, 40°C to 55°C, 40°C to 60°C, 40°C to 65°C, 40°C to 70°C, 40°C to 75°C, 45°C to 50°C, 45°C to 55°C, 45°C to 60°C, 45°C to 65°C, 45°C to 70°C, 45°C to 75°C, 50°C to 55°C, 50°C to 60°C, 50°C to 65°C, 50°C to 70°C, 50°C to 75°C, 55°C to 60°C, 55°C to 65°C, 55°C to 70°C, 55°C to 75°C, 60°C to 65°C, 60°C to 70°C, 60°C to 75°C, 65°C to 70°C, 65°C to 75°C, or 70°C to 75°C.

A method disclosed herein may be carried out at a temperature sufficient to incorporate the one or more room temperature solid lipids into the solution comprising the therapeutic compound. In addition, a method disclosed herein comprises heating a mixture comprising a therapeutic compound disclosed herein to a temperature of, *e.g.,* 40°C, 45°C, 50°C, 55°C, 60°C, 65°C, 70°C, or 75°C in order to incorporate the one or more room temperature solid lipids. In addition, a method disclosed herein comprises heating a mixture comprising a therapeutic compound disclosed herein to a temperature of, *e.g.,* at least 40°C, at least 45°C, at least 50°C, at least 55°C, at least 60°C, at least 65°C, at least 70°C, or at least 75°C in order to incorporate the one or more room temperature solid lipids. In addition a method disclosed herein comprises heating a mixture comprising a therapeutic compound disclosed herein to a temperature of, *e.g.,* at most 40°C, at most 45°C, at most 50°C, at most 55°C, at most 60°C, at most 65°C, at most 70°C, or at most 75°C in order to incorporate the one or more room temperature solid lipids. In addition a method disclosed herein comprises heating a mixture comprising a therapeutic compound disclosed herein to a temperature of, *e.g.,* 40°C to 45°C, 40°C to 50°C, 40°C to 55°C, 40°C to 60°C, 40°C to 65°C, 40°C to 70°C, 40°C to 75°C, 45°C to 50°C, 45°C to 55°C, 45°C to 60°C, 45°C to 65°C, 45°C to 70°C, 45°C to 75°C, 50°C to 55°C, 50°C to 60°C, 50°C to 65°C, 50°C to 70°C, 50°C to 75°C, 55°C to 60°C, 55°C to 65°C, 55°C to 70°C, 55°C to 75°C, 60°C to 65°C, 60°C to 70°C, 60°C to 75°C, 65°C to 70°C, 65°C to 75°C, or 70°C to 75°C in order to incorporate the one or more room temperature solid lipids.

Aspects of the present specification disclose, but are not claimed, in part, contacting a therapeutic compound disclosed herein with one or more lipids. In aspects of this embodiment, one or more lipids includes one, two, three, four, or five different lipids disclosed herein. In other aspects of this embodiment, one or more lipids includes two or more, three or more, four or more, or five or more different lipids disclosed herein. In yet other aspects of this embodiment, one or more lipids includes one to five different lipids disclosed herein, two to five different lipids disclosed herein, three to five different lipids disclosed herein, one to four different lipids disclosed herein, two to four different lipids disclosed herein, or two to three different lipids disclosed herein.

In other aspects of this embodiment, one or more lipids includes one or more room temperature solid lipids and one or more room temperature liquid lipids. In aspects of this embodiment, one or more lipids includes one, two, three, four, or five different hard fats disclosed herein and one, two, three, four, or five different room temperature liquid lipids disclosed herein. In other aspects of this embodiment, one or more lipids includes two or more, three or more, four or more, or five or more different room temperature solid lipids disclosed herein and two or more, three or more, four or more, or five or more different room temperature liquid lipids disclosed herein. In yet other aspects of this embodiment, one or more lipids includes one to five different hard fats disclosed herein, two to five different room temperature solid lipids disclosed herein, three to five different room temperature solid lipids disclosed herein, one to four different room temperature solid lipids disclosed herein, two to four different room temperature solid lipids disclosed herein, ortwo to three different room temperature solid lipids disclosed herein and one to five different room temperature liquid lipids disclosed herein, two to five different room temperature liquid lipids disclosed herein, three to five different room temperature liquid lipids disclosed herein, one to four different room temperature liquid lipids disclosed herein, two to four different room temperature liquid lipids disclosed herein, ortwo to three different room temperature liquid lipids disclosed herein.

A method disclosed herein may use a room temperature solid lipid and a room temperature liquid lipid in a ratio of solid lipid:liquid lipid of, e.g., at least 1:1, at least 2:1, at least 3:1, at least 4:1, at least 5:1, at least 6:1, at least 7:1, at least 8:1, at least 9:1, at least 10:1, at least 15:1, or at least 20:1. In addition the method disclosed herein may use a room temperature solid lipid and a room temperature liquid lipid in a ratio of solid lipid:liquid lipid of, *e.g.,* 1:1 to 20:1, 5:1 to 20:1, 2:1 to 15:1, 5:1 to 15:1, 4:1 to 12:1, or6:1 to 10:1.

A method disclosed herein may use a plurality of room temperature solid lipids and a plurality of room temperature liquid lipids in a ratio of total solid lipid:total liquid lipid of, *e.g.,* at least 1:1, at least 2:1, at least 3:1, at least 4:1, at least 5:1, at least 6:1, at least 7:1, at least 8:1, at least 9:1, at least 10:1, at least 15:1, or at least 20:1. In addition the method disclosed herein may use a plurality of room temperature solid lipids and a plurality of room temperature liquid lipid in a ratio of total solid lipid:total liquid lipid of, *e.g.,* 1:1 to 20:1, 5:1 to 20:1, 2:1 to 15:1, 5:1 to 15:1, 4:1 to 12:1, or 6:1 to 10:1.

The contacting a therapeutic compound and one or more lipids may comprise mixing the, e.g., by stirring, inversion, sonication, or vortexing. The mixing may be carried out for, e.g., at least 1 second, at least 5 seconds, at least 10 seconds, at least 20 seconds, at least 30 seconds, at least 45 seconds, at least 60 seconds, or more, until the therapeutic compound is fully dissolved in the lipid/glycol polymer mixture.

Aspects of the present specification disclose, in part, contacting the compound/lipid solution with one or more glycol polymers disclosed herein.

The method disclosed herein is carried out under conditions which allow the solidification of a solid solution composition. In addition, the method may be carried out at a temperature sufficient to cool the composition to a temperature where the solution solidifies.

In certain methods disclosed herein, a rapid cooling step may be used to reduce the temperature of a pharmaceutical composition disclosed herein after its formation. For example, a rapid cooling step may be used in procedures were temperatures greater than room temperature are used to allow a therapeutic compound to dissolve fully in the pharmaceutically-acceptable solvent and/or to allow the solution comprising the therapeutic compound to form the pharmaceutical composition. In aspects of this method, a rapid cooling step results in a temperature decrease of, *e.g.,* 30°C in 20 minutes, 25°C in 20 minutes, 20°C in 20 minutes, 15°C in 20 minutes, 30°C in 15 minutes, 25°C in 15 minutes, 20°C in 15 minutes, 15°C in 15 minutes, 30°C in 10 minutes, 25°C in 10 minutes, 20°C in 10 minutes, 15°C in 10 minutes, 30°C in 5 minutes, 25°C in 5 minutes, 20°C in 5 minutes, 15°C in 5 minutes. In other aspects of this method, a rapid cooling step results in a temperature decrease of, *e.g.,* 20°C to 30°C in 20 minutes, 20°C to 30°C in 15 minutes, 20°C to 30°C in 10 minutes, 20°C to 30°C in 5 minutes, 15°C to 25°C in 20 minutes, 15°C to 25°C in 15 minutes, 15°C to 25°C in 10 minutes, 15°C to 25°C in 5 minutes, 10°C to 20°C in 20 minutes, 10°C to 20°C in 15 minutes, 10°C to 20°C in 10 minutes, or 10°C to 20°C in 5 minutes.

In yet further aspects of this method, a rapid cooling step results in a temperature decrease of, *e.g.,* 2.0°C/minute, 1.9°C/minute, 1.8°C/minute, 1.7°C/minute, 1.6°C/minute, 1.5°C/minute, 1.4°C/minute, 1.3°C/minute, 1.2°C/minute, 1.1°C/minute, 1.0°C/minute, 0.9°C/minute, 0.8°C/minute, 0.7°C/minute, 0.6°C/minute, 0.5°C/minute, 0.4°C/minute, 0.3°C/minute, 0.2°C/minute, or 0.1°C/minute. In still further aspects of this method, a rapid cooling step results in a temperature decrease of, *e.g.,* 0.1°C to 0.4°C/minute, 0.2°C to 0.6°C/minute, 0.4°C to 0.8°C/minute, 0.6°C to 1.0°C/minute, 0.8°C to 1.2°C/minute, 1.0°C to 1.4°C/minute, 1.2°C to 1.6°C/minute, 1.4°C to 1.8°C/minute, 1.6°C to 2.0°C/minute, 0.1°C to 0.5°C/minute, 0.5°C to 1.0°C/minute, 1.0°C to 1.5°C/minute, 1.5°C to 2.0°C/minute, 0.5°C to 1.5°C/minute, or 1.0°C to 2.0°C/minute.

A formulation disclosed herein result in the formation of a solid solution of the lipids and therapeutic compound. Such formulations do not form liposomal emulsions and/or micellular particles and/or any other type of multi-phasic compositions. In addition, such formulations do not require a hydrophilic solvent, such as, *e.g.,* water or a buffered solution. As such, a pharmaceutical composition disclosed herein need not be formulated with a hydrophilic solvent. In an embodiment, a pharmaceutical composition disclosed herein does not comprise a pharmaceutically-acceptable hydrophilic solvent.

The amount of a therapeutic compound, room temperature liquid lipid, room temperature solid lipid (hard fat), stabilizing agent and neutralizing agent used in the method disclosed herein are used in the amounts defined by the claims. Factors used to determine the amount of each component used include, without limitation, the final amount the therapeutic compound desired in the pharmaceutical composition, the desired concentration of a therapeutic compound in the solution, the hydrophobicity of the therapeutic compound, the lipophobicity of the therapeutic compound, the final amount of a pharmaceutical composition desired, and the conditions used to produce the solid solution pharmaceutical composition.

In one embodiment, which is not claimed, a pharmaceutical composition comprises: a) one or more therapeutic compounds having an anti-pain activity comprising 5% to 55% by weight of the pharmaceutical composition; b) one or more lipids that are solid at 20 °C comprising 30% to 75% by weight of the pharmaceutical composition wherein the one or more lipids have a melting point of 40°C to 50°C, and wherein the lipids comprise a triglyceride with one saturated or unsaturated fatty acid having a carbon length of C₁₂-C₂₄, two saturated or unsaturated fatty acids each having a carbon length of C₁₂-C₂₄, or three saturated or unsaturated fatty acids each having a carbon length of C₁₂-C₂₄; c) one or more lipids that are liquid at 20 °C comprising 1% to 20% by weight of the pharmaceutical composition, wherein the solid solution pharmaceutical composition has a melting point of 30°C or higher, but does not comprise a pharmaceutically-acceptable hydrophilic solvent.

In one embodiment, which is not claimed, a pharmaceutical composition comprises: a) one or more therapeutic compounds having an anti-pain activity comprising 5% to 55% by weight of the pharmaceutical composition; b) one or more lipids that are solid at 20 °C comprising 30% to 75% by weight of the pharmaceutical composition wherein the one or more lipids have a melting point of 40°C to 50°C, and wherein the lipids comprise a triglyceride with one saturated or unsaturated fatty acid having a carbon length of C₁₂-C₂₄, two saturated or unsaturated fatty acids each having a carbon length of C₁₂-C₂₄, or three saturated or unsaturated fatty acids each having a carbon length of C₁₂-C₂₄; c) one or more lipids that are liquid at 20 °C comprising 1% to 20% by weight of the pharmaceutical composition, wherein the solid solution pharmaceutical composition has a melting point of 30°C or higher, and one or more stabilizing agents, but does not comprise a pharmaceutically-acceptable hydrophilic solvent. In aspects of this embodiment, a solid solution pharmaceutical composition comprises a therapeutic compound, a room temperature solid lipid or hard fat, a room temperature liquid lipid, and a liquid glycol polymer and/or a monohydric alcohol, and/or isosorbide dimethyl ether, and/or diethylene glycol monoethyl ether (2-(2-ethoxyethoxy)ethanol), but does not comprise a pharmaceutically-acceptable hydrophilic solvent. In other aspects of this embodiment, a solid solution pharmaceutical composition comprises a therapeutic compound, a triglyceride mixture, a monoglyceride mixture, and a liquid PEG polymer and/or a C₁-C₅ monohydric alcohol, and/or isosorbide dimethyl ether, and/or diethylene glycol monoethyl ether (2-(2-ethoxyethoxy)ethanol), but does not comprise a pharmaceutically-acceptable hydrophilic solvent. In yet other aspects of this embodiment, a pharmaceutical composition comprises a therapeutic compound, GELUCIRE® 43/01 (Gattefosse), a waxy solid (as room temperature solid lipid) having a melting point of between 41°C to 45°C and comprising a mixture of saturated C₁₀-C₁₈ triglycerides, MAISINE® 35-1 (Gattefosse), a glyceryl monolinoleate, and a liquid PEG polymer and/or a C₁-C₅ monohydric alcohol, and/or isosorbide dimethyl ether, and/or diethylene glycol monoethyl ether (2-(2-ethoxyethoxy)ethanol), but does not comprise a pharmaceutically-acceptable hydrophilic solvent.

A solid solution composition disclosed herein, but which is not claimed, comprises 1% to 55% by weight of therapeutic compound, 40% to 90% by weight of room temperature solid lipid or hard fat, 1% to 10% by weight of room temperature liquid lipid, and 1% to 5% of a stabilizing agent. A version of this solid solution composition comprises 1% to 55% by weight of therapeutic compound, 40% to 90% by weight of room temperature solid lipid or hard fat, 1% to 10% by weight of room temperature liquid lipid, and 1% to 5% of a liquid glycol polymer and/or a monohydric alcohol, and/or isosorbide dimethyl ether, and/or diethylene glycol monoethyl ether (2-(2-ethoxyethoxy)ethanol). In an embodiment, a solid solution composition comprises 5% to 50% by weight of therapeutic compound, 40% to 70% by weight of room temperature solid lipid or hard fat, 1% to 7% by weight of room temperature liquid lipid, and 1% to 3% of a liquid glycol polymer and/or a monohydric alcohol, and/or isosorbide dimethyl ether, and/or diethylene glycol monoethyl ether (2-(2-ethoxyethoxy)ethanol).

In one embodiment, a pharmaceutical composition comprises one or more therapeutic compounds, one or more room temperature solid lipids or hard fats, one or more room temperature liquid lipids, and one or more neutralizing agents, but does not comprise a pharmaceutically-acceptable hydrophilic solvent. In other aspects of this embodiment, a solid solution pharmaceutical composition comprises a therapeutic compound, a room temperature solid lipid or hard fat, a room temperature liquid lipid, and a fatty acid, but does not comprise a pharmaceutically-acceptable hydrophilic solvent. In yet other aspects of this embodiment, a solid solution pharmaceutical composition comprises a therapeutic compound, a triglyceride mixture, a monoglyceride mixture, and a C₁₆-C₁₈ fatty acid, but does not comprise a pharmaceutically-acceptable hydrophilic solvent. In still other aspects of this embodiment, a pharmaceutical composition comprises a therapeutic compound, GELUCIRE® 43/01 (Gattefosse), a waxy solid (as room temperature solid lipid) having a melting point of between 41°C to 45°C and comprising a mixture of saturated C₁₀-C₁₈ triglycerides, MAISINE® 35-1 (Gattefosse), a glyceryl monolinoleate, and stearic acid, but does not comprise a pharmaceutically-acceptable hydrophilic solvent. Only the compositions defined by the claim scope are part of the invention.

A solid solution composition disclosed herein may comprise 1% to 55% by weight of therapeutic compound, 30% to 70% by weight of room temperature solid lipid or hard fat, 1% to 10% by weight of room temperature liquid lipid, and 1% to 55% of neutralizing agent. In other aspects of this embodiment, a solid solution composition comprises 1% to 55% by weight of therapeutic compound, 30% to 70% by weight of room temperature solid lipid or hard fat, 1% to 10% by weight of room temperature liquid lipid, and 1% to 55% of fatty acid. In an embodiment, a solid solution composition comprises 5% to 50% by weight of therapeutic compound, 30% to 60% by weight of room temperature solid lipid or hard fat, 1% to 7% by weight of room temperature liquid lipid, and 5% to 50% of a fatty acid.

In other aspects of this embodiment, a solid solution pharmaceutical composition comprises one or more therapeutic compounds, one or more room temperature solid lipids or hard fats, one or more room temperature liquid lipids, and triethanolamine, but does not comprise a pharmaceutically-acceptable hydrophilic solvent. In yet other aspects of this embodiment, a solid solution pharmaceutical composition comprises a therapeutic compound, a triglyceride mixture, a monoglyceride mixture, and triethanolamine, but does not comprise a pharmaceutically-acceptable hydrophilic solvent. In still other aspects of this embodiment, a pharmaceutical composition comprises a therapeutic compound, GELUCIRE® 43/01 (Gattefosse), a waxy solid (as room temperature solid lipid) having a melting point of between 41°C to 45°C and comprising a mixture of saturated C₁₀-C₁₈ triglycerides, MAISINE® 35-1 (Gattefosse), a glyceryl monolinoleate, and triethanolamine, but does not comprise a pharmaceutically-acceptable hydrophilic solvent.

A solid solution composition disclosed herein comprises 1% to 55% by weight of therapeutic compound, 30% to 70% by weight of room temperature solid lipid or hard fat, 1% to 10% by weight of room temperature liquid lipid, and 1% to 55% of triethanolamine. In an embodiment, a solid solution composition comprises 5% to 50% by weight of therapeutic compound, 30% to 60% by weight of room temperature solid lipid or hard fat, 1% to 7% by weight of room temperature liquid lipid, and 5% to 50% of a triethanolamine.

In one embodiment, a pharmaceutical composition may comprise: a) one or more therapeutic compounds having an anti-pain activity comprising 5% to 55% by weight of the pharmaceutical composition; b) one or more lipids that are solid at 20 °C comprising 30% to 75% by weight of the pharmaceutical composition wherein the one or more lipids have a melting point of 40°C to 50°C, and wherein the lipids comprise a triglyceride with one saturated or unsaturated fatty acid having a carbon length of C₁₂-C₂₄, two saturated or unsaturated fatty acids each having a carbon length of C₁₂-C₂₄, or three saturated or unsaturated fatty acids each having a carbon length of C₁₂-C₂₄; c) one or more lipids that are liquid at 20 °C comprising 1% to 20% by weight of the pharmaceutical composition, wherein the solid solution pharmaceutical composition has a melting point of 30°C or higher, but does not comprise a pharmaceutically-acceptable hydrophilic solvent.

Also disclosed herein are pharmaceutical compositions of the invention for treating an individual with a severe pain condition. Treatment with the pharmaceutical compositions of the invention comprises the step of administering to an individual in need thereof a pharmaceutical composition disclosed herein, wherein administration reduces a symptom associated with the severe pain, thereby treating the individual.

Aspects of the present specification disclose pharmaceutical compositions of the invention for treating an individual suffering from a severe pain condition. As used herein, the term "treating," refers to reducing or eliminating in an individual a clinical symptom of a severe pain condition; or delaying or preventing in an individual the onset of a clinical symptom of a severe pain condition. For example, the term "treating" can mean reducing a symptom of a condition characterized by a severe pain condition by, *e.g.,* at least 20%, at least 25%, at least 30%, at least 35%, at least 40%, at least 45%, at least 50%, at least 55%, at least 60%, at least 65%, at least 70%, at least 75%, at least 80%, at least 85%, at least 90% at least 95%, or at least 100%. The actual symptoms associated with a severe pain condition are well known and can be determined by a person of ordinary skill in the art by taking into account factors, including, without limitation, the location of the severe pain condition, the cause of the severe pain condition, the severity of the severe pain condition, and/or the tissue or organ affected by the severe pain condition. Those of skill in the art will know the appropriate symptoms or indicators associated with a specific type of severe pain condition and will know how to determine if an individual is a candidate for treatment as disclosed herein.

Pain may be measured using one or more pain thresholds. Pain thresholds are measured by gradually increasing the intensity of a stimulus such as electric current or heat applied to the body. The pain perception threshold is the point at which the stimulus begins to hurt, and the pain tolerance threshold is reached when the individual acts to stop the pain.

Pain may be measured using one or more pain scale questionnaires. Although a self-reported questionaire by an individual suffering from a severe pain condition tend to be the most reliable measure of pain, reports from health care professionals may also be used. A number of pain measurement scales have been developed including, without limitation, Alder Hey Triage Pain Score, Behavioral Pain Scale (BPS), Brief Pain Inventory (BPI), Checklist of Nonverbal Pain Indicators (CNPI), Critical-Care Pain Observation Tool (CPOT), COMFORT scale, Dallas Pain Questionnaire, Descriptor differential scale (DDS), Disease-Specific Pain Scale (DSPI), Dolorimeter Pain Index (DPI), Faces Pain Scale - Revised (FPS-R), Face Legs Activity Cry Consolability scale, Lequesne algofunctional index, Original index, McGill Pain Questionnaire (MPQ), Multidimensional Pain Inventory (MPI), Neck Pain and Disability Scale -NPAD, Numerical 11 point box (BS-11), Numeric Rating Scale (NRS-11), Pediatric Pain Questionnaire (PPQ), Roland-Morris Back Pain Questionnaire, Visual analog scale (VAS), and Wong-Baker FACES Pain Rating Scale. As one example, a NRS-11 scale is an 11-point scale for patient self-reporting that ranks the pain being experienced on a scale of 0 to 10, with 0 being no pain at all, 1-3 being mild pain that interferes little with activities of daily life, 4-6 being moderate pain that interferes significantly with activities of daily life, and 7-10 being severe pain that completely interferes with activities of daily life. Quality can be established by having the individual complete the McGill Pain Questionnaire indicating which words best describe their pain.

Severe pain condition symptoms include, without limitation, a coldness, a numbness, an itching, a paresthesias, an electric shock sensation, a burning sensation, an ice-burn sensation, dysesthesia, allodynia, hyperalgesia, hyperpathia, a somatic pain sensation, and a visceral pain sensation. The actual symptoms associated with a severe pain condition are well known and can be determined by a person of ordinary skill in the art by taking into account factors, including, without limitation, the location of the severe pain, the cause of the severe pain, the severity of the severe pain, the tissue or organ affected, and the associated disorder.

A pharmaceutical composition disclosed herein is used to treat severe pain. Pain is any unpleasant sensory and/or emotional experience associated with actual or potential tissue damage, or described in terms of such damage. Pain may occur in varying degrees of severity and is usually a consequence of an injury, a disease, or an emotional disorder, although the underlying cause may or may not be apparent to a healthcare provider. A severe pain is one where the severity of the pain response significantly interferes or prevents an individual from performing one or more activities of daily life. An activity of daily life (ADL) is an activity an individual normally does such as feeding, bathing, dressing, grooming, work, homemaking, and leisure. ADLs may be categorized based on whether the activity is a basic, an instrumental, or an occupational one. Basic ADLs (BADLs) comprise self-care tasks, including, *e.g.,* personal hygiene and grooming, dressing and undressing, self feeding, functional transfers (getting into and out of bed or wheelchair, getting onto or off toilet, etc.), bowel and bladder management, and ambulation (walking with or without use of an assistive device (walker, cane, or crutches) or using a wheelchair). Instrumental ADLs (IADLs) are not necessary for fundamental functioning, but let an individual live independently in a community. IADLs include, *e.g.,* performing housework, taking medications as prescribed, managing money, shopping for groceries or clothing, using communication devices, using technology, using transportation within the community. Occupational ADLs (OADLs) are generally optional in nature and may be delegated to others. OADLs include, *e.g.,* caring of others (including selecting and supervising caregivers), caring of pets, child rearing, using of communication devices, community mobility, financial management, health management and maintenance, meal preparation and cleanup, and safety procedures and emergency responses.

As used herein, the term "severe pain" comprises any acute, subacute, chronic nociceptive, pathological pain, or psychological pain. In aspects of this embodiment, a severe pain is not an inflammatory pain or a pain whose primary origin is due to an inflammatory response.

A severe pain condition may be classified according to duration and pattern of occurrence, such as, e.g., acute pain, subacute pain, and chronic pain. Acute pain is an organic pain state that is typically transitory and of sudden onset, lasting only until the noxious stimulus is removed and/or the underlying damage or pathology has healed. Chronic pain is an organic pain state that is persistent and extends beyond the expected period of healing and may be present continuously or intermediately. Subacute pain is an organic pain state that refers to a pain that is somewhere in between acute pain and chronic pain. Although somewhat arbitrary, the distinction between acute, subacute, and chronic pain may be defined based on the interval of time from onset. Thus, an acute pain is one that lasts less than one month, a subacute pain is one that lasts from one to six months, and a chronic pain is one that lasts six months or more.

A severe pain condition may also be classified as 1) a nociceptive pain; 2) a pathological pain; and 3) an inflammatory pain.

In one embodiment, a severe pain condition comprises a nociceptive pain. Nociceptive pain is a severe pain condition where an organic pain state is caused by a noxious insult or injury of peripheral nerve fibers that respond only to stimuli approaching or exceeding harmful intensity (nociceptors), and is commonly described as an aching pain. This type of pain is associated with damage to non-neural tissue, such as skin, muscles, visceral organs, joints, tendons, or bones, and is represented by a normally functioning somatosensory nervous system. Nociceptive pain may be classified according to the mode of noxious stimulation, such as, e.g., thermal pain, mechanical pain, and chemical pain, or according to the location of the pain, such as, e.g., somatic pain or visceral pain.

In one embodiment, a severe pain condition comprises a somatic pain. Somatic pain is a nociceptive pain that originates from ligaments, tendons, bones, blood vessels, and even nerves themselves. It is detected with somatic nociceptors. The scarcity of pain receptors in these areas produces a dull, poorly-localized pain of longer duration than cutaneous pain. Non-limiting examples of somatic pain include 1) excessive muscle tension can be caused, for example, by a sprain or a strain; 2) repetitive motion disorders can result from overuse of the hands, wrists, elbows, shoulders, neck, back, hips, knees, feet, legs, or ankles; 3) muscle disorders causing somatic pain include, for example, a polymyositis, a dermatomyositis, a lupus, a fibromyalgia, a polymyalgia rheumatica, and a rhabdomyolysis; 4) myalgia including, e.g., muscle pain caused by overuse, over-stretching, viral infection, metabolic myopathy, a nutritional deficiency, or chronic fatigue syndrome; 5) infections including, e.g., an abscess, a trichinosis, an influenza, a Lyme disease, a malaria, a Rocky Mountain spotted fever, Avian influenza, the common cold, community-acquired pneumonia, meningitis, monkeypox, Severe Acute Respiratory Syndrome, toxic shock syndrome, trichinosis, typhoid fever, and upper respiratory tract infection; and 6) drugs including, e.g., cocaine, a statin for lowering cholesterol (such as atorvastatin, simvastatin, and lovastatin), and an ACE inhibitor for lowering blood pressure (such as enalapril and captopril). Somatic pain may be classified as a superficial somatic pain or a deep somatic pain. Superficial somatic pain is initiated by activation of nociceptors in the skin or other superficial tissue, and is sharp, well-defined and clearly located. Examples of injuries that produce superficial somatic pain include minor wounds and minor (first degree) burns. Deep somatic pain is initiated by stimulation of nociceptors in ligaments, tendons, bones, blood vessels, fasciae and muscles, and is dull, aching, poorly localized pain. Examples include sprains and broken bones.

In one embodiment, a severe pain condition comprises a visceral pain. Visceral pain is a nociceptive pain that is initiated by stimulation of nociceptors in the body's organs and internal cavities typically caused by stretching or ischemia. Visceral pain is a diffuse, poorly localized pain and may be described as aching pain, sickening pain, dull pain, squeezing pain, and/or deep pain, and it may be accompanied by nausea and vomiting. Visceral pain is extremely difficult to localize, and several injuries to visceral tissue may exhibit "referred" pain, where the sensation is localized to an area completely unrelated to the site of injury. The even greater scarcity of nociceptors in these areas produces pain that is usually more aching and of a longer duration than somatic pain. Non-limiting examples of somatic pain include 1) functional visceral pain including, e.g., an irritable bowel syndrome and a chronic functional abdominal pain (CFAP), a functional constipation and a functional dyspepsia, a non-cardiac chest pain (NCCP) and a chronic abdominal pain; 2) chronic gastrointestinal inflammation including, e.g., a gastritis, an inflammatory bowel disease, like, e.g., a Crohn's disease, an ulcerative colitis, a microscopic colitis, a diverticulitis and a gastroenteritis; an interstitial cystitis; an intestinal ischemia; a cholecystitis; an appendicitis; a gastroesophageal reflux; an ulcer, a nephrolithiasis, an urinary tract infection, a pancreatitis and a hernia; 3) autoimmune pain including, e.g., a sarcoidosis and a vasculitis; 4) organic visceral pain including, e.g., pain resulting from a traumatic, inflammatory or degenerative lesion of the gut or produced by a tumor impinging on sensory innervation; and 5) treatment-induced visceral pain including, e.g., a pain attendant to chemotherapy therapy or a pain attendant to radiation therapy.

In one embodiment, a severe pain condition comprises a pathological pain. Damage A pathological pain is a severe pain condition where an organic pain state caused by disease or damage to any part of the nervous system or by caused by an abnormal functioning of the nervous system. This type of pain is associated with damage to neural tissue and an abnormally functioning somatosensory nervous system, rather than stimulation of pain receptors. Non-limiting examples of a pathological pain include neuropathic pain and dysfunctional pain.

In one embodiment, a severe pain condition comprises a neuropathic pain. Damage Neuropathic pain is a pathological pain where an organic pain state is caused by damage or disease of the somatosensory nervous system, resulting in abnormal sensory firing from the peripheral nervous system, central nervous systems, or both. Neuropathic pain may involve spontaneous or evoked pain, and may have continuous and/or episodic (paroxysmal) components. Neuropathic pain may be associated with dysesthesia (abnormal pain responses), such as, e.g., allodynia (a painful response to a stimulus that normally is not painful), hyperalgesia (an accentuated response to a painful stimulus that usually causes only a mild discomfort), and hyperpathia (where a short discomfort becomes a prolonged severe pain). Painful sensations can be described as a coldness, numbness, itching, paresthesias (tingling or "pins and needles" sensation), an electric shock, and a burning or ice-burn pain. Non-limiting examples of a neuropathic pain include central neuropathic pain, peripheral neuropathic pain, and deafferentation pain.

In one embodiment, a severe pain condition comprises a central neuropathic pain. Damage or disease affecting nerves of the central nervous system is referred to as a central neuropathic pain. Typically, the damage or disease occurs as cerebral lesions, predominantly thalamic but may involve suprathalamic and infrathalamic regions. Generally, the onset of central neuropathic pain is usually delayed after the occurrence of the initial episode that results in damage to the central nervous system; onset of pain may occur during the phase of recovery from neurologic deficits. Central neuropathic pain may be present in post-stroke conditions including, e.g., thalamic infarction, brainstem infarction, or subarachnoid hemorrhage, cerebral venous thrombosis, cerebral tumors compressing the thalamus or brainstem, cerebral abscesses compressing the thalamus or brainstem, traumatic brain injury, post-surgical pain following brain or spine surgery, complications following brain surgery including thalamotomy for movement disorders, multiple sclerosis, and Parkinson disease, spinal cord injury, complications following spinal surgery including anterolateral cordotomy and commissural myelotomy, ischemic lesions including anterior spinal artery syndrome and Wallenberg syndrome, syringomyelia, radiation myelopathy, HIV myelopathy.

In one embodiment, a severe pain condition comprises a peripheral neuropathic pain. Damage or disease affecting sensory, motor, and/or autonomic nerves of the peripheral nervous system is referred to as a peripheral neuropathic pain. Peripheral neuropathic pain occurs when peripheral nerves fail to carry information to and from the brain and spinal cord, resulting in pain, loss of sensation, or inability to control muscles. In some cases, the failure of nerves that control blood vessels, intestines, and other organs results in abnormal blood pressure, digestion problems, and loss of other basic body processes. Symptoms also depend on whether the condition affects the whole body or just one nerve. Risk factors for neuropathy include diabetes, heavy alcohol use, exposure to certain chemicals and drugs, prolonged pressure on a nerve. Some people have a hereditary predisposition for peripheral neuropathy. The four cardinal patterns of peripheral neuropathic pain are mononeuropathy, mononeuropathic multiplex, polyneuropathy, and autonomic neuropathy.

In one embodiment, a severe pain condition comprises a mononeuropathic. A mononeuropathy is a peripheral neuropathy involving functional loss or pathological change affecting a single nerve or nerve group. Mononeuropathy is most often caused by damage to a local area resulting from injury or trauma, although occasionally systemic disorders may cause isolated nerve damage. The usual causes are direct trauma, prolonged pressure on the nerve, and compression of the nerve by swelling or injury to nearby body structures. The damage includes destruction of the myelin sheath (covering) of the nerve or of part of the nerve cell (the axon). This damage slows or prevents conduction of impulses through the nerve. Mononeuropathy may involve any part of the body. Mononeuropathic pain is associated with, e.g., a sciatic nerve dysfunction, a common peroneal nerve dysfunction. a radial nerve dysfunction, an ulnar nerve dysfunction, a cranial mononeuropathy VI, a cranial mononeuropathy VII, a cranial mononeuropathy III (compression type), a cranial mononeuropathy III (diabetic type), an axillary nerve dysfunction, a carpal tunnel syndrome, a femoral nerve dysfunction, a tibial nerve dysfunction, a Bell's palsy, a thoracic outlet syndrome, a carpal tunnel syndrome or other focal entrapment neuropathy, and a sixth (abducent) nerve palsy

In one embodiment, a severe pain condition comprises a mononeuropathic multiplex. A mononeuropathic multiplex is a peripheral neuropathy involving functional loss or pathological change that sequentially or simultaneously affects several non-contiguous nerves in an asymmetric manner. A neuropathic pain based on mononeuropathy multiplex may develop over days to years and typically presents with acute or subacute loss of sensory and motor function of individual nerves. The pattern of involvement is asymmetric; however, as the disease progresses deficit(s) becomes more confluent and symmetrical, making it difficult to differentiate from polyneuropathy. Mononeuropathic multiplex may also cause pain characterized as deep, aching pain that is worse at night, and frequently present in the lower back, hip, or leg. Mononeuropathic multiplex may also cause pain characterized as acute, unilateral, severe limb pain followed by anterior muscle weakness and loss of knee reflex. Mononeuropathic multiplex pain is associated with, e.g., diabetes mellitus, infections, such as, e.g., leprosy, lyme disease, HIV, and toxicity.

In one embodiment, a severe pain condition comprises a polyneuropathy. A polyneuropathy is a peripheral neuropathy involving functional loss or pathological change affecting multiple nerves throughout the body in a symmetric manner. A polyneuropathy may be acute and appear without warning, or chronic and develop gradually over a longer period of time. Many polyneuropathies have both motor and sensory involvement, and some also involve dysfunction of the autonomic nervous system. These disorders are often symmetrical, and usually due to various systematic illnesses and disease processes that affect the peripheral nervous system in its entirety. A polyneuropathy frequently affect the feet and hands, causing weakness, loss of sensation, pins-and-needle sensations or burning pain. Polyneuropathies can be classified in different ways, such as by cause, by speed of progression, or by the parts of the body involved. Classes of polyneuropathy are also distinguished by which part of the nerve cell is mainly affected: the axon, the myelin sheath, or the cell body.

In one embodiment, a severe pain condition comprises a distal axonopathy. Distal axonopathy, or "dying-back neuropathy", is the result of some metabolic or toxic derangement of a neuron. They may be caused by metabolic diseases such as diabetes, renal failure, deficiency syndromes such as malnutrition and alcoholism, or the effects of toxins or drugs such as chemotherapy. The most common cause of distal axonopathy is diabetes, and the most common distal axonopathy is diabetic neuropathy. They can be divided according to the type of axon affected: large-fiber, small-fiber, or both. The most distal portions of axons are usually the first to degenerate, and axonal atrophy advances slowly towards the nerve's cell body. If the cause is removed, regeneration is possible, though the prognosis depends on the duration and severity of the stimulus. People with distal axonopathies usually present with sensorimotor disturbances that have a symmetrical "stocking and glove" distribution. Deep tendon reflexes and autonomic nervous system functions are also lost or diminished in affected areas.

In one embodiment, a severe pain condition comprises a myelinopathy. Myelinopathy, or "demyelinating polyneuropathy", is due to a loss of myelin (or of the Schwann cells that make and contain it). This demyelination leaves the axon intact, but slows down or completely blocks the conduction of action potentials through the axon of the nerve cell. The most common cause is acute inflammatory demyelinating polyneuropathy (AIDP, the most common form of Guillain-Barre syndrome), though other causes include chronic inflammatory demyelinating polyneuropathy (CIDP), genetic metabolic disorders (e.g., leukodystrophy), and toxins.

In one embodiment, a severe pain condition comprises a neuronopathy. Neuronopathy is the result of destruction of peripheral nervous system neurons. They may be caused by motor neuron diseases, sensory neuronopathies (e.g., Herpes zoster), toxins or autonomic dysfunction. Neurotoxins may cause neuronopathies, such as the chemotherapy agent vincristine. A person with neuronopathy may present in different ways, depending on the cause, the way it affects the nerve cells, and the type of nerve cell that is most affected.

There are numerous conditions that can cause polyneuropathy. Peripheral neuropathic pain include, without limitation, neuropathies associated with systemic disease like diabetic neuropathy, neuropathies associated with metabolic conditions like alcoholic neuropathy and burning feet syndrome, neuropathies associated with viral infections like herpes zoster and HIV, neuropathies associated with nutritional deficiencies, neuropathies associated with toxins, neuropathies associated with tumor compression, neuropathies associated with remote manifestations of malignancies, neuropathies associated with drugs like chemotherapy, neuropathies associated with radiation, neuropathies associated with immune mediated disorders, and neuropathies associated with physical trauma to a nerve trunk. Polyneuropathic pain, includes, without limitation, post-polio syndrome, postmastectomy syndrome, diabetic neuropathy, alcohol neuropathy, amyloid, toxins, AIDS, hypothyroidism, uremia, vitamin deficiencies, chemotherapy-induced pain, 2',3'-didexoycytidine (ddC) treatment, Guillain-Barre syndrome or Fabry's disease.

In one embodiment, a severe pain condition comprises an autonomic neuropathy. An autonomic neuropathy is a peripheral neuropathy involving functional loss or pathological change affecting the non-voluntary, non-sensory nervous system (i.e., the autonomic nervous system). Autonomic neuropathy is a form of polyneuropathy which affects mostly the internal organs such as the bladder, muscles, the cardiovascular system, the digestive tract, and the genital organs.

Peripheral neuropathic pain may be present in systemic diseases, metabolic disorders, nutrient disorders, drug-induced disorders, traumatic injury, traumatic and entrapment syndromes, post-surgical pain surgery, complications following surgery, HIV sensory neuropathy, demyelinating polyradiculoneuropathy, postherpetic neuralgia, nerve root avulsions, cranial neuralgias like trigeminal neuralgia, neuropathic cancer pain, compression of peripheral nerves, nerve plexuses, and nerve roots, paraneoplastic peripheral neuropathy, ganglionopathy, complication of cancer therapies like chemotherapy, radiation, and surgery, and complex regional pain syndrome like Type 1 and Type 2.

In one embodiment, a severe pain condition comprises a neuralgia. A neuralgia is a peripheral neuropathic pain that radiates along the course of one or more specific nerves usually without any demonstrable pathological change in the nerve structure. The affected nerves are responsible for sensing touch, temperature and pressure. Simple stimuli such as eating, talking, washing the face, or any light touch or sensation can trigger an attack (even the sensation of a gentle breeze). The attacks can occur in clusters or as an isolated attack. Generally, a neuralgia causes short episodes of excruciating pain, usually for less than two minutes. However, in the atypical forms of neuralgia, the pain can also present as merely aching to severe pain and last for extended periods. Symptoms include sharp, stabbing pain or constant, burning pain located anywhere, usually on or near the surface of the body, in the same location for each episode; pain along the path of a specific nerve; impaired function of affected body part due to pain, or muscle weakness due to concomitant motor nerve damage; increased sensitivity of the skin or numbness of the affected skin area; and any touch or pressure is interpreted as pain. Movement may also be painful. A neuralgia, includes, without limitation, a trigeminal neuralgia, a glossopharyngeal neuralgia, a post-herpetic neuralgia (caused by, e.g., herpesvirus, syphilis and Lyme disease), a carpal tunnel syndrome, a meralgia paresthetica, a sciatica and an atypical facial pain.

In one embodiment, a severe pain condition comprises a Complex Regional Pain Syndrome (CRPS). A CRPS is a neuropathy resulting from sympathetically-maintained pain. Although unknown, mechanisms probably involve abnormal sympathetic-somatic nerve connections (ephapses), local inflammatory changes, and changes in the spinal cord. CRPS presents in two forms. CRPS 1 (reflex sympathetic dystrophy syndrome) is a chronic nerve disorder that occurs most often in the arms or legs after a minor or major injury. CRPS 1 is associated with severe pain; changes in the nails, bone, and skin; and an increased sensitivity to touch in the affected limb. CRPS 2 (causalgia) is caused by an identified injury to the nerve and results in a syndrome of sustained burning pain, allodynia, and hyperpathia after a traumatic nerve lesion, often combined with vasomotor and sudomotor dysfunction and later trophic changes.

In one embodiment, a severe pain condition comprises a referred pain. Referred pain arises from pain localized to an area separate from the site of pain stimulation. Often, referred pain arises when a nerve is compressed or damaged at or near its origin. In this circumstance, the sensation of pain will generally be felt in the territory that the nerve serves, even though the damage originates elsewhere. A common example occurs in intervertebral disc herniation, in which a nerve root arising from the spinal cord is compressed by adjacent disc material. Although pain may arise from the damaged disc itself, pain will also be felt in the region served by the compressed nerve (for example, the thigh, knee, or foot). Relieving the pressure on the nerve root may ameliorate the referred pain, provided that permanent nerve damage has not occurred. Myocardial ischaemia (the loss of blood flow to a part of the heart muscle tissue) is possibly the best known example of referred pain; the sensation can occur in the upper chest as a restricted feeling, or as an ache in the left shoulder, arm or even hand.

In one embodiment, a severe pain condition comprises a deafferentation pain. Damage or disease affecting peripheral or central afferent neural activity is referred to as a deafferentation pain. Deafferentation pain is due to partial or complete loss of the sensory input from a portion of the body, and can be caused by interruption of either peripheral sensory fibres or nerves from the central nervous system. The mechanism underlying this type of pain is unknown but may involve sensitization of central neurons, with lower activation thresholds and expansion of receptive fields. A deafferentation pain syndrome, includes, without limitation, a phantom pain, a brain injury, a spinal cord injury, a lumbar radiculopathy, a post-stroke pain, a paraplegia, avulsion of the brachial plexus or other types of lesions of peripheral nerves, a pathology of the central nervous system.

In one embodiment, a severe pain condition comprises a dysfunctional pain. Dysfunctional pain is a pathological pain where an organic pain state is caused by abnormal function of the somatosensory nervous system, but which are not initiated by an identifiable lesion of any part of the nervous system. Similar to neuropathic pain, dysfunctional pain is commonly described as a burning pain, a coldness, an electric shock, a "pins and needles" sensation, numbness and itching.

In one embodiment, a severe pain condition comprises a headache. A severe pain condition may be a headache (medically known as cephalgia) is a condition of mild to severe pain in the head; sometimes neck or upper back pain may also be interpreted as a headache. It may indicate an underlying local or systemic disease or be a disorder in itself. A headache includes, without limitation, a muscular/myogenic headache, a vascular headache, a traction headache, inflammatory headache, a chronic sinusitis headache, a hormone headache, a rebound headache, an organic headache, and an ictal headache.

In one embodiment, a severe pain condition comprises a muscular/myogenic headache. Muscular/myogenic headaches appear to involve the tightening or tensing of facial and neck muscles; they may radiate to the forehead. Tension headache is the most common form of myogenic headache. A tension headache is a condition involving pain or discomfort in the head, scalp, or neck, usually associated with muscle tightness in these areas. Tension headaches result from the contraction of neck and scalp muscles. One cause of this muscle contraction is a response to stress, depression or anxiety. Any activity that causes the head to be held in one position for a long time without moving can cause a headache. Such activities include typing or use of computers, fine work with the hands, and use of a microscope. Sleeping in a cold room or sleeping with the neck in an abnormal position may also trigger this type of headache. A tension-type headache, includes, without limitation, an episodic tension headache and a chronic tension headache.

In one embodiment, a severe pain condition comprises a vascular headache. The most common type of vascular headache is migraine. Other kinds of vascular headaches include cluster headaches, which cause repeated episodes of intense pain, and headaches resulting from high blood pressure.

In one embodiment, a severe pain condition comprises a migraine headache. A migraine is a heterogeneous disorder that generally involves recurring headaches. Migraines are different from other headaches because they occur with other symptoms, such as, e.g., nausea, vomiting, or sensitivity to light. In most people, a throbbing pain is felt only on one side of the head. Clinical features such as type of aura symptoms, presence of prodromes, or associated symptoms such as vertigo, may be seen in subgroups of patients with different underlying pathophysiological and genetic mechanisms. A migraine headache, includes, without limitation, a migraine without aura (common migraine), a migraine with aura (classic migraine), a menstrual migraine, a migraine equivalent (acephalic headache), a complicated migraine, an abdominal migraine and a mixed tension migraine.

In one embodiment, a severe pain condition comprises a cluster headache. Cluster headaches affect one side of the head (unilateral) and may be associated with tearing of the eyes and nasal congestion. They occurs in clusters, happening repeatedly every day at the same time for several weeks and then remitting.

In one embodiment, a severe pain condition comprises a sinusitis headache. Sinusitis is inflammation, either bacterial, fungal, viral, allergic or autoimmune, of the paranasal sinuses. Chronic sinusitis is one of the most common complications of the common cold. Symptoms include: Nasal congestion; facial pain; headache; fever; general malaise; thick green or yellow discharge; feeling of facial 'fullness' worsening on bending over. In a small number of cases, chronic maxillary sinusitis can also be brought on by the spreading of bacteria from a dental infection. Chronic hyperplastic eosinophilic sinusitis is a noninfective form of chronic sinusitis.

In one embodiment, a severe pain condition comprises a traction headache. Traction and inflammatory headaches are usually symptoms of other disorders, ranging from stroke to sinus infection.

In one embodiment, a severe pain condition comprises a rebound headache. Rebound headaches, also known as medication overuse headaches, occur when medication is taken too frequently to relieve headache. Rebound headaches frequently occur daily and can be very painful.

In one embodiment, a severe pain condition comprises a ictal headache. Ictal headaches are headaches associated with seizure activity.

In one embodiment, a severe pain condition comprises a psychogenic pain. Psychogenic pain, also called psychalgia or somatoform pain, is pain caused, increased, or prolonged by mental, emotional, or behavioral factors. Headache, back pain, and stomach pain are sometimes diagnosed as psychogenic. Sufferers are often stigmatized, because both medical professionals and the general public tend to think that pain from a psychological source is not "real". However, specialists consider that it is no less actual or hurtful than pain from any other source. Individuals with long term pain frequently display psychological disturbance.

In one embodiment, a severe pain condition comprises an inflammatory pain. An inflammatory pain is a severe pain condition where an organic pain state is caused by the release of mediators at a site of tissue inflammation which activate and sensitize the nociceptive pain pathway. This type of pain is associated with any tissue damage caused by a chronic inflammatory response. Inflammatory pain may be associate with an arthritic disorder, an autoimmune disease, a connective tissue disorder, an injury, an infection, and a neuritis.

In one embodiment, a severe pain condition does not comprise an inflammatory pain.

In one embodiment, a severe pain condition is not caused by an inflammatory response.

A pharmaceutical composition disclosed herein is administered to an individual. An individual is typically a human being. Typically, any individual who is a candidate for a conventional severe pain treatment is a candidate for a severe pain treatment disclosed herein. Pre-operative evaluation typically includes routine history and physical examination in addition to thorough informed consent disclosing all relevant risks and benefits of the procedure.

A pharmaceutical composition disclosed herein may comprise a therapeutic compound in a therapeutically effective amount. As used herein, the term "effective amount" is synonymous with "therapeutically effective amount", "effective dose", or "therapeutically effective dose" and when used in reference to treating a severe pain condition refers to the minimum dose of a therapeutic compound disclosed herein necessary to achieve the desired therapeutic effect and includes a dose sufficient to reduce a symptom associated with a severe pain condition. The effectiveness of a therapeutic compound disclosed herein in treating a severe pain condition can be determined by observing an improvement in an individual based upon one or more clinical symptoms, and/or physiological indicators associated with the condition. An improvement in a severe pain condition also can be indicated by a reduced need for a concurrent therapy.

The appropriate effective amount of a therapeutic compound disclosed herein to be administered to an individual for a particular severe pain condition can be determined by a person of ordinary skill in the art by taking into account factors, including, without limitation, the type of the severe pain condition, the location of the severe pain condition, the cause of the severe pain condition, the severity of the severe pain condition, the degree of relief desired, the duration of relief desired, the particular therapeutic compound used, the rate of excretion of the therapeutic compound used, the pharmacodynamics of the therapeutic compound used, the nature of the other compounds to be included in the composition, the particular formulation, the particular route of administration, the particular characteristics, history and risk factors of the patient, such as, e.g., age, weight, general health and the like, or any combination thereof. Additionally, where repeated administration of a therapeutic compound is used, an effective amount of a therapeutic compound will further depend upon factors, including, without limitation, the frequency of administration, the half-life of the therapeutic compound, or any combination thereof. In is known by a person of ordinary skill in the art that an effective amount of a therapeutic compound disclosed herein can be extrapolated from *in vitro* assays and *in vivo* administration studies using animal models prior to administration to humans.

In aspects of this embodiment, a therapeutically effective amount of a therapeutic compound disclosed herein reduces a symptom associated with a severe pain condition by, e.g., at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95% or at least 100%. In other aspects of this embodiment, a therapeutically effective amount of a therapeutic compound disclosed herein reduces a symptom associated with a severe pain condition by, e.g., at most 10%, at most 15%, at most 20%, at most 25%, at most 30%, at most 35%, at most 40%, at most 45%, at most 50%, at most 55%, at most 60%, at most 65%, at most 70%, at most 75%, at most 80%, at most 85%, at most 90%, at most 95% or at most 100%. In yet other aspects of this embodiment, a therapeutically effective amount of a therapeutic compound disclosed herein reduces a symptom associated with a severe pain condition by, *e.g.,* 10% to 100%, 10% to 90%, 10% to 80%, 10% to 70%, 10% to 60%, 10% to 50%, 10% to 40%, 20% to 100%, 20% to 90%, 20% to 80%, 20% to 20%, 20% to 60%, 20% to 50%, 20% to 40%, 30% to 100%, 30% to 90%, 30% to 80%, 30% to 70%, 30% to 60%, or 30% to 50%.

In yet other aspects of this embodiment, a therapeutically effective amount of a therapeutic compound disclosed herein generally is in the range of 0.001 mg/kg/day to 100 mg/kg/day. In aspects of this embodiment, an effective amount of a therapeutic compound disclosed herein may be, *e.g.,* at least 0.001 mg/kg/day, at least 0.01 mg/kg/day, at least 0.1 mg/kg/day, at least 1.0 mg/kg/day, at least 5.0 mg/kg/day, at least 10 mg/kg/day, at least 15 mg/kg/day, at least 20 mg/kg/day, at least 25 mg/kg/day, at least 30 mg/kg/day, at least 35 mg/kg/day, at least 40 mg/kg/day, at least 45 mg/kg/day, or at least 50 mg/kg/day. In other aspects of this embodiment, an effective amount of a therapeutic compound disclosed herein may be in the range of, *e.g.,* 0.001 mg/kg/day to 10 mg/kg/day, 0.001 mg/kg/day to 15 mg/kg/day, 0.001 mg/kg/day to 20 mg/kg/day, 0.001 mg/kg/day to 25 mg/kg/day, 0.001 mg/kg/day to 30 mg/kg/day, 0.001 mg/kg/day to 35 mg/kg/day, 0.001 mg/kg/day to 40 mg/kg/day, 0.001 mg/kg/day to 45 mg/kg/day, 0.001 mg/kg/day to 50 mg/kg/day, 0.001 mg/kg/day to 75 mg/kg/day, or 0.001 mg/kg/day to 100 mg/kg/day. In yet other aspects of this embodiment, an effective amount of a therapeutic compound disclosed herein may be in the range of, *e.g.,* 0.01 mg/kg/day to 10 mg/kg/day, 0.01 mg/kg/day to 15 mg/kg/day, 0.01 mg/kg/day to 20 mg/kg/day, 0.01 mg/kg/day to 25 mg/kg/day, 0.01 mg/kg/day to 30 mg/kg/day, 0.01 mg/kg/day to 35 mg/kg/day, 0.01 mg/kg/day to 40 mg/kg/day, 0.01 mg/kg/day to 45 mg/kg/day, 0.01 mg/kg/day to 50 mg/kg/day, 0.01 mg/kg/day to 75 mg/kg/day, or 0.01 mg/kg/day to 100 mg/kg/day. In still other aspects of this embodiment, an effective amount of a therapeutic compound disclosed herein may be in the range of, *e.g.,* 0.1 mg/kg/day to 10 mg/kg/day, 0.1 mg/kg/day to 15 mg/kg/day, 0.1 mg/kg/day to 20 mg/kg/day, 0.1 mg/kg/day to 25 mg/kg/day, 0.1 mg/kg/day to 30 mg/kg/day, 0.1 mg/kg/day to 35 mg/kg/day, 0.1 mg/kg/day to 40 mg/kg/day, 0.1 mg/kg/day to 45 mg/kg/day, 0.1 mg/kg/day to 50 mg/kg/day, 0.1 mg/kg/day to 75 mg/kg/day, or 0.1 mg/kg/day to 100 mg/kg/day.

In other aspects of this embodiment, an effective amount of a therapeutic compound disclosed herein may be in the range of, *e.g.,* 1 mg/kg/day to 10 mg/kg/day, 1 mg/kg/day to 15 mg/kg/day, 1 mg/kg/day to 20 mg/kg/day, 1 mg/kg/day to 25 mg/kg/day, 1 mg/kg/day to 30 mg/kg/day, 1 mg/kg/day to 35 mg/kg/day, 1 mg/kg/day to 40 mg/kg/day, 1 mg/kg/day to 45 mg/kg/day, 1 mg/kg/day to 50 mg/kg/day, 1 mg/kg/day to 75 mg/kg/day, or 1 mg/kg/day to 100 mg/kg/day. In yet other aspects of this embodiment, an effective amount of a therapeutic compound disclosed herein may be in the range of, *e.g.,* 5 mg/kg/day to 10 mg/kg/day, 5 mg/kg/day to 15 mg/kg/day, 5 mg/kg/day to 20 mg/kg/day, 5 mg/kg/day to 25 mg/kg/day, 5 mg/kg/day to 30 mg/kg/day, 5 mg/kg/day to 35 mg/kg/day, 5 mg/kg/day to 40 mg/kg/day, 5 mg/kg/day to 45 mg/kg/day, 5 mg/kg/day to 50 mg/kg/day, 5 mg/kg/day to 75 mg/kg/day, or 5 mg/kg/day to 100 mg/kg/day.

Dosing can be single dosage or cumulative (serial dosing), and can be readily determined by one skilled in the art. For instance, treatment of a severe pain condition may comprise a one-time administration of an effective dose of a pharmaceutical composition disclosed herein. Alternatively, treatment of a severe pain condition may comprise multiple administrations of an effective dose of a pharmaceutical composition carried out over a range of time periods, such as, *e.g.,* once daily, twice daily, trice daily, once every few days, or once weekly. The timing of administration can vary from individual to individual, depending upon such factors as the severity of an individual's symptoms. For example, an effective dose of a pharmaceutical composition disclosed herein can be administered to an individual once daily for an indefinite period of time, or until the individual no longer requires therapy. A person of ordinary skill in the art will recognize that the condition of the individual can be monitored throughout the course of treatment and that the effective amount of a pharmaceutical composition disclosed herein that is administered can be adjusted accordingly.

Various routes of administration can be useful for administering a therapeutic compound disclosed herein, according to a method of treating a severe pain condition disclosed herein. A pharmaceutical composition may be administered to an individual by any of a variety of means depending, *e.g.,* on the type of the severe pain condition to be treated, the location of the severe pain condition to be treated, the specific therapeutic compound or composition used, or other compound to be included in the composition, and the history, risk factors and symptoms of the individual. As such, topical, enteral or parenteral routes of administration may be suitable for of treating a severe pain condition disclosed herein and such routes include both local and systemic delivery of a therapeutic compound or composition disclosed herein. Compositions comprising either a single therapeutic compound disclosed herein, or two or more therapeutic compounds disclosed herein are intended for inhaled, topical, intranasal, sublingual, injection, infusion, instillation, rectal and/or vaginal use may be prepared according to any method known to the art for the manufacture of pharmaceutical compositions.

Upon administration to an individual, a pharmaceutical composition comprising a therapeutic compound disclosed herein results in a bio-distribution of the therapeutic compound different than a bio-distribution of the therapeutic compound included in the same pharmaceutical composition, except without an adjuvant disclosed herein.

In another embodiment, upon administration to an individual, a therapeutic compound of the pharmaceutical composition disclosed herein is delivered to a macrophage. Macrophages are one of the key cell types believed to be involved in the control of the inflammation response which can lead to pain. The resultant high level of a therapeutic compound having anti-pain activity present in the macrophages results in a clinically effective treatment of a severe pain condition. In an aspect of this embodiment, upon administration to an individual, a therapeutically effective amount of a therapeutic compound of the pharmaceutical composition disclosed herein is preferentially delivered to a macrophage. In other aspect of this embodiment, upon administration to an individual, a therapeutic compound of the pharmaceutical composition disclosed herein is substantially delivered to a macrophage. In yet other aspect of this embodiment, upon administration to an individual, the amount of a therapeutic compound of the pharmaceutical composition disclosed herein delivered to a macrophage is, e.g., at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 100% of the total amount of the therapeutic compound contained in the administered pharmaceutical composition. In still other aspects of this embodiment, upon administration to an individual, the amount of a therapeutic compound of the pharmaceutical composition disclosed herein delivered to a macrophage is in a range of, e.g., 5% to 100%, 10% to 100%, 15% to 100%, 20% to 100%, 25% to 100%, 30% to 100%, 35% to 100%, 40% to 100%, 45% to 100%, 50% to 100%, 5% to 90%, 10% to 90%, 15% to 90%, 20% to 90%, 25% to 90%, 30% to 90%, 35% to 90%, 40% to 90%, 45% to 90%, 50% to 90%, 5% to 80%, 10% to 80%, 15% to 80%, 20% to 80%, 25% to 80%, 30% to 80%, 35% to 80%, 40% to 80%, 45% to 80%, 50% to 80%, 5% to 70%, 10% to 70%, 15% to 70%, 20% to 70%, 25% to 70%, 30% to 70%, 35% to 70%, 40% to 70%, 45% to 70%, or 50% to 70% of the total amount of the therapeutic compound contained in the administered pharmaceutical composition.

In another embodiment, upon administration to an individual, a therapeutic compound of the pharmaceutical composition disclosed herein is delivered to a dentritic cell. Dendritic cells are one of the key cell types believed to coordinate the interplay between innate and adaptive immunity. The resultant high level of a therapeutic compound having anti-pain activity present in the dentritic cells results in a clinically effective treatment of a severe pain condition. In an aspect of this embodiment, upon administration to an individual, a therapeutically effective amount of a therapeutic compound of the pharmaceutical composition disclosed herein is preferentially delivered to a dentritic cell. In other aspect of this embodiment, upon administration to an individual, a therapeutic compound of the pharmaceutical composition disclosed herein is substantially delivered to a dentritic cell. In yet other aspect of this embodiment, upon administration to an individual, the amount of a therapeutic compound of the pharmaceutical composition disclosed herein delivered to a dentritic cell is, e.g., at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 100% of the total amount of the therapeutic compound contained in the administered pharmaceutical composition. In still other aspects of this embodiment, upon administration to an individual, the amount of a therapeutic compound of the pharmaceutical composition disclosed herein delivered to a dentritic cell is in a range of, e.g., 5% to 100%, 10% to 100%, 15% to 100%, 20% to 100%, 25% to 100%, 30% to 100%, 35% to 100%, 40% to 100%, 45% to 100%, 50% to 100%, 5% to 90%, 10% to 90%, 15% to 90%, 20% to 90%, 25% to 90%, 30% to 90%, 35% to 90%, 40% to 90%, 45% to 90%, 50% to 90%, 5% to 80%, 10% to 80%, 15% to 80%, 20% to 80%, 25% to 80%, 30% to 80%, 35% to 80%, 40% to 80%, 45% to 80%, 50% to 80%, 5% to 70%, 10% to 70%, 15% to 70%, 20% to 70%, 25% to 70%, 30% to 70%, 35% to 70%, 40% to 70%, 45% to 70%, or 50% to 70% of the total amount of the therapeutic compound contained in the administered pharmaceutical composition.

In another embodiment, upon administration to an individual, a pharmaceutical composition disclosed herein reduces gastric irritation. In an aspect of this embodiment, a pharmaceutical composition disclosed herein substantially reduces gastric irritation. In yet another embodiment, upon administration to an individual, a pharmaceutical composition disclosed herein reduces gastric irritation when compared to the same pharmaceutical composition disclosed herein, except without the pharmaceutically-acceptable adjuvant. In an aspect of this embodiment, a pharmaceutical composition disclosed herein substantially reduces gastric irritation when compared to the same pharmaceutical composition disclosed herein, except without the pharmaceutically-acceptable adjuvant. In other aspects of this embodiment, a pharmaceutical composition disclosed herein reduces gastric irritation by, e.g., at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 100%. In yet other aspects of this embodiment, a pharmaceutical composition disclosed herein reduces gastric irritation in a range of, e.g., 5% to 100%, 10% to 100%, 15% to 100%, 20% to 100%, 25% to 100%, 30% to 100%, 35% to 100%, 40% to 100%, 45% to 100%, 50% to 100%, 5% to 90%, 10% to 90%, 15% to 90%, 20% to 90%, 25% to 90%, 30% to 90%, 35% to 90%, 40% to 90%, 45% to 90%, 50% to 90%, 5% to 80%, 10% to 80%, 15% to 80%, 20% to 80%, 25% to 80%, 30% to 80%, 35% to 80%, 40% to 80%, 45% to 80%, 50% to 80%, 5% to 70%, 10% to 70%, 15% to 70%, 20% to 70%, 25% to 70%, 30% to 70%, 35% to 70%, 40% to 70%, 45% to 70%, or 50% to 70%.

In another embodiment, upon administration to an individual, a pharmaceutical composition disclosed herein reduces intestinal irritation. In an aspect of this embodiment, a pharmaceutical composition disclosed herein substantially reduces intestinal irritation. In yet another embodiment, upon administration to an individual, a pharmaceutical composition disclosed herein reduces intestinal irritation when compared to the same pharmaceutical composition disclosed herein, except without the pharmaceutically-acceptable adjuvant. In an aspect of this embodiment, a pharmaceutical composition disclosed herein substantially reduces intestinal irritation when compared to the same pharmaceutical composition disclosed herein, except without the pharmaceutically-acceptable adjuvant. In other aspects of this embodiment, a pharmaceutical composition disclosed herein reduces intestinal irritation by, e.g., at least 5%, at least 10%, at least 20%, at least 30%, at least 40%, at least 50%, at least 60%, at least 70%, at least 80%, at least 90%, or at least 100% when compared to the same pharmaceutical composition disclosed herein, except without the pharmaceutically-acceptable adjuvant. In yet other aspects of this embodiment, a pharmaceutical composition disclosed herein reduces intestinal irritation by, e.g., 5% to 100%, 10% to 100%, 15% to 100%, 20% to 100%, 25% to 100%, 30% to 100%, 35% to 100%, 40% to 100%, 45% to 100%, 50% to 100%, 5% to 90%, 10% to 90%, 15% to 90%, 20% to 90%, 25% to 90%, 30% to 90%, 35% to 90%, 40% to 90%, 45% to 90%, 50% to 90%, 5% to 80%, 10% to 80%, 15% to 80%, 20% to 80%, 25% to 80%, 30% to 80%, 35% to 80%, 40% to 80%, 45% to 80%, 50% to 80%, 5% to 70%, 10% to 70%, 15% to 70%, 20% to 70%, 25% to 70%, 30% to 70%, 35% to 70%, 40% to 70%, 45% to 70%, or 50% to 70% when compared to the same pharmaceutical composition disclosed herein, except without the pharmaceutically-acceptable adjuvant.

A pharmaceutical composition disclosed herein can also be administered to an individual in combination with other therapeutic compounds to increase the overall therapeutic effect of the treatment. The use of multiple compounds to treat an indication can increase the beneficial effects while reducing the presence of side effects.

### EXAMPLES

The following non-limiting examples are provided for illustrative purposes only in order to facilitate a more complete understanding of the disclosed subject matter. These examples should not be construed to limit any of the embodiments described in the present specification, including those pertaining to the pharmaceutical compositions, methods of preparing pharmaceutical compositions, or methods or uses of treating a severe pain or disease associated with severe pain.

### Example 1

### Differential Scanning Calorimetry

This example illustrates formulation of solid solution pharmaceutical composition as disclosed herein comprising a therapeutic compound. The formulation which comprise a compound having an anti-pain activity are according to the invention.

To prepare a solid solution pharmaceutical composition disclosed herein the following general procedure was used. All ingredient, except the room temperature solid lipid, were mixed together. This mixture was heated to a temperature in the range 50°C to 60°C with stirring in order to dissolve the therapeutic compound thereby creating a solution. The room temperature solid lipid was then added to the solution and the mixture stirred until incorporated. The mixture was then allowed to solidify by cooling to room temperature. Representative formulations containing various therapeutic compounds are show in Table 1. The three last examples of Table 1 are according to the claimed invention.

| **Table 1. Solid Solution Formulations of Ibuprofen** | | | | | |
|---|---|---|---|---|---|
| **Formulation** | **Therapeutic Compound** | **RT Liquid Lipid** | **Stabilizing Agent** | **Neutralizing Agent** | **RT Solid Lipid** |
| Ibuprofen | 200 mg | - | - | - | - |
| RT Solid Lipid | - | - | - | - | 622 mg G43^{a} |
| Vehicle | - | 0.26 mL M35-1^{b} | 0.06 mL PEG400 | - | 260 mg G43 |
| Ibuprofen LA 3-51 | 200 mg | 0.38 mL M35-1 | 0.04 mL PEG400 | - | 160 mg G43 |
| Ibuprofen LA 3-57 | 200 mg | 0.26 mL M35-1 | 0.06 mL PEG400 | - | 260 mg G43 |
| Ibuprofen LA 35-1 | 200 mg | 0.18 mL M35-1 | 0.08 mL PEG400 | - | 380 mg G43 |
| Ibuprofen LA 35-2 | 200 mg | 0.10 mL M35-1 | 0.08 mL PEG400 | - | 460 mg G43 |
| Ibuprofen LA 35-3 | 200 mg | 0.04 mL M35-1 | 0.08 mL PEG400 | - | 520 mg G43 |
| ^{a} G43 is GELUCIRE® 43/01 | | | | | |
| ^{b} M35-1 is MAISINE® 35-1 | | | | | |

The mixture was then allowed to solidify by cooling to room temperature. Solidified compositions were then assessed by appearance and by Differential Scanning Calorimetry (DSC).

During the course of the analysis using DSC, a general trend was observed where a formulation which solidified with a cloudy appearance formed a classic solid composition having a crystalline structure while one which solidified with a clear appearance suggested a solid solution composition having an amorphic structure. Additionally, solidified compositions with a clear appearance that were then remelted with no appearance of a precipitate where indicative of a solid solution composition having an amorphic structure.

Using the visual appearance and remelt assays, large numbers of formulations was accessed for the ability to for a solid-solution composition. A formulation that failed to form a solid, or formed a solid with a cloudy appearance were not further analyzed and discarded. Similarly, formulations were not further analyzed and discarded if a precipitate formed after remelting of a solidified compositions with a clear appearance. Formulations that formed a clear solid were also subsequently analyzed by DSC.

Data for representative ibuprofen formulations solidifying with a clear appearance are shown in FIG. 1. The DSC analysis revealed that individual components exhibited sharp, well-defined peaks. For example, ibuprofen possessed a well-defined melting point range of 75°C to 78°C (FIG. 1A). Similarly, a room temperature solid lipid, or hard fat, like GELUCIRE® 43/01 had a well-defined melting point range of 41°C to 45°C (FIG. 1B). These sharp, well defined melting point temperature ranges are indicative of a composition in a classic solid transition phase having a well-defined crystalline structure. MAISINE® 35-1 and PEG 400 are liquids at room temperature, and as such has a melting point below 20°C. For example, MAISINE® 35-1 has a melting point temperature range of 14°C to 16°C and PEG 400 has a melting point temperature range of 4°C to 8°C.

Unexpectedly, when a vehicle comprising a room temperature solid lipid (hard fat), a room temperature liquid lipid and a stabilizing agent were examined by DSC, a new melting point temperature peak appeared. For example, in addition to the GELUCIRE® 43/01 peak of 41°C to 45°C, DSC analysis identified a new broad melting point temperature range of 32°C to 38°C (FIG. 1C). This temperature range was different than that for the individual components alone: 41°C to 45°C for GELUCIRE® 43/01, 14°C to 16°C for MAISINE® 35-1, and 4°C to 8°C for PEG 400. These results indicate that a portion of the composition was forming into a solid solution phase instead of a classic solid phase.

Surprisingly, formulation comprising the therapeutic compound with this vehicle exhibited a broad melting range with a melting point temperature different than the individual components (FIGS 1D-1H). Furthermore, no peaks relating to either the therapeutic compound alone or the hard fat alone were detected. For example, ibuprofen has a melting point temperature range of 75°C to 78°C, GELUCIRE® 43/01 had a melting point of 41°C to 45°C, MAISINE® 35-1 has a melting point temperature of 14°C to 16°C, and PEG400 has a melting point temperature of 4°C to 8°C. However, a formulation comprising these components results in a composition with a melting point of between 35°C to 40°C, depending on the amounts and ratios used. In a classic mixed phase of distinct solid components, each individual peak would have been resolved, i.e., 75°C to 78°C for Ibuprofen, 41°C to 45°C and GELUCIRE® 43/01. This was not the case, these peaks disappeared altogether. The presence of a single new melting point peak, and the concomitant disappearance of the individual component melting point peaks, for the solidified composition indicates that a novel solid solution structure of therapeutic compound and room temperature solid lipid (hard fat) was formed.

### Example 2

### Solid solution pharmaceutical compositions comprising Artemether

This example illustrates how to make a solid solution pharmaceutical composition disclosed herein comprising Artemether.

To prepare a solid solution pharmaceutical composition disclosed herein using Artemether, the following method was performed. 1.0 g GELUCIRE® 43/01 (Gattefosse), a waxy solid (as room temperature solid lipid) having a melting point of between 41°C to 45°C and comprising a mixture of saturated C₁₀-C₁₈ triglycerides, and 0.5 mL tributyrin (as room temperature liquid lipid) were added to a vessel and heated to 40°C to 50°C with stirring until all components of the mixture were incorporated. 40 mg Artemether was added to 0.8 mL of this incorporated mixture and stirred until dissolved. The heated mixture was then cooled to 37°C to 40°C, and aliquoted by pouring into molds and cooled to room temperature. Alternatively, the mixture can be cooled to room temperature and then subsequently reheated to 40°C to 45°C for aliquoting into molds.

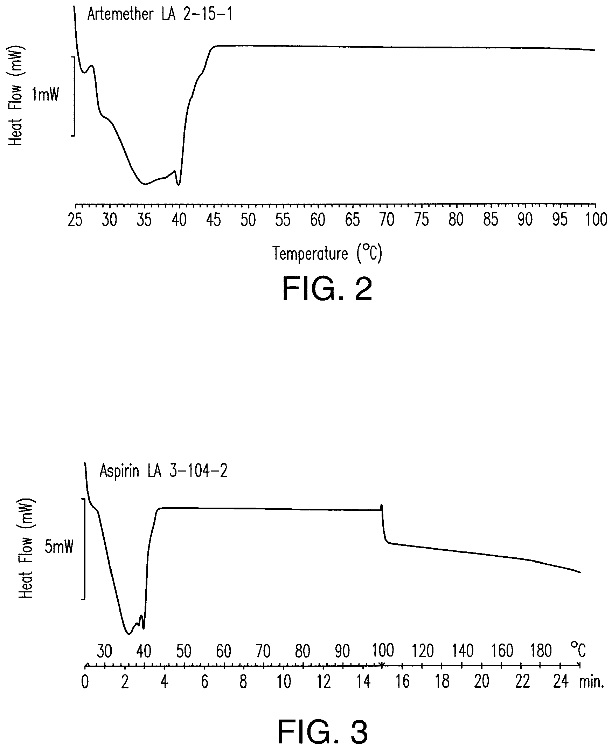

The formulation solidified with a clear appearance. In addition, DSC analysis indicated a new broad melting point temperature range of 35°C to 40°C (FIG. 2). This temperature range was different than that for the individual components alone: 41°C to 45°C for GELUCIRE^{®} 43/01 (Gattefosse) and 86°C to 90°C for Artemether. Tributyrin is a liquid at room temperature, and as such has a melting point below 20°C. These results indicate that a solid solution formulation as disclosed herein comprising Artemether was formed.

### Example 3

### Solid solution pharmaceutical compositions comprising Aspirin

This example illustrates how to make a solid solution pharmaceutical composition disclosed herein comprising Aspirin.

To prepare a solid solution pharmaceutical composition disclosed herein using Aspirin, the following method was performed. 120 mg Aspirin and 0.5 mL isosorbide dimethyl ester (as stabilizing agent) were added to a vessel and heated to 50°C to 60°C with stirring until all components of the mixture were dissolved. 1.0 g GELUCIRE® 43/01 (Gattefosse), a waxy solid (as room temperature solid lipid) having a melting point of between 41°C to 45°C and comprising a mixture of saturated C₁₀-C₁₈ triglycerides, was added to this solution and stirred until incorporated. The heated mixture was then cooled to 37°C to 40°C, and aliquoted by pouring into molds and cooled to room temperature. Alternatively, the mixture can be cooled to room temperature and then subsequently reheated to 40°C to 45°C for aliquoting into molds.

The formulation solidified with a clear appearance. In addition, DSC analysis indicated a new broad melting point temperature range of 35°C to 40°C (FIG. 3). This temperature range was different than that for the individual components alone: 41°C to 45°C for GELUCIRE® 43/01 (Gattefosse) and 138°C to 140°C for Aspirin. Isosorbide dimethyl ester is a liquid at room temperature, and as such has a melting point below 20°C. These results indicate that a solid solution formulation as disclosed herein comprising Aspirin was formed.

### Example 4

### Solid solution pharmaceutical compositions comprising Dantrolene

This example illustrates how to make a solid solution pharmaceutical composition disclosed herein comprising Dantrolene.

To prepare a solid solution pharmaceutical composition disclosed herein using Dantrolene, the following method was performed. Add 232.7 mg stearic acid (as neutralizing agent) to a vessel and heat to 70°C to 75°C with stirring until melted. Add 250.2 mg Dantrolene sodium salt and 20 mL isosorbide dimethyl ester (as stabilizing agent) to melted stearic acid and stir until uniform consistency is achieved. 20.02 g GELUCIRE® 43/01 (Gattefosse), a waxy solid (as room temperature solid lipid) having a melting point of between 41°C to 45°C and comprising a mixture of saturated C₁₀-C₁₈ triglycerides was added to this solution and stirred until incorporated. The heated mixture was then cooled to 37°C to 40°C, and aliquoted by pouring into molds and cooled to room temperature. Alternatively, the mixture can be cooled to room temperature and then subsequently reheated to 40°C to 45°C for aliquoting into molds.

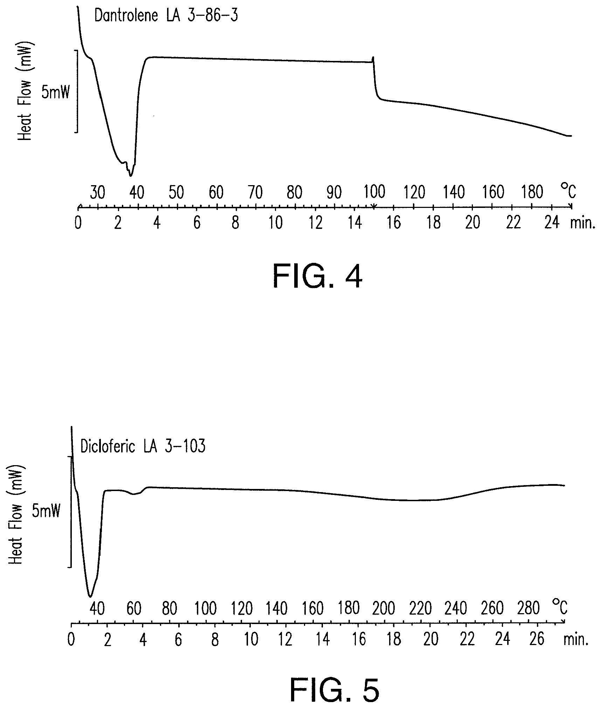

The formulation solidified with a clear appearance. In addition, DSC analysis indicated a new broad melting point temperature range of 34°C to 39°C (FIG. 4). This temperature range was different than that for the individual components alone: 41°C to 45°C for GELUCIRE® 43/01 (Gattefosse), stearic acid is 70°C, and 279°C to 280°C for Dantrolene. Isosorbide dimethyl ester is a liquid at room temperature, and as such has a melting point below 20°C. These results indicate that a solid solution formulation as disclosed herein comprising Dantrolene was formed.

To prepare a solid solution pharmaceutical composition disclosed herein using Dantrolene, the following method was performed. Add 309.8 mg stearic acid (as neutralizing agent) to a vessel and heat to 70°C to 75°C with stirring until melted. Add 59.6 mg Dantrolene sodium salt to melted stearic acid and stir until uniform consistency is achieved. Add 0.75 mL isosorbide dimethyl ester (as stabilizing agent) and stir mixture until all components are dissolved. 760.3 mg GELUCIRE® 43/01 (Gattefosse), a waxy solid (as room temperature solid lipid) having a melting point of between 41°C to 45°C and comprising a mixture of saturated C₁₀-C₁₈ triglycerides was added to this solution and stirred until incorporated. The heated mixture was then cooled to 37°C to 40°C, and aliquoted by pouring into molds and cooled to room temperature. Alternatively, the mixture can be cooled to room temperature and then subsequently reheated to 40°C to 45°C for aliquoting into molds. The formulation solidified with a clear appearance and remelted without forming a precipitate. These results indicate that a solid solution formulation as disclosed herein comprising Dantrolene was formed.

To prepare a solid solution pharmaceutical composition disclosed herein using Dantrolene, the following method was performed. Add 250.2 mg stearic acid (as neutralizing agent) to a vessel and heat to 70°C to 75°C with stirring until melted. Add 50.3 mg Dantrolene sodium salt to melted stearic acid and stir until uniform consistency is achieved. Add 5.0 mL isosorbide dimethyl ester (as stabilizing agent) and stir mixture until all components are dissolved. 5.0 g GELUCIRE® 43/01 (Gattefosse), a waxy solid (as room temperature solid lipid) having a melting point of between 41°C to 45°C and comprising a mixture of saturated C₁₀-C₁₈ triglycerides was added to this solution and stirred until incorporated. The heated mixture was then cooled to 37°C to 40°C, and aliquoted by pouring into molds and cooled to room temperature. Alternatively, the mixture can be cooled to room temperature and then subsequently reheated to 40°C to 45°C for aliquoting into molds. The formulation solidified with a clear appearance and remelted without forming a precipitate. These results indicate that a solid solution formulation as disclosed herein comprising Dantrolene was formed.

To prepare a solid solution pharmaceutical composition disclosed herein using Dantrolene, the following method was performed. Add 225.0 mg stearic acid (as neutralizing agent) to a vessel and heat to 70°C to 75°C with stirring until melted. Add 25.1 mg Dantrolene sodium salt to melted stearic acid and stir until uniform consistency is achieved. Add 2.0 mL isosorbide dimethyl ester (as stabilizing agent) and stir mixture until all components are dissolved. 2.07 g GELUCIRE® 43/01 (Gattefosse), a waxy solid (as room temperature solid lipid) having a melting point of between 41°C to 45°C and comprising a mixture of saturated C₁₀-C₁₈ triglycerides was added to this solution and stirred until incorporated. The heated mixture was then cooled to 37°C to 40°C, and aliquoted by pouring into molds and cooled to room temperature. Alternatively, the mixture can be cooled to room temperature and then subsequently reheated to 40°C to 45°C for aliquoting into molds. The formulation solidified with a clear appearance and remelted without forming a precipitate. These results indicate that a solid solution formulation as disclosed herein comprising Dantrolene was formed.

To prepare a solid solution pharmaceutical composition disclosed herein using Dantrolene, the following method was performed. Add 224.9 mg stearic acid (as neutralizing agent) to a vessel and heat to 70°C to 75°C with stirring until melted. Add 25.1 mg Dantrolene sodium salt to melted stearic acid and stir until uniform consistency is achieved. Add 0.75 mL isosorbide dimethyl ester (as stabilizing agent) and stir mixture until all components are dissolved. 304.1 mg GELUCIRE® 43/01 (Gattefosse), a waxy solid (as room temperature solid lipid) having a melting point of between 41°C to 45°C and comprising a mixture of saturated C₁₀-C₁₈ triglycerides was added to this solution and stirred until incorporated. The heated mixture was then cooled to 37°C to 40°C, and aliquoted by pouring into molds and cooled to room temperature. Alternatively, the mixture can be cooled to room temperature and then subsequently reheated to 40°C to 45°C for aliquoting into molds. The formulation solidified with a clear appearance and remelted without forming a precipitate. These results indicate that a solid solution formulation as disclosed herein comprising Dantrolene was formed.

### Example 5

### Solid solution pharmaceutical compositions comprising Diclofenac

This example illustrates how to make a solid solution pharmaceutical composition disclosed herein comprising Diclofenac.

To prepare a solid solution pharmaceutical composition disclosed herein using Diclofenac, the following method was performed. 119 mg Diclofenac, 1.0 mL MAISINE® 35-1 (Gattefosse), a glyceryl monolinoleate (as room temperature liquid lipid), and 0.3 mL isosorbide dimethyl ester (as stabilizing agent) were added to a vessel and heated to 50°C to 60°C with stirring until all components of the mixture were dissolved. 1.0 g GELUCIRE® 43/01 (Gattefosse), a waxy solid (as room temperature solid lipid) having a melting point of between 41°C to 45°C and comprising a mixture of saturated C₁₀-C₁₈ triglycerides, was added to this solution and stirred until incorporated. The heated mixture was then cooled to 37°C to 40°C, and aliquoted by pouring into molds and cooled to room temperature. Alternatively, the mixture can be cooled to room temperature and then subsequently reheated to 40°C to 45°C for aliquoting into molds.

The formulation solidified with a clear appearance. In addition, DSC analysis indicated a new broad melting point temperature range of 35°C to 40°C (FIG. 5). This temperature range was different than that for the individual components alone: 41°C to 45°C for GELUCIRE® 43/01 (Gattefosse) and 157°C to 158°C for Diclofenac. MAISINE® 35-1 and isosorbide dimethyl ester are liquids at room temperature, and as such both have melting points below 20°C. These results indicate that a solid solution formulation as disclosed herein comprising Diclofenac was formed.

### Example 6

### Solid solution pharmaceutical compositions comprising Fenofibrate

This example illustrates how to make a solid solution pharmaceutical composition disclosed herein comprising Fenofibrate.

To prepare a solid solution pharmaceutical composition disclosed herein using Fenofibrate, the following method was performed. 400 mg Fenofibrate and 4.0 g GELUCIRE® 43/01 (Gattefosse), a waxy solid (as room temperature solid lipid) having a melting point of between 41°C to 45°C and comprising a mixture of saturated C₁₀-C₁₈ triglycerides, were added to a vessel and heated to 45°C to 55°C with stirring until all components of the mixture were incorporated. 0.76 mL isosorbide dimethyl ester (as stabilizing agent) was added to this mixture and stirred until incorporated. The heated mixture was then cooled to 37°C to 40°C, and aliquoted by pouring into molds and cooled to room temperature. Alternatively, the mixture can be cooled to room temperature and then subsequently reheated to 40°C to 45°C for aliquoting into molds.

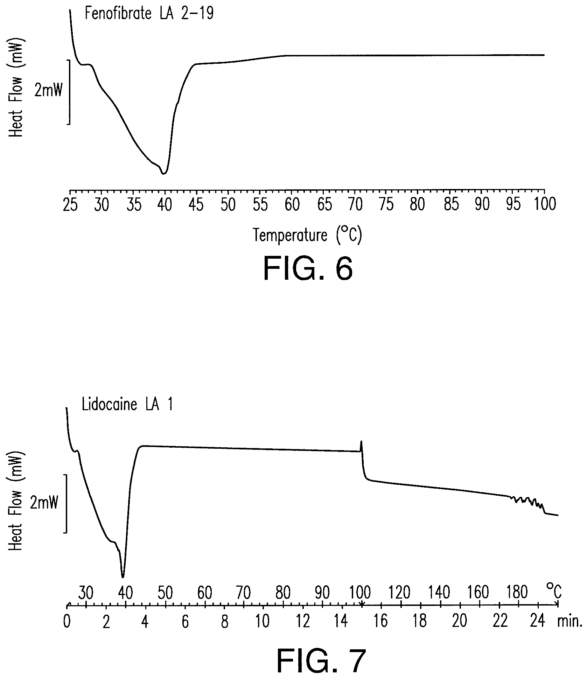

The formulation solidified with a clear appearance. In addition, DSC analysis indicated a new broad melting point temperature range of 34°C to 39°C (FIG. 6). This temperature range was different than that for the individual components alone: 41°C to 45°C for GELUCIRE® 43/01 (Gattefosse) and 80°C to 85°C for Fenofibrate. Isosorbide dimethyl ester is a liquid at room temperature, and as such has a melting point below 20°C. These results indicate that a solid solution formulation as disclosed herein comprising Fenofibrate was formed.

### Example 7

### Solid solution pharmaceutical compositions comprising Gemifibrozil

This example illustrates how to make a solid solution pharmaceutical composition disclosed herein comprising Gemifibrozil.

To prepare a solid solution pharmaceutical composition disclosed herein using Gemifibrozil, the following method was performed. 1 g Gemifibrozil, 0.9 mL MAISINE® 35-1 (Gattefosse), a glyceryl monolinoleate (as room temperature liquid lipid), and 0.4 mL PEG 400 (as stabilizing agent) were added to a vessel and heated to 50°C to 60°C with stirring until all components of the mixture were dissolved. 1.9 g GELUCIRE® 43/01 (Gattefosse), a waxy solid (as room temperature solid lipid) having a melting point of between 41°C to 45°C and comprising a mixture of saturated C₁₀-C₁₈ triglycerides, was added to this solution and stirred until incorporated. The heated mixture was then cooled to 37°C to 40°C, and aliquoted by pouring into molds and cooled to room temperature. Alternatively, the mixture can be cooled to room temperature and then subsequently reheated to 40°C to 45°C for aliquoting into molds. The formulation solidified with a clear appearance and remelted without forming a precipitate. These results indicate that a solid solution formulation as disclosed herein comprising Gemifibrozil was formed.

To prepare a solid solution pharmaceutical composition disclosed herein using Gemifibrozil, the following method was performed. 1 g Gemifibrozil and 7.5 g Cocoa Butter a waxy solid (as room temperature solid lipid) having a melting point of between 34°C to 38°C and comprising a mixture of saturated C₁₆-C₁₈ triglycerides, were added to a vessel and heated to 50°C to 60°C with stirring until all components of the mixture were incorporated. The heated mixture was then cooled to 37°C to 40°C, and aliquoted by pouring into molds and cooled to room temperature. Alternatively, the mixture can be cooled to room temperature and then subsequently reheated to 40°C to 45°C for aliquoting into molds. The formulation solidified with a clear appearance and remelted without forming a precipitate. These results indicate that a solid solution formulation as disclosed herein comprising Gemifibrozil was formed.

### Example 8

### Solid solution pharmaceutical compositions comprising Ibuprofen

This example illustrates how to make a solid solution pharmaceutical composition disclosed herein comprising Ibuprofen.

To prepare a solid solution pharmaceutical composition disclosed herein using Ibuprofen, the following method was performed. 1 g ibuprofen sodium salt, 0.9 mL MAISINE® 35-1 (Gattefosse), a glyceryl monolinoleate (as room temperature liquid lipid), and 0.4 mL PEG 400 were added to a vessel heated to 50°C to 60°C with stirring until all components of the mixture were dissolved. 1.9 g GELUCIRE® 43/01 (Gattefosse), a waxy solid (as room temperature solid lipid) having a melting point of between 41°C to 45°C and comprising a mixture of saturated C₁₀-C₁₈ triglycerides, was added to this solution and stirred until incorporated. The heated mixture was then cooled to 37°C to 40°C, and aliquoted by pouring into molds and cooled to room temperature. Alternatively, the mixture can be cooled to room temperature and then subsequently reheated to 40°C to 45°C for aliquoting into molds.

The formulation solidified with a clear appearance. In addition, DSC analysis indicated a new broad melting point temperature range of 32°C to 44°C (FIG. 1D). This temperature range was different than that for the individual components alone: 41°C to 45°C for GELUCIRE® 43/01 (Gattefosse) and 75°C to 78°C for Ibuprofen. MAISINE® 35-1 and PEG 400 are liquids at room temperature, and as such both have melting points below 20°C. These results indicate that a solid solution formulation as disclosed herein comprising Ibuprofen was formed.

To prepare a solid solution pharmaceutical composition disclosed herein using Ibuprofen, the following method was performed. 1 g ibuprofen sodium salt, 0.5 mL MAISINE® 35-1 (Gattefosse), a glyceryl monolinoleate (as room temperature liquid lipid), and 0.4 mL PEG 400 were added to a vessel heated to 50°C to 60°C with stirring until all components of the mixture were dissolved. 2.3 g GELUCIRE® 43/01 (Gattefosse), a waxy solid (as room temperature solid lipid) having a melting point of between 41°C to 45°C and comprising a mixture of saturated C₁₀-C₁₈ triglycerides, was added to this solution and stirred until incorporated. The heated mixture was then cooled to 37°C to 40°C, and aliquoted by pouring into molds and cooled to room temperature. Alternatively, the mixture can be cooled to room temperature and then subsequently reheated to 40°C to 45°C for aliquoting into molds.

The formulation solidified with a clear appearance. In addition, DSC analysis indicated a new broad melting point temperature range of 32°C to 43°C (FIG. 1E). This temperature range was different than that for the individual components alone: 41°C to 45°C for GELUCIRE® 43/01 (Gattefosse) and 75°C to 78°C for Ibuprofen. MAISINE® 35-1 and PEG 400 are liquids at room temperature, and as such both have melting points below 20°C. These results indicate that a solid solution formulation as disclosed herein comprising Ibuprofen was formed.

To prepare a solid solution pharmaceutical composition disclosed herein using Ibuprofen, the following method was performed. 1 g ibuprofen sodium salt, 0.2 mL MAISINE® 35-1 (Gattefosse), a glyceryl monolinoleate (as room temperature liquid lipid), and 0.4 mL PEG 400 were added to a vessel heated to 50°C to 60°C with stirring until all components of the mixture were dissolved. 2.6 g GELUCIRE® 43/01 (Gattefosse), a waxy solid (as room temperature solid lipid) having a melting point of between 41°C to 45°C and comprising a mixture of saturated C₁₀-C₁₈ triglycerides, was added to this solution and stirred until incorporated. The heated mixture was then cooled to 37°C to 40°C, and aliquoted by pouring into molds and cooled to room temperature. Alternatively, the mixture can be cooled to room temperature and then subsequently reheated to 40°C to 45°C for aliquoting into molds.

The formulation solidified with a clear appearance. In addition, DSC analysis indicated a new broad melting point temperature range of 32°C to 42°C (FIG. 1F). This temperature range was different than that for the individual components alone: 41°C to 45°C for GELUCIRE® 43/01 (Gattefosse) and 75°C to 78°C for Ibuprofen. MAISINE® 35-1 and PEG 400 are liquids at room temperature, and as such both have melting points below 20°C. These results indicate that a solid solution formulation as disclosed herein comprising Ibuprofen was formed.

To prepare a solid solution pharmaceutical composition disclosed herein using Ibuprofen, the following method was performed. 5 g ibuprofen sodium salt, 9.5 mL MAISINE® 35-1 (Gattefosse), a glyceryl monolinoleate (as room temperature liquid lipid), and 1.0 mL PEG 400 were added to a vessel heated to 50°C to 60°C with stirring until all components of the mixture were dissolved. 4.0 g GELUCIRE® 43/01 (Gattefosse), a waxy solid (as room temperature solid lipid) having a melting point of between 41°C to 45°C and comprising a mixture of saturated C₁₀-C₁₈ triglycerides, was added to this solution and stirred until incorporated. The heated mixture was then cooled to 37°C to 40°C, and aliquoted by pouring into molds and cooled to room temperature. Alternatively, the mixture can be cooled to room temperature and then subsequently reheated to 40°C to 45°C for aliquoting into molds.

The formulation solidified with a clear appearance. In addition, DSC analysis indicated a new broad melting point temperature range of 32°C to 38°C (FIG. 1G). This temperature range was different than that for the individual components alone: 41°C to 45°C for GELUCIRE® 43/01 (Gattefosse) and 75°C to 78°C for Ibuprofen. MAISINE® 35-1 and PEG 400 are liquids at room temperature, and as such both have melting points below 20°C. These results indicate that a solid solution formulation as disclosed herein comprising Ibuprofen was formed.

To prepare a solid solution pharmaceutical composition disclosed herein using Ibuprofen, the following method was performed. 5 g ibuprofen sodium salt, 6.5 mL MAISINE® 35-1 (Gattefosse), a glyceryl monolinoleate (as room temperature liquid lipid), and 1.5 mL PEG 400 were added to a vessel heated to 50°C to 60°C with stirring until all components of the mixture were dissolved. 6.5 g GELUCIRE® 43/01 (Gattefosse), a waxy solid (as room temperature solid lipid) having a melting point of between 41°C to 45°C and comprising a mixture of saturated C₁₀-C₁₈ triglycerides, was added to this solution and stirred until incorporated. The heated mixture was then cooled to 37°C to 40°C, and aliquoted by pouring into molds and cooled to room temperature. Alternatively, the mixture can be cooled to room temperature and then subsequently reheated to 40°C to 45°C for aliquoting into molds.

The formulation solidified with a clear appearance. In addition, DSC analysis indicated a new broad melting point temperature range of 32°C to 42°C (FIG. 1H). This temperature range was different than that for the individual components alone: 41°C to 45°C for GELUCIRE® 43/01 (Gattefosse) and 75°C to 78°C for Ibuprofen. MAISINE® 35-1 and PEG 400 are liquids at room temperature, and as such both have melting points below 20°C. These results indicate that a solid solution formulation as disclosed herein comprising Ibuprofen was formed.

To prepare a solid solution pharmaceutical composition disclosed herein using Ibuprofen, the following method was performed. 1 g ibuprofen sodium salt, 0.9 mL MAISINE® 35-1 (Gattefosse), a glyceryl monolinoleate (as room temperature liquid lipid), 0.4 mL PEG 400, and 0.3 mL propylene glycol were added to a vessel heated to 50°C to 60°C with stirring until all components of the mixture were dissolved. 1.9 g GELUCIRE® 43/01 (Gattefosse), a waxy solid (as room temperature solid lipid) having a melting point of between 41°C to 45°C and comprising a mixture of saturated C₁₀-C₁₈ triglycerides, was added to this solution and stirred until incorporated. The heated mixture was then cooled to 37°C to 40°C, and aliquoted by pouring into molds and cooled to room temperature. Alternatively, the mixture can be cooled to room temperature and then subsequently reheated to 40°C to 45°C for aliquoting into molds. The formulation solidified with a clear appearance and remelted without forming a precipitate. These results indicate that a solid solution formulation as disclosed herein comprising Ibuprofen was formed.

To prepare a solid solution pharmaceutical composition disclosed herein using Ibuprofen, the following method was performed. 5 g ibuprofen free acid, 5 g ibuprofen sodium salt, 8 mL MAISINE® 35-1 (Gattefosse), a glyceryl monolinoleate (as room temperature liquid lipid), 3 mL PEG 400, and 1 mL propylene glycol were added to a vessel heated to 50°C to 60°C with stirring until all components of the mixture were dissolved. 19 g GELUCIRE® 43/01 (Gattefosse), a waxy solid (as room temperature solid lipid) having a melting point of between 41°C to 45°C and comprising a mixture of saturated C₁₀-C₁₈ triglycerides, was added to this solution and stirred until incorporated. The heated mixture was then cooled to 37°C to 40°C, and aliquoted by pouring into molds and cooled to room temperature. Alternatively, the mixture can be cooled to room temperature and then subsequently reheated to 40°C to 45°C for aliquoting into molds. The formulation solidified with a clear appearance and remelted without forming a precipitate. These results indicate that a solid solution formulation as disclosed herein comprising Ibuprofen was formed.

### Example 9

### Solid solution pharmaceutical compositions comprising Lidocaine

This example illustrates how to make a solid solution pharmaceutical composition disclosed herein comprising Lidocaine.

To prepare a solid solution pharmaceutical composition disclosed herein using Lidocaine, the following method was performed. 200 mg Lidocaine base and 2.0 mL isosorbide dimethyl ester (as stabilizing agent) were added to a vessel and heated to 50°C to 60°C with stirring until all components of the mixture were dissolved. 8.8 g GELUCIRE® 43/01 (Gattefosse), a waxy solid (as room temperature solid lipid) having a melting point of between 41°C to 45°C and comprising a mixture of saturated C₁₀-C₁₈ triglycerides, was added to this solution and stirred until incorporated. The heated mixture was then cooled to 37°C to 40°C, and aliquoted by pouring into molds and cooled to room temperature. Alternatively, the mixture can be cooled to room temperature and then subsequently reheated to 40°C to 45°C for aliquoting into molds.

The formulation solidified with a clear appearance. In addition, DSC analysis indicated a new broad melting point temperature range of 34°C to 40°C (FIG. 7). This temperature range was different than that for the individual components alone: 41°C to 45°C for GELUCIRE® 43/01 (Gattefosse) and 66°C to 69°C for Lidocaine. Isosorbide dimethyl ester is a liquid at room temperature, and as such has a melting point below 20°C. These results indicate that a solid solution formulation as disclosed herein comprising Lidocaine was formed.

To prepare a solid solution pharmaceutical composition disclosed herein using Lidocaine, the following method was performed. 250.1 mg Lidocaine base and 1.74 mL isosorbide dimethyl ester (as stabilizing agent) were added to a vessel and heated to 50°C to 60°C with stirring until all components of the mixture were dissolved. 8.72 g GELUCIRE® 43/01 (Gattefosse), a waxy solid (as room temperature solid lipid) having a melting point of between 41°C to 45°C and comprising a mixture of saturated C₁₀-C₁₈ triglycerides, was added to this solution and stirred until incorporated. The heated mixture was then cooled to 37°C to 40°C, and aliquoted by pouring into molds and cooled to room temperature. Alternatively, the mixture can be cooled to room temperature and then subsequently reheated to 40°C to 45°C for aliquoting into molds.

To prepare a solid solution pharmaceutical composition disclosed herein using Lidocaine, the following method was performed. 500.4 mg Lidocaine base and 1.74 mL isosorbide dimethyl ester (as stabilizing agent) were added to a vessel and heated to 50°C to 60°C with stirring until all components of the mixture were dissolved. 8.5 g GELUCIRE® 43/01 (Gattefosse), a waxy solid (as room temperature solid lipid) having a melting point of between 41°C to 45°C and comprising a mixture of saturated C₁₀-C₁₈ triglycerides, was added to this solution and stirred until incorporated. The heated mixture was then cooled to 37°C to 40°C, and aliquoted by pouring into molds and cooled to room temperature. Alternatively, the mixture can be cooled to room temperature and then subsequently reheated to 40°C to 45°C for aliquoting into molds.

To prepare a solid solution pharmaceutical composition disclosed herein using Lidocaine, the following method was performed. 250.4 mg Lidocaine base and 0.87 mL isosorbide dimethyl ester (as stabilizing agent) were added to a vessel and heated to 50°C to 60°C with stirring until all components of the mixture were incorporated. Separately, 250.1 mg Prilocaine HCI base, 0.13 mL triethanolamine (as neutralizing agent), and 0.87 mL isosorbide dimethyl ester (as stabilizing agent) were added to a vessel and heated to 50°C to 60°C with stirring until all components of the mixture were incorporated. The Lidocaine and Prilocaine mixtures were combined and heated to 50°C to 60°C with stirring until all components were dissolved. 8.49 g GELUCIRE® 43/01 (Gattefosse), a waxy solid (as room temperature solid lipid) having a melting point of between 41°C to 45°C and comprising a mixture of saturated C₁₀-C₁₈ triglycerides, was added to this solution and stirred until incorporated. The heated mixture was then cooled to 37°C to 40°C, and aliquoted by pouring into molds and cooled to room temperature. Alternatively, the mixture can be cooled to room temperature and then subsequently reheated to 40°C to 45°C for aliquoting into molds.

### Example 10

### Solid solution pharmaceutical compositions comprising Nabumetone

This example illustrates how to make a solid solution pharmaceutical composition disclosed herein comprising Nabumetone.

To prepare a solid solution pharmaceutical composition disclosed herein using Nabumetone, the following method was performed. 126 mg Nabumetone and 0.5 mL MAISINE® 35-1 (Gattefosse), a glyceryl monolinoleate (as room temperature liquid lipid), were added to a vessel and heated to 50°C to 60°C with stirring until all components of the mixture were dissolved. 1.0 g GELUCIRE® 43/01 (Gattefosse), a waxy solid (as room temperature solid lipid) having a melting point of between 41°C to 45°C and comprising a mixture of saturated C₁₀-C₁₈ triglycerides, was added to this solution and stirred until incorporated. The heated mixture was then cooled to 37°C to 40°C, and aliquoted by pouring into molds and cooled to room temperature. Alternatively, the mixture can be cooled to room temperature and then subsequently reheated to 40°C to 45°C for aliquoting into molds.

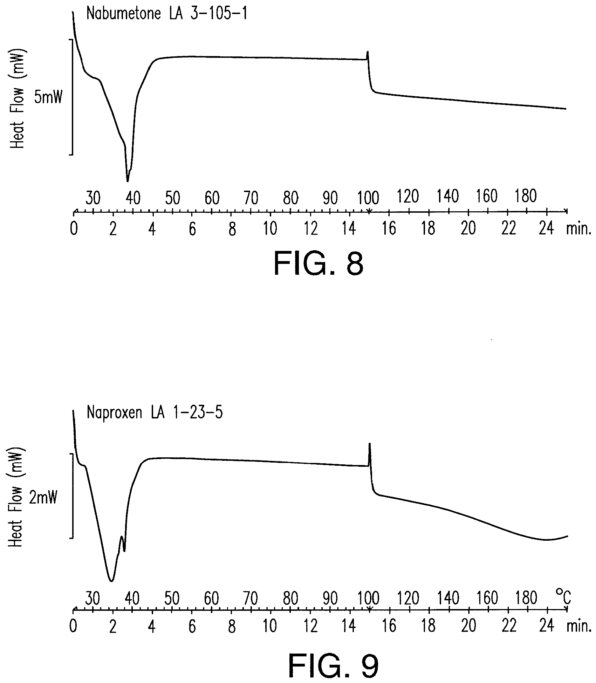

The formulation solidified with a clear appearance. In addition, DSC analysis indicated a new broad melting point temperature range of 35°C to 40°C (FIG. 8). This temperature range was different than that for the individual components alone: 41°C to 45°C for GELUCIRE® 43/01 (Gattefosse) and 80°C to 81°C for Nabumetone. MAISINE® 35-1 is a liquid at room temperature, and as such has a melting point below 20°C. These results indicate that a solid solution formulation as disclosed herein comprising Nabumetone was formed.

### Example 11

### Solid solution pharmaceutical compositions comprising Naproxen

This example illustrates how to make a solid solution pharmaceutical composition disclosed herein comprising Naproxen.

To prepare a solid solution pharmaceutical composition disclosed herein using Naproxen, the following method was performed. 250.1 mg Naproxen and 0.75 mL MAISINE® 35-1 (Gattefosse), a glyceryl monolinoleate (as room temperature liquid lipid), were added to a vessel and heated to 50°C to 60°C with stirring until all components of the mixture were dissolved. 750.9 mg GELUCIRE® 43/01 (Gattefosse), a waxy solid (as room temperature solid lipid) having a melting point of between 41°C to 45°C and comprising a mixture of saturated C₁₀-C₁₈ triglycerides, was added to this solution and stirred until incorporated. The heated mixture was then cooled to 37°C to 40°C, and aliquoted by pouring into molds and cooled to room temperature. Alternatively, the mixture can be cooled to room temperature and then subsequently reheated to 40°C to 45°C for aliquoting into molds. The formulation solidified with a clear appearance. These results indicate that a solid solution formulation as disclosed herein comprising Naproxen was formed.

To prepare a solid solution pharmaceutical composition disclosed herein using Naproxen, the following method was performed. 650.5 mg Naproxen and 1.2 mL MAISINE® 35-1 (Gattefosse), a glyceryl monolinoleate (as room temperature liquid lipid), were added to a vessel and heated to 50°C to 60°C with stirring until all components of the mixture were dissolved. 1.234 g GELUCIRE® 43/01 (Gattefosse), a waxy solid (as room temperature solid lipid) having a melting point of between 41°C to 45°C and comprising a mixture of saturated C₁₀-C₁₈ triglycerides, was added to this solution and stirred until incorporated. The heated mixture was then cooled to 37°C to 40°C, and aliquoted by pouring into molds and cooled to room temperature. Alternatively, the mixture can be cooled to room temperature and then subsequently reheated to 40°C to 45°C for aliquoting into molds.

The formulation solidified with a clear appearance. In addition, DSC analysis indicated a new broad melting point temperature range of 30°C to 39°C (FIG. 9). This temperature range was different than that for the individual components alone: 41°C to 45°C for GELUCIRE® 43/01 (Gattefosse) and 153°C to 154°C for Naproxen. MAISINE® 35-1 is a liquid at room temperature, and as such has a melting point below 20°C. These results indicate that a solid solution formulation as disclosed herein comprising Naproxen was formed.

### Example 12

### Solid solution pharmaceutical compositions comprising Pentoxifylline

This example illustrates how to make a solid solution pharmaceutical composition disclosed herein comprising Pentoxifylline.

To prepare a solid solution pharmaceutical composition disclosed herein using Pentoxifylline, the following method was performed. 208 mg Pentoxifylline, 1.0 mL MAISINE® 35-1 (Gattefosse), a glyceryl monolinoleate (as room temperature liquid lipid), and 0.2 mL isosorbide dimethyl ester (as stabilizing agent) were added to a vessel and heated to 50°C to 60°C with stirring until all components of the mixture were dissolved. 1.0 g GELUCIRE® 43/01 (Gattefosse), a waxy solid (as room temperature solid lipid) having a melting point of between 41°C to 45°C and comprising a mixture of saturated C₁₀-C₁₈ triglycerides, was added to this solution and stirred until incorporated. The heated mixture was then cooled to 37°C to 40°C, and aliquoted by pouring into molds and cooled to room temperature. Alternatively, the mixture can be cooled to room temperature and then subsequently reheated to 40°C to 45°C for aliquoting into molds. The formulation solidified with a clear appearance and remelted without forming a precipitate. These results indicate that a solid solution formulation as disclosed herein comprising Pentoxifylline was formed.

### Example 13

### Solid solution pharmaceutical compositions comprising Salbutamol

This example illustrates how to make a solid solution pharmaceutical composition disclosed herein comprising Salbutamol.

To prepare a solid solution pharmaceutical composition disclosed herein using Salbutamol, the following method was performed. 61 mg Salbutamol, 0.6 mL MAISINE® 35-1 (Gattefosse), a glyceryl monolinoleate (as room temperature liquid lipid), 1.0 mL isosorbide dimethyl ether (as stabilizing agent), and 1.0 mL absolute ethanol (as stabilizing agent) were added to a vessel and heated to 50°C to 60°C with stirring until all components of the mixture were dissolved. 10 g GELUCIRE® 43/01 (Gattefosse), a waxy solid (as room temperature solid lipid) having a melting point of between 41°C to 45°C and comprising a mixture of saturated C₁₀-C₁₈ triglycerides, was added to this solution and stirred until incorporated. The heated mixture was then cooled to 37°C to 40°C, and aliquoted by pouring into molds and cooled to room temperature. Alternatively, the mixture can be cooled to room temperature and then subsequently reheated to 40°C to 45°C for aliquoting into molds.

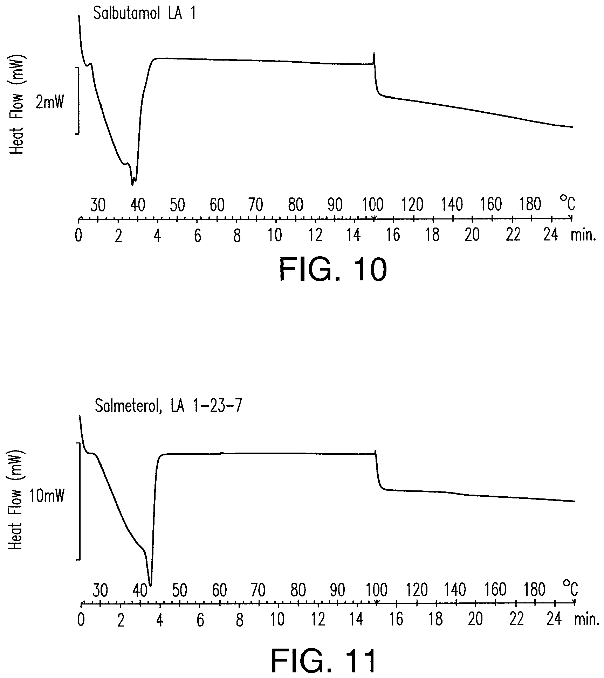

The formulation solidified with a clear appearance. In addition, DSC analysis indicated a new broad melting point temperature range of 32°C to 40°C (FIG. 10). This temperature range was different than that for the individual components alone: 41°C to 45°C for GELUCIRE® 43/01 (Gattefosse) and 157°C to 158°C for Salbutamol. MAISINE® 35-1, isosorbide dimethyl ether, and absolute ethanol are liquids at room temperature, and as such all have melting points below 20°C. These results indicate that a solid solution formulation as disclosed herein comprising Salbutamol was formed.

### Example 14

### Solid solution pharmaceutical compositions comprising Salmeterol

This example illustrates how to make a solid solution pharmaceutical composition disclosed herein comprising Salmeterol.

To prepare a solid solution pharmaceutical composition disclosed herein using Salmeterol, the following method was performed. 11 mg Salmeterol xinafoate, 1.0 mL MAISINE® 35-1 (Gattefosse), a glyceryl monolinoleate (as room temperature liquid lipid), and 1.0 mL absolute ethanol (as stabilizing agent) were added to a vessel and heated to 50°C to 60°C with stirring until all components of the mixture were dissolved. 2.04 g GELUCIRE® 43/01 (Gattefosse), a waxy solid (as room temperature solid lipid) having a melting point of between 41°C to 45°C and comprising a mixture of saturated C₁₀-C₁₈ triglycerides, was added to this solution and stirred until incorporated. The heated mixture was then cooled to 37°C to 40°C, and aliquoted by pouring into molds and cooled to room temperature. Alternatively, the mixture can be cooled to room temperature and then subsequently reheated to 40°C to 45°C for aliquoting into molds.

The formulation solidified with a clear appearance. In addition, DSC analysis indicated a new broad melting point temperature range of 34°C to 43°C (FIG. 11). This temperature range was different than that for the individual components alone: 41°C to 45°C for GELUCIRE® 43/01 (Gattefosse) and 137°C to 138°C for Salmeterol. MAISINE® 35-1 and absolute ethanol are liquids at room temperature, and as such both have melting points below 20°C. These results indicate that a solid solution formulation as disclosed herein comprising Salmeterol was formed.

### Example 15

### Solid solution pharmaceutical compositions comprising Simvastatin

This example illustrates how to make a solid solution pharmaceutical composition disclosed herein comprising Simvastatin.

To prepare a solid solution pharmaceutical composition disclosed herein using Simvastatin, the following method was performed. 200 mg Simvastatin and 2.5 mL MAISINE® 35-1 (Gattefosse), a glyceryl monolinoleate (as room temperature liquid lipid) were added to a vessel and heated to 50°C to 60°C with stirring until all components of the mixture were dissolved. 5 g GELUCIRE® 43/01 (Gattefosse), a waxy solid (as room temperature solid lipid) having a melting point of between 41°C to 45°C and comprising a mixture of saturated C₁₀-C₁₈ triglycerides, was added to this solution and stirred until incorporated. The heated mixture was then cooled to 37°C to 40°C, and aliquoted by pouring into molds and cooled to room temperature. Alternatively, the mixture can be cooled to room temperature and then subsequently reheated to 40°C to 45°C for aliquoting into molds.

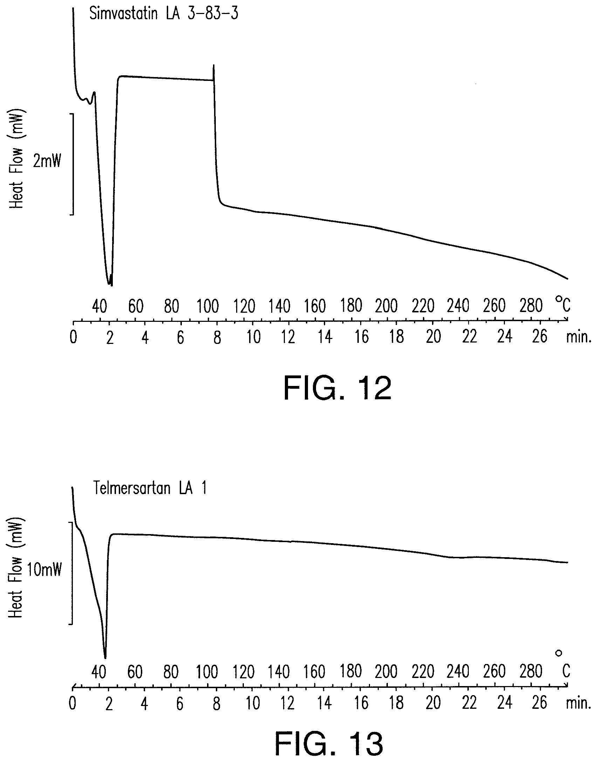

The formulation solidified with a clear appearance. In addition, DSC analysis indicated a new broad melting point temperature range of 32°C to 42°C (FIG. 12). This temperature range was different than that for the individual components alone: 41°C to 45°C for GELUCIRE® 43/01 (Gattefosse) and 135°C to 138°C for Simvastatin. MAISINE® 35-1 and absolute ethanol are liquids at room temperature, and as such both have melting points below 20°C. These results indicate that a solid solution formulation as disclosed herein comprising Simvastatin was formed.

### Example 16

### Solid solution pharmaceutical compositions comprising Telmisartan

This example illustrates how to make a solid solution pharmaceutical composition disclosed herein comprising Telmisartan.

To prepare a solid solution pharmaceutical composition disclosed herein using Telmisartan, the following method was performed. 60.1 mg Telmisartan and 2.0 mL isosorbide dimethyl ether (as stabilizing agent) were added to a vessel and heated to 50°C to 60°C with stirring until all components of the mixture were dissolved. 2.03 g GELUCIRE® 43/01 (Gattefosse), a waxy solid (as room temperature solid lipid) having a melting point of between 41°C to 45°C and comprising a mixture of saturated C₁₀-C₁₈ triglycerides, was added to this solution and stirred until incorporated. The heated mixture was then cooled to 37°C to 40°C, and aliquoted by pouring into molds and cooled to room temperature. Alternatively, the mixture can be cooled to room temperature and then subsequently reheated to 40°C to 45°C for aliquoting into molds. The formulation solidified with a clear appearance and remelted without forming a precipitate. These results indicate that a solid solution formulation as disclosed herein comprising Telmisartan was formed.

To prepare a solid solution pharmaceutical composition disclosed herein using Telmisartan, the following method was performed. 160.2 mg Telmisartan, 1.0 mL MAISINE® 35-1 (Gattefosse), a glyceryl monolinoleate (as room temperature liquid lipid), and 1.0 mL absolute ethanol (as stabilizing agent) were added to a vessel and heated to 50°C to 60°C with stirring until all components of the mixture were dissolved. 2.03 g GELUCIRE® 43/01 (Gattefosse), a waxy solid (as room temperature solid lipid) having a melting point of between 41°C to 45°C and comprising a mixture of saturated C₁₀-C₁₈ triglycerides, was added to this solution and stirred until incorporated. The heated mixture was then cooled to 37°C to 40°C, and aliquoted by pouring into molds and cooled to room temperature. Alternatively, the mixture can be cooled to room temperature and then subsequently reheated to 40°C to 45°C for aliquoting into molds.

The formulation solidified with a clear appearance. In addition, DSC analysis indicated a new broad melting point temperature range of 34°C to 43°C (FIG. 13). This temperature range was different than that for the individual components alone: 41°C to 45°C for GELUCIRE® 43/01 (Gattefosse) and 261°C to 263°C for Telmisartan. MAISINE® 35-1 and absolute ethanol are liquids at room temperature, and as such both have melting points below 20°C. These results indicate that a solid solution formulation as disclosed herein comprising Telmisartan was formed.

### Example 17

### Macrophage Uptake Experiment

This example illustrates that a solid solution pharmaceutical composition disclosed herein preferentially targets a therapeutic compound to the immune system.

Cultures of U937 monocyte cell line were grown in RPMI-1640 supplemented with 10% fetal calf serum (FCS) until the cells reached 90% confluent monolayer. These cells were then treated with PMA and incubated in a 37°C incubator under 5% carbon dioxide until the cells differentiated into macrophages. Monolayers of macrophages were washed with fresh medium and then 3 mL of one of the following test solutions were added: A) solid solution formulation as disclosed herein comprising ibuprofen, GELUCIRE® 43/01 (Gattefosse), MAISINE® 35-1 (Gattefosse), and PEG 400; B) liquid formulation disclosed herein comprising ibuprofen, rapeseed oil and ethanol; C) ibuprofen free acid; and D) vehicle with no therapeutic compound. After incubation for 45 minutes the test solution supernatants were removed and saved for analysis, and the cells were then washed in PBS several times and lysed using two cycles of freeze-thawing. Therapeutic compound concentration present in the test solution, test solution supernatant, and cell lysate fractions was measured by HPLC. The percentage therapeutic compound taken up by the macrophages was calculated using the following formula: % therapeutic compound adsorbed = 100 × (compound mass recovered from cell lysate) / (compound mass delivered in test solution - compound mass recovered from test solution supernatant). Results are shown in the Table 1 below. These results indicate that mean uptake of a therapeutic compound by macrophage increase 550% or more using the formulations of the pharmaceutical compositions presently claimed relative to compositions not formulated in this manner.

| **Table 3. Macrophage Uptake of Therapeutic Compound** | | | |
|---|---|---|---|
| **Formulation** | **N** | **Mean Mass Compound Uptake** | **% Increase Compound Uptake** |
| A | 2 | 2.4% | 600% |
| B | 2 | 2.2% | 550% |
| C | 2 | 0.4% | - |
| D | 2 | 0.0% | - |

### Example 18

### Animal Model for Intestinal Erosion

To assess whether a pharmaceutical composition disclosed herein reduced gastric irritation, experiments were conducted using an intestinal erosion murine model.

Sprague-Dawley rats were divided into seven experimental groups containing five animals each. After fasting overnight, the animals were challenged with one with one of seven different treatments. Group A was a control in which each mouse was orally administered 1% methylcellulose/0.5% polysorbate 80 vehicle only. Group B was a control in which each mouse was orally administered solvent/adjuvant vehicle only (gavage of 10% ethanol and 90% linseed oil). Group C was a control in which each mouse was orally administered 150 mg/kg aspirin. Group D was a control in which each mouse was orally administered 100 mg/kg ibuprofen suspended in 1% methylcellulose/0.5% polysorbate 80. Group E was the experimental group in which each mouse was administered a pharmaceutical composition disclosed herein (BC1054-100) comprising 100 mg/kg of ibuprofen, 10% ethanol, and 90% linseed oil. Group F was a control in which each mouse was orally administered 100 mg/kg ibuprofen suspended in 1% methylcellulose/0.5% polysorbate 80. Group G was the experimental group in which each mouse was administered a pharmaceutical composition disclosed herein (BC1054-200) comprising 200 mg/kg of ibuprofen, 10% ethanol, and 90% linseed oil. Animals were sacrificed 4 hours after treatment and the stomachs were examined for degree of hemorrhage and severity of mucosal erosive lesions. Gastric irritation was scored as follows: 0, no lesions; 1, hyperemia; 2, one or two slight lesions; 3, more than two slight lesions or severe lesions; and 4, very severe lesions. A score of 50% or more relative to Group C (aspirin-treated control group), which was set to 100%, was considered a positive score for gastric irritation.

Results are shown in Table 4. Group D (100 mg/kg of ibuprofen-treated control group) and Group F (200 mg/kg of ibuprofen-treated control group) produced gastric lesions that were 75% and 95%, respectively, severe as those induced by Group C (aspirin-treated control group). However, Group E (BC1054-100-treated experimental group) and Group G (BC1054-200-treated experimental group) produced gastric lesions that were 20% and 40%, respectively, as severe as those associated with Group C (aspirin-treated control group). These results demonstrate that that a pharmaceutical composition disclosed herein reduced the extent to which a therapeutic compound may cause mucosal lesions and cause gastric irritation.

| **Table 4. Results from Intestinal Erosion Assay** | | |
|---|---|---|
| **Group** | **Mean Ulceration Score** | **% Aspirin Erosion** |
| A | 0 | 0 |
| B | 0 | 0 |
| C | 4 | (100) |
| D | 3 | 75¹ |
| E | 0.8 | 20 |
| F | 3.8 | 95¹ |
| G | 1.6 | 40 |
| ¹ Positive score for gastric erosion. | | |

### Example 19

### Animal Model for Inflammatory Bowel Disease

To assess the effectiveness of a pharmaceutical composition disclosed herein in treating an inflammatory bowel disease, experiments were conducted using a TBS-induced colitis murine model.

C57BI/6 male mice (6-7 weeks old) were divided into seven experimental groups containing at least ten animals each. On day 0, colitis was induced in mice from Groups B-G by intrarectal administration of 100 µL of TNBS (4 mg) in 50% ethanol under isoflurane anesthesia. Animals were dosed either once or three times a day from day -1 to day 5 with one of seven different treatments. Group A was a control in which each mouse was orally administered ethanol vehicle only. Group B was a control in which each mouse was orally administered 1% methylcellulose vehicle only. Group C was a control in which each mouse was orally administered solvent/adjuvant vehicle only (gavage of 10% ethanol and 90% linseed oil). Group D was a control in which each mouse was orally administered 3 mg/kg of Prednisolone. Group E was a control in which each mouse was orally administered 20 mg/kg of ibuprofen suspended in 1% methylcellulose (1 mL/kg) (no adjuvant). Group F was the experimental group in which each mouse was administered a pharmaceutical composition disclosed herein (BC1054-20) comprising 20 mg/kg of ibuprofen, 10% ethanol, and 90% linseed oil. Group G was the experimental group in which each mouse was administered a pharmaceutical composition disclosed herein (BC1054-30) comprising 30 mg/kg of ibuprofen, 10% ethanol, and 90% linseed oil. All animals were weighed daily and assessed visually for the presence of diarrhea and/or bloody stool. On day 3 and on day 5 colitis severity was assessed in all animals using video endoscopy, where images were taken and colitis severity scored visually by a blinded observer on a scale from 0 to 4 as follows: 0, normal; 1, loss of vascularity; 2, loss of vascularity and friability; 3, friability and erosions; and 4, ulcerations and bleeding. Following endoscopy on day 5, animals were sacrificed and the colon removed and its length and weight measured. Serum samples were obtained and the colon was fixed in 10% formalin. An additional piece of colon tissue was collected, weighed, and snap frozen in liquid nitrogen.

Results from these experiments are shown in Table 5. Group B (TNBS-treated control group) showed a statistically significant difference in mean weight change when compared to Group A (untreated ethanol control group), all other group comparisons showed no difference in mean weight change. Group B (TNBS-treated control group) showed a statistically significant decrease in mean colon length when compared to Group A (untreated ethanol control group). Additionally, Group D (Prednisolone-treated control group), Group F (BC1054-20-treated experimental group), and Group G (BC1054-30-treated experimental group) all showed a statistically significant increase in mean colon length when compared to Group B (TNBS-treated control group). Although Group B (TNBS-treated control group) showed a statistically significant increase in mean colon weight when compared to Group A (untreated ethanol control group), all other group comparisons showed no difference in mean colon weight. With regards to the endoscopy colitis score, Group D (Prednisolone-treated control group) showed a statistically significant reduced mean colitis scores on both day 3 and day 5 when compared to Group B (TNBS-treated control group). In a similar manner, both Group F (BC1054-20-treated experimental group) and Group G (BC1054-30-treated experimental group) showed a statistically significant reduced mean colitis scores on day 5 when compared to Group B (TNBS-treated control group). These results indicate that a pharmaceutical composition disclosed herein was effective in treating an inflammatory bowel disease.

| **Table 5. Results from Inflammatory Bowel Disease** | | | | | |
|---|---|---|---|---|---|
| **Group** | **Mean Animal Weight** | **Mean Colon Length** | **Mean Colon Weight** | **Endoscopy Colitis Severity Score** | |
| | | | | **Day 3** | **Day 5** |
| A | 23.93 g | 8.5 cm | 215 mg | 0.2 | 0 |
| B | 21.98 g¹ | 7.3 cm² | 295 mg⁶ | 3.1 | 2.7 |
| C | 23.64 g | 7.8 cm | 239 mg | 2.9 | 2.4 |
| D | 23.33 g | 8.4 cm³ | 267 mg | 2.3⁷ | 1.7⁸ |
| E | 23.82 g | 7.9 cm | 267 mg | 2.7 | 2.2 |
| F | 23.69 g | 8.4 cm⁴ | 258 mg | 2.6 | 1.9⁹ |
| G | 24.25 g | 7.9 cm⁵ | 284 mg | 2.4 | 1.4¹⁰ |
| ¹ Statistically significance difference compared to Group A (p = 0.029). | | | | | |
| ² Statistically significance difference compared to Group A (p = 0.001). | | | | | |
| ³ Statistically significance difference compared to Group B (p = 0.001). | | | | | |
| ⁴ Statistically significance difference compared to Group B (p = 0.001). | | | | | |
| ⁵ Statistically significance difference compared to Group B (p = 0.034). | | | | | |
| ⁶ Statistically significance difference compared to Group A (p = 0.009). | | | | | |
| ⁷ Statistically significance difference compared to Group B (p = 0.005). | | | | | |
| ⁸ Statistically significance difference compared to Group B (p = 0.002). | | | | | |
| ⁹ Statistically significance difference compared to Group B (p = 0.045). | | | | | |
| ¹⁰ Statistically significance difference compared to Group B (p = 0.002). | | | | | |

### Example 20

### Animal Model for a Systemic Arthritis

To assess the effectiveness of a pharmaceutical composition disclosed herein in treating arthritis, experiments were conducted using an α-collagen antibody induced arthritis (ACAIA) murine model that mimics a systemic arthritis like rheumatoid arthritis.

Male BALB/c mice were divided into eight groups, each containing 10 animals. To induce arthritic symptoms, mice from all eight groups were intravenously injected with 200 µL of an antibody solution comprising a 2 mg cocktail of four α-collagen II monoclonal antibodies (ARTHRITOMAB™, MD Biosciences) on study day 0 (study commencement), followed by a 200 µL intraperitoneal injection of a solution containing 100 µg lipopolysaccharide (LPS) on study day 3. Each group was subjected daily to a control or test treatment administered from day 0-11 as follows: Group 1 mice (1M) were treated orally with a vehicle preparation containing 1% methyl cellulose administered thrice daily; Group 2 mice (2M) were treated intraperitoneally with a positive control preparation containing 10mg/kg etanercept (ENBREL®, Wyeth) administered once daily; Group 3 mice (3M) were treated orally with a 20mg/kg test liquid formulation comprising ibuprofen and rapeseed oil (BC1054 LF-RO) administered once daily; Group 4 mice (4M) were treated orally with a 20mg/kg test liquid formulation comprising ibuprofen and rapeseed oil (BC1054 LF-RO) administered thrice daily; Group 5 mice (5M) were treated orally with a 20mg/kg test liquid formulation comprising ibuprofen and a glyceryl monolinoleate (MAISINE® 35-1, Gattefosse) (BC1054 LF-MA) administered thrice daily; Group 6 mice (6M) were treated orally with a 20mg/kg test solid formulation comprising ibuprofen and theobroma oil (BC1054 LF-TO) administered thrice daily; Group 7 mice (7M) were treated orally with a control preparation 1 comprising 20mg/kg of ibuprofen administered thrice daily; and Group 8 mice (8M) were treated orally with a 20mg/kg test solid formulation comprising ibuprofen and a waxy solid having a melting point of between 37°C to 41°C and comprising a mixture of saturated C₁₀-C₁₈ triglycerides (GELUCIRE® 39/01, Gattefosse) (BC1054 LF-GE) administered thrice daily (Table 6). The dose administered was calculated based on the assumption that each animal weighed, on average, 20g. A fixed volume of 100µL was administered to each mouse, except those animals receiving the positive control (2M) were administered 200µL.

| **Table 6. Constitution of Test Groups and Dose Levels** | | | | | | |
|---|---|---|---|---|---|---|
| **Group** | **Size** | **Treatment** | **Dose** | **Volume** | **Route** | **Regime** |
| 1M | 10 | Vehicle | N/A | 5ml/kg | PO | Thrice daily |
| 2M | 10 | Etanercept | 10mg/kg | 10ml/kg | IP | Once daily |
| 3M | 10 | BC1054 LF-RO | 20mg/kg | 5ml/kg | PO | Once daily |
| 4M | 10 | BC1054 LF-RO | 20mg/kg | 5ml/kg | PO | Thrice daily |
| 5M | 10 | BC1054 LF-MA | 20mg/kg | 5ml/kg | PO | Thrice daily |
| 6M | 10 | BC1054 SF-TO | 20mg/kg | 5ml/kg | PO | Thrice daily |
| 7M | 10 | Ibuprofen | 20mg/kg | 5ml/kg | PO | Thrice daily |
| 8M | 10 | BC1054 SF-GE | 20mg/kg | 5ml/kg | PO | Thrice daily |
| IP = Intraperitoneal | | | | | | |
| PO = Per Os | | | | | | |
| N/A = Not applicable | | | | | | |

Arthritic development and clinical examinations were monitored in all mice on study day 0 shortly before arthritis induction and subsequently on study days 3-7, 9, 10 and 12 (study termination). To access arthritic development, both an arthritis score and paw thickness (plethysmography) measurements were obtained. The arthritis score was based on visual assessment of arthritis reactions using a 0-4 scale in ascending order of severity with Grade 0 indicating no arthritis reaction; Grade 1 indicating mild, but definite redness and swelling of the ankle/wrist or apparent redness and swelling limited to individual digits, regardless of the number of affected digits; Grade 2 indicating moderate to severe redness and swelling of the ankle/wrist; Grade 3 indicating redness and swelling of the entire paw including digits; and Grade 4 indicating maximally inflamed limb with involvement of multiple joints. Paw thickness was measured for both hind paws just above the foot pad and below the calcaneum using a dial caliper (Kroeplin, Munich, Germany). Mean values for paw thickness measurements were determined, and where appropriate, analysis of the data by ANOVA with Tukey post hoc analysis was applied to determine significance of treatment effects.

Clinical examinations included changes in body weight, condition of skin, fur, eyes, mucous membranes, occurrence of secretions and excretions (e.g. diarrhea), and autonomic activity (e.g. lacrimation, salivation, piloerection, pupil size, unusual respiratory pattern). Changes in gait, posture and response to handling, as well as the presence of bizarre behavior, tremors, convulsions, sleep and coma were also noted. Serum was collected at study termination.

Arthritis incidence increased in all groups from day 3. In Group 1M animals incidence peaked on day 7 with 9/10 animals showing arthritis reactions which remained relatively constant until the end of the study. In Etanercept-treated Group 2M mice, incidence peaked on day 6 at 9/10 animals showing signs, but had decreased to 1/10 by day 12. The peak incidence of arthritis in Group 3M and Group 4M animals receiving BC1054 LS-RO once or thrice daily was on day 7 (9/10 and 7/10 animals, respectively), and this had decreased to 4/10 mice in both groups by day 12. The arthritis incidence peaked on day 6 in the Group 5M animals receiving BC1054 LS-MA with 8/10 animals affected, and the incidence fluctuated with between 6 and 8 animals scoring until the end of the study. By day 6, 9/10 animals presented with arthritis in Group 6M animals receiving BC1054 SF-TO, but this also fluctuated and ended at 7/10 on day 12. In the Ibuprofen-treated Group 7M, the peak of arthritis incidence was recorded on day 6 with 8/10 animals affected, and this remained relatively constant until the study termination. Group 8M mice receiving a BC1054 LS-GE exhibited peak incidence on day 6 with 9/10 animals presenting with signs of arthritis, but this had decreased to 4/10 by day 12.

Clinical signs associated with LPS-administration developed in all groups following the LPS boost on day 3. These had disappeared in all groups by day 12. No mortalities occurred during this study or significant differences in body weight between the vehicle-treated group and test item-treated groups.

The results of mean paw thickness are given in Table 7. Mean rear paw thickness in Group 1M animals (vehicle-treated) was 1.72 ± 0.01 on day 0. Thickness increased and peaked on day 9 at 2.33 ± 0.15, and ended at 2.17 ± 0.11 on day 12. In Etanercept-treated Group 2M mice, mean rear paw thickness began at 1.70 ± 0.02 on day 0. This increased, peaking at 1.96 ± 0.05 on day 6 before decreasing back to 1.77 ± 0.02 on day 12. Etanercept treatment resulted in significantly decreased paw volume compared to the positive control mice on days 9, 10 and 12. In Group 3M, which received BC1054 LS-RO once daily, rear paw thickness was 1.71 ± 0.02 on day 0. By day 7 the swelling in this group had peaked at 1.96 ± 0.05 where it remained relatively constant thereafter. There were significant reductions in the mean paw swelling on days 6 and 9 following administration of BC1054 LS-RO. The mean rear paw thickness in Group 4M, which received BC1054 LS-RO thrice daily, increased to 1.97 ± 0.08 on day 10 (from 1.70 ± 0.03 on day 0), from day 10 paw volumes remained relative constant till the end of the study. BC1054 LS-RO thrice daily resulted in significantly reduced mean paw thickness compared to vehicle-treated mice (Group 1M) on days 6, 7 and 9. Group 5M mice treated with BC1054 LS-MA had peak paw volume at day 7 (1.97 ± 0.05 from 1.69 ± 0.02 on day 0), this group had significantly reduced measurements on day 6 and day 9 when compared to the vehicle treated Group 1M animals. In Group 6M animals treated with BC1054 LS-TO, mean rear paw thickness began at 1.74 ± 0.01 on day 0. This increased to a peak of 2.05 ± 0.10 on day 7 before decreasing back to 1.94 ± 0.06 on day 12. No significant differences were recorded between BC1054 LS-TO-treated animals (Group 6M) and those treated with vehicle control (Group 1M). In the group which received Ibuprofen (Group 7M), rear paw thickness was 1.71 ± 0.02 on day 0. By day 7 the swelling in this group had peaked at 2.15 ± 0.10 before decreasing to 2.02 ±0.08 on day 12. No significant differences were observed when this group was compared to the vehicle control Group 1M. Group 8M animals treated with BC1054 LS-GE exhibited a slight increase in rear paw thickness from 1.72 ± 0.02 on day 0 to 1.85 ± 0.06 on day 7, this remained relatively constant finishing at 1.77 ± 0.03 on day 12. Administration of BC1054 LS-GE resulted in significantly reduced paw swelling in animals (Group 8M) compared to vehicle controls (Group 1M) on days 6, 7, 9, 10 and 12.

| **Table 7. Mean Rear Paw Thickness** | | | | | | | | | | | |
|---|---|---|---|---|---|---|---|---|---|---|---|
| **Group** | **N** | **Treatment** | **Mean Rear Paw Thickness (mm)** | | | | | | | | |
| | | | **0** | **3** | **4** | **5** | **6** | **7** | **9** | **10** | **12** |
| 1M | 10 | Vehicle | 1.72±0.01 | 1.72±0.02 | 1.63±0.02 | 1.69±0.02 | 2.03±0.09 | 2.26±0.13 | 2.33±0.15 | 2.32±0.15 | 2.17±0.11 |
| 2M | 10 | Etanercept | 1.70±0.02 | 1.73±0.03 | 1.64±0.02 | 1.63±0.02 | 1.96±0.05 | 1.95±0.04 | 1.88±0.03* | 1.80±0.04* | 1.77±0.02* |
| 3M | 10 | BC1054 LF-RO | 1.71±0.02 | 1.75±0.02 | 1.68±0.02 | 1.71±0.03 | 1.78±0.03* | 1.96±0.05 | 1.91±0.08* | 1.94±0.10 | 1.90±0.08 |
| 4M | 10 | BC1054 LF-RO | 1.70±0.03 | 1.70±0.01 | 1.61±0.02 | 1.65±0.03 | 1.75±0.03* | 1.89±0.07* | 1.91±0.09* | 1.97±0.08 | 1.93±0.07 |
| 5M | 10 | BC1054 LF-MA | 1.69±0.02 | 1.73±0.02 | 1.66±0.02 | 1.67±0.02 | 1.79±0.04* | 1.97±0.05 | 1.92±0.06* | 1.96±0.07 | 1.91±0.06 |
| 6M | 10 | BC1054 SF-TO | 1.74±0.01 | 1.72±0.03 | 1.64±0.01 | 1.70±0.03 | 1.91±0.06 | 2.05±0.10 | 1.96±0.09 | 1.99±0.07 | 1.94±0.06 |
| 7M | 10 | Ibuprofen | 1.71±0.02 | 1.72±0.01 | 1.64±0.02 | 1.71±0.02 | 1.90±0.05 | 2.15±0.10 | 2.08±0.11 | 2.07±0.11 | 2.02±0.08 |
| 8M | 10 | BC1054 SF-GE | 1.72±0.02 | 1.71±0.02 | 1.63±0.01 | 1.67±0.03 | 1.78±0.03* | 1.85±0.06* | 1.73±0.04* | 1.83±0.03* | 1.77±0.03* |

In view of the findings above, significant anti-arthritic activity was observed in Group 3M animals receiving once daily administration of BC1054 LS-RO, Group 4M animals receiving thrice daily administration of BC1054 LS-RO, Group 5M animals receiving thrice daily administration of BC1054 LS-MA, and Group 8M animals receiving thrice daily administration of BC1054SF-GE.

### Example 21

### Case Studies for the Treatment of Severe Pain

A 51 year old male experienced severe dental pain due to the exposure of a nerve after a tooth filling disintegrated. The pain was perceived to be too severe to be controlled by ibuprofen or diclofenac, but the patient was reluctant to use codeine (30 mg) with paracetamol (500 mg) which was available to him. Prior to the patient coming into to see the dentist for remedial dental work, he took 7 day course of a pharmaceutical composition disclosed herein (BC1054) comprising 20 mg/kg of ibuprofen, 10% ethanol, and 90% rapeseed oil (800 mg b.id), which effectively controlled the pain. Relief of pain occurred within 30 minutes of taking each dose and lasted for approximately 12 hours before redosing. The pain control was so good that he was no longer aware of pain in the affected tooth.

A 50 year old male was diagnosed with a maison neuve fracture in the ankle after a sport injury. The patient was initially administered 30 mg codeine with 500 mg paracetamol bid, along with 10 mg diclofenac tid for 8 months to control his severe pain. After experiencing unacceptable side effects he ceased opiate paracetamol and diclofenac therapy and commenced a 5 day course of a pharmaceutical composition disclosed herein (BC1054) comprising 20 mg/kg of ibuprofen, 10% ethanol, and 90% rapeseed oil (600 mg bid). After 2 days reported a significant improvement in his pain, and then after 3 days he reported that the pain was completely controlled. After a 2 month follow the patient is still free of severe pain and he has since resumed an active sporting life.

### Example 22

### Treatment of a Severe Pain Condition

A 62 year old female complains of severe lower back pain after lifting a heavy box the day before. A physician determines that the lower pain is due to an acute pain. The woman is treated by oral administration a pharmaceutical composition comprising ibuprofen as disclosed herein taken twice daily. Alternatively, the woman is treated by oral administration a pharmaceutical composition comprising aspirin as disclosed herein taken thrice daily. Alternatively, the woman is treated by oral administration a pharmaceutical composition comprising naproxen as disclosed herein taken twice daily. The woman's condition is monitored and after 3 days of treatment the woman indicates there is reduced pain. At one and three week check-ups, the woman indicates that she continues to have reduced pain. This reduction in acute pain symptoms indicates successful treatment with the pharmaceutical composition disclosed herein. In a similar manner, any of the therapeutic compounds such as, e.g., a salicylate derivative NSAID, a p-amino phenol derivative NSAID, a propionic acid derivative NSAID, an acetic acid derivative NSAID, an enolic acid derivative NSAID, a fenamic acid derivative NSAID, a non-selective cyclo-oxygenase (COX) inhibitor, a selective cyclooxygenase 1 (COX 1) inhibitor, a selective cyclooxygenase 2 (COX 2) inhibitor, or a fibrate, will be formulated into a pharmaceutical composition and administered to the patient as described above.

A 22 year old male complains of severe pain in his right shoulder that occurred while he was lifting weights in the gym one month ago. A physician determines that the severe pain is a subacute pain. The man is treated by oral administration a pharmaceutical composition comprising ibuprofen as disclosed herein taken twice daily. Alternatively, the man is treated by oral administration a pharmaceutical composition comprising aspirin as disclosed herein taken thrice daily. Alternatively, the man is treated by oral administration a pharmaceutical composition comprising naproxen as disclosed herein taken twice daily. The man's condition is monitored and after 3 days of treatment the man indicates there is improvement in his ability to move his arm without pain in his shoulder. At one week and one and three month check-ups, the man indicates that he continues to have improved shoulder mobility and no pain. This reduction in subacute pain symptoms indicates successful treatment with the pharmaceutical composition disclosed herein. In a similar manner, any of the therapeutic compounds such as, *e.g.,* a salicylate derivative NSAID, a p-amino phenol derivative NSAID, a propionic acid derivative NSAID, an acetic acid derivative NSAID, an enolic acid derivative NSAID, a fenamic acid derivative NSAID, a non-selective cyclo-oxygenase (COX) inhibitor, a selective cyclooxygenase 1 (COX 1) inhibitor, a selective cyclooxygenase 2 (COX 2) inhibitor, or a fibrate, will be formulated into a pharmaceutical composition and administered to the patient as described above.

A 67 year old male complains of severe pain in his ankle from a fall he took two months before. A physician determines that the pain is a chronic pain. The man is treated by oral administration a pharmaceutical composition comprising ibuprofen as disclosed herein taken twice daily. Alternatively, the man is treated by oral administration a pharmaceutical composition comprising aspirin as disclosed herein taken thrice daily. Alternatively, the man is treated by oral administration a pharmaceutical composition comprising naproxen as disclosed herein taken twice daily. The man's condition is monitored and after 3 days of treatment the man indicates there is reduced pain in his ankle and mobility in his ankle is better. At one and three month check-ups, the man indicates that he continues to have improved ankle mobility and no pain. This reduction in chronic pain symptoms indicates successful treatment with the pharmaceutical composition disclosed herein. In a similar manner, any of the therapeutic compounds such as, e.g., a salicylate derivative NSAID, a p-amino phenol derivative NSAID, a propionic acid derivative NSAID, an acetic acid derivative NSAID, an enolic acid derivative NSAID, a fenamic acid derivative NSAID, a non-selective cyclo-oxygenase (COX) inhibitor, a selective cyclooxygenase 1 (COX 1) inhibitor, a selective cyclooxygenase 2 (COX 2) inhibitor, or a fibrate, will be formulated into a pharmaceutical composition and administered to the patient as described above.

A 73 year old female complains of severe pain after burning her forearm on a hot oven. A physician determines that the pain is due to superficial somatic noceceptive pain. The woman is treated by oral administration a pharmaceutical composition comprising ibuprofen as disclosed herein taken twice daily. Alternatively, the woman is treated by oral administration a pharmaceutical composition comprising aspirin as disclosed herein taken thrice daily. Alternatively, the woman is treated by oral administration a pharmaceutical composition comprising naproxen as disclosed herein taken twice daily. The woman's condition is monitored and after 3 days of treatment the woman indicates that she no longer feels pain in her forearm. At one and three week check-ups, the woman indicates that she still feels no pain. This reduction in superficial somatic noceceptive pain symptoms indicates successful treatment with the pharmaceutical composition disclosed herein. A similar type of oral administration of a pharmaceutical composition disclosed herein will be used to treat a patient suffering from severe pain associated with any other deep somatic noceceptive pain, including, without limitation, an excessive muscle tension, a repetitive motion disorder, a muscle disorder, a myalgia, an infection, and a drug-induced pain. In a similar manner, any of the therapeutic compounds such as, *e.g.,* a salicylate derivative NSAID, a p-amino phenol derivative NSAID, a propionic acid derivative NSAID, an acetic acid derivative NSAID, an enolic acid derivative NSAID, a fenamic acid derivative NSAID, a non-selective cyclo-oxygenase (COX) inhibitor, a selective cyclooxygenase 1 (COX 1) inhibitor, a selective cyclooxygenase 2 (COX 2) inhibitor, or a fibrate, will be formulated into a pharmaceutical composition and administered to the patient as described above.

A 37 year old male complains of severe pain due to a lower leg bone fracture while skiing. A physician determines that the pain is due to deep somatic noceceptive pain. The man is treated by oral administration a pharmaceutical composition comprising ibuprofen as disclosed herein taken twice daily. Alternatively, the man is treated by oral administration a pharmaceutical composition comprising aspirin as disclosed herein taken thrice daily. Alternatively, the man is treated by oral administration a pharmaceutical composition comprising naproxen as disclosed herein taken twice daily. The man's condition is monitored and after 3 days of treatment the man indicates there is reduced pain in his leg. At one and two month check-ups, the man indicates that he continues to have reduced pain. This reduction in deep somatic noceceptive pain symptoms indicates successful treatment with the pharmaceutical composition disclosed herein. A similar type of oral administration of a pharmaceutical composition disclosed herein will be used to treat a patient suffering from severe pain associated with any other deep somatic noceceptive pain, including, without limitation, an excessive muscle tension, a repetitive motion disorder, a muscle disorder, a myalgia, an infection, and a drug-induced pain. In a similar manner, any of the therapeutic compounds such as, *e.g.,* a salicylate derivative NSAID, a p-amino phenol derivative NSAID, a propionic acid derivative NSAID, an acetic acid derivative NSAID, an enolic acid derivative NSAID, a fenamic acid derivative NSAID, a non-selective cyclo-oxygenase (COX) inhibitor, a selective cyclooxygenase 1 (COX 1) inhibitor, a selective cyclooxygenase 2 (COX 2) inhibitor, or a fibrate, will be formulated into a pharmaceutical composition and administered to the patient as described above.

A 33 year old female complains of chronic abdominal pain. A physician determines that the pain is due to deep visceral noceceptive pain. The woman is treated by oral administration a pharmaceutical composition comprising ibuprofen as disclosed herein taken twice daily. Alternatively, the woman is treated by oral administration a pharmaceutical composition comprising aspirin as disclosed herein taken thrice daily. Alternatively, the woman is treated by oral administration a pharmaceutical composition comprising naproxen as disclosed herein taken twice daily. The woman's condition is monitored and after 3 days of treatment the woman indicates that there is a reduction in abdominal pain. At one and three month check-ups, the woman indicates that she continues to have reduced abdominal pain. This reduction in deep visceral noceceptive pain symptoms indicates successful treatment with the pharmaceutical composition disclosed herein. A similar type of oral administration of a pharmaceutical composition disclosed herein will be used to treat a patient suffering from severe pain associated with any other deep visceral noceceptive pain, including, without limitation, a functional visceral pain, a chronic gastrointestinal inflammation, an autoimmune pain, an organic visceral pain, and a treatment-induced visceral pain. In a similar manner, any of the therapeutic compounds such as, *e.g.,* a salicylate derivative NSAID, a p-amino phenol derivative NSAID, a propionic acid derivative NSAID, an acetic acid derivative NSAID, an enolic acid derivative NSAID, a fenamic acid derivative NSAID, a non-selective cyclo-oxygenase (COX) inhibitor, a selective cyclooxygenase 1 (COX 1) inhibitor, a selective cyclooxygenase 2 (COX 2) inhibitor, or a fibrate, will be formulated into a pharmaceutical composition and administered to the patient as described above.

A 66 year old male complains of severe pain after suffering a stroke. A physician determines that the pain is due to central neuropathic pain. The man is treated by oral administration a pharmaceutical composition comprising ibuprofen as disclosed herein taken twice daily. Alternatively, the man is treated by oral administration a pharmaceutical composition comprising aspirin as disclosed herein taken thrice daily. Alternatively, the man is treated by oral administration a pharmaceutical composition comprising naproxen as disclosed herein taken twice daily. The man's condition is monitored and after 3 days of treatment the man indicates that there is a reduction in abdominal pain. At one and three month check-ups, the man indicates that he continues to have reduced pain. This reduction in central neuropathic pain symptoms indicates successful treatment with the pharmaceutical composition disclosed herein. A similar type of oral administration of a pharmaceutical composition disclosed herein will be used to treat a patient suffering from severe pain associated with any other central neuropathic or dysfunctional pain, including, without limitation, cerebral venous thrombosis, cerebral tumors or abscesses compressing a brain portion, traumatic brain or spinal cord injury, complications following brain or spinal surgery, multiple sclerosis, and Parkinson disease, ischemic lesions, syringomyelia, radiation myelopathy, and HIV myelopathy. In a similar manner, any of the therapeutic compounds such as, *e.g.,* a salicylate derivative NSAID, a p-amino phenol derivative NSAID, a propionic acid derivative NSAID, an acetic acid derivative NSAID, an enolic acid derivative NSAID, a fenamic acid derivative NSAID, a non-selective cyclo-oxygenase (COX) inhibitor, a selective cyclooxygenase 1 (COX 1) inhibitor, a selective cyclooxygenase 2 (COX 2) inhibitor, or a fibrate, will be formulated into a pharmaceutical composition and administered to the patient as described above.

A 58 year old male, who is a diabetic, complains of severe pain. A physician determines that the pain is due to peripheral neuropathic pain from diabetic neuropathy. The man is treated by oral administration a pharmaceutical composition comprising ibuprofen as disclosed herein taken twice daily. Alternatively, the man is treated by oral administration a pharmaceutical composition comprising aspirin as disclosed herein taken thrice daily. Alternatively, the man is treated by oral administration a pharmaceutical composition comprising naproxen as disclosed herein taken twice daily. The man's condition is monitored and after 3 days of treatment the man indicates there is reduction in the pain. At one and three month check-ups, the man indicates that he still experiences reduced pain. This reduction in a peripheral neuropathic pain indicates successful treatment with the pharmaceutical composition disclosed herein. A similar type of oral administration of a pharmaceutical composition disclosed herein will be used to treat a patient suffering from severe pain associated with any other peripheral neuropathic or dysfunctional pain, including, without limitation, systemic diseases, metabolic disorders, nutrient disorders, drug-induced disorders, traumatic and entrapment syndromes, complications following surgery, distal axonopathy, HIV sensory neuropathy, demyelinating polyradiculoneuropathy, postherpetic neuralgia, nerve root avulsions, cranial neuralgias like trigeminal neuralgia, neuropathic cancer pain, compression of peripheral nerves, nerve plexuses, and nerve roots, paraneoplastic peripheral neuropathy, ganglionopathy, complication of cancer therapies like chemotherapy, radiation, and surgery, and complex regional pain syndrome like Type 1 and Type 2. In a similar manner, any of the therapeutic compounds such as, *e.g.,* a salicylate derivative NSAID, a p-amino phenol derivative NSAID, a propionic acid derivative NSAID, an acetic acid derivative NSAID, an enolic acid derivative NSAID, a fenamic acid derivative NSAID, a non-selective cyclo-oxygenase (COX) inhibitor, a selective cyclooxygenase 1 (COX 1) inhibitor, a selective cyclooxygenase 2 (COX 2) inhibitor, or a fibrate, will be formulated into a pharmaceutical composition and administered to the patient as described above.

A 59 year old female complains of severe pain by even the faintest amount of pressure is applied to her forearm. A physician determines that the pain is due to allodynia. The woman is treated by topical administration a pharmaceutical composition comprising ibuprofen as disclosed herein taken twice daily. Alternatively, the woman is treated by topical administration a pharmaceutical composition comprising aspirin as disclosed herein taken thrice daily. Alternatively, the woman is treated by topical administration a pharmaceutical composition comprising naproxen as disclosed herein taken twice daily. The woman's condition is monitored and after 3 days of treatment the woman indicates there is reduced pain. At one and three month check-ups, the woman indicates that she continues to have reduced pain. This reduction in allodynia symptoms indicates successful treatment with the pharmaceutical composition disclosed herein. A similar type of topical administration of a pharmaceutical composition disclosed herein will be used to treat a patient suffering from severe pain associated with any dysesthesia, such as, *e.g.,* hyperalgesia or hyperpathia. In a similar manner, any of the therapeutic compounds such as, *e.g.,* a salicylate derivative NSAID, a p-amino phenol derivative NSAID, a propionic acid derivative NSAID, an acetic acid derivative NSAID, an enolic acid derivative NSAID, a fenamic acid derivative NSAID, a non-selective cyclo-oxygenase (COX) inhibitor, a selective cyclooxygenase 1 (COX 1) inhibitor, a selective cyclooxygenase 2 (COX 2) inhibitor, or a fibrate, will be formulated into a pharmaceutical composition and administered to the patient as described above.

A 47 year old female complains of severe pain down her left leg when she leans over to do the dishes. A physician determines that the leg pain is due to a sciatic nerve dysfunction. The woman is treated by oral administration a pharmaceutical composition comprising ibuprofen as disclosed herein taken twice daily. Alternatively, the woman is treated by oral administration a pharmaceutical composition comprising aspirin as disclosed herein taken thrice daily. Alternatively, the woman is treated by oral administration a pharmaceutical composition comprising naproxen as disclosed herein taken twice daily. The woman's condition is monitored and after 3 days of treatment the woman indicates there is reduced pain. At one and three month check-ups, the woman indicates that she continues to have reduced pain in her leg. This reduction in sciatic nerve dysfunction symptoms indicates successful treatment with the pharmaceutical composition disclosed herein. A similar type of topical administration of a pharmaceutical composition disclosed herein will be used to treat a patient suffering from severe pain associated with any mononeuropathy, such as, *e.g.,* a common peroneal nerve dysfunction. a radial nerve dysfunction, an ulnar nerve dysfunction, a cranial mononeuropathy VI, a cranial mononeuropathy VII, a cranial mononeuropathy III (compression type), a cranial mononeuropathy III (diabetic type), an axillary nerve dysfunction, a carpal tunnel syndrome, a femoral nerve dysfunction, a tibial nerve dysfunction, a Bell's palsy, a thoracic outlet syndrome, a carpal tunnel syndrome or other focal entrapment neuropathy, and a sixth (abducent) nerve palsy. In a similar manner, any of the therapeutic compounds such as, *e.g.,* a salicylate derivative NSAID, a p-amino phenol derivative NSAID, a propionic acid derivative NSAID, an acetic acid derivative NSAID, an enolic acid derivative NSAID, a fenamic acid derivative NSAID, a non-selective cyclo-oxygenase (COX) inhibitor, a selective cyclooxygenase 1 (COX 1) inhibitor, a selective cyclooxygenase 2 (COX 2) inhibitor, or a fibrate, will be formulated into a pharmaceutical composition and administered to the patient as described above.

A 22 year old male complains of severe pain in his right leg that began after a hike in the woods. A physician determines that the severe pain is sue to a Lyme disease. The man is treated by intravenous injection administration of a pharmaceutical composition comprising ibuprofen as disclosed herein taken twice daily. Alternatively, the man is treated by intravenous injection administration of a pharmaceutical composition comprising aspirin as disclosed herein taken thrice daily. Alternatively, the man is treated by intravenous injection administration of a pharmaceutical composition comprising naproxen as disclosed herein taken twice daily. The man's condition is monitored and after 3 days of treatment the man indicates there is reduced pain. At one week and one and three month check-ups, the man indicates that he continues to no pain. This reduction in Lyme disease symptoms indicates successful treatment with the pharmaceutical composition disclosed herein. A similar type of intravenous injection administration of a pharmaceutical composition disclosed herein will be used to treat a patient suffering from severe pain associated with any mononeuropathic multiplex, such as, *e.g.,* systemic diseases, metabolic disorders, nutrient disorders, drug-induced disorders, traumatic and entrapment syndromes, toxicity, and infections. In a similar manner, any of the therapeutic compounds such as, *e.g.,* a salicylate derivative NSAID, a p-amino phenol derivative NSAID, a propionic acid derivative NSAID, an acetic acid derivative NSAID, an enolic acid derivative NSAID, a fenamic acid derivative NSAID, a non-selective cyclo-oxygenase (COX) inhibitor, a selective cyclooxygenase 1 (COX 1) inhibitor, a selective cyclooxygenase 2 (COX 2) inhibitor, or a fibrate, will be formulated into a pharmaceutical composition and administered to the patient as described above.

A 67 year old male, a chronic alcoholic, complains of severe pain. A physician determines that the pain is a polyneuropathy. The man is treated by oral administration a pharmaceutical composition comprising ibuprofen as disclosed herein taken twice daily. Alternatively, the man is treated by oral administration a pharmaceutical composition comprising aspirin as disclosed herein taken thrice daily. Alternatively, the man is treated by oral administration a pharmaceutical composition comprising naproxen as disclosed herein taken twice daily. The man's condition is monitored and after 3 days of treatment the man indicates there is reduced pain. At one and three month check-ups, the man indicates that he continues to have no pain. This reduction in polyneuropathic pain symptoms indicates successful treatment with the pharmaceutical composition disclosed herein. A similar type of oral administration of a pharmaceutical composition disclosed herein will be used to treat a patient suffering from severe pain associated with any other polyneuropathy, including, without limitation, acute inflammatory demyelinating polyneuropathy, chronic inflammatory demyelinating polyneuropathy, and a genetic metabolic disorder. In a similar manner, any of the therapeutic compounds such as, *e.g.,* a salicylate derivative NSAID, a p-amino phenol derivative NSAID, a propionic acid derivative NSAID, an acetic acid derivative NSAID, an enolic acid derivative NSAID, a fenamic acid derivative NSAID, a non-selective cyclo-oxygenase (COX) inhibitor, a selective cyclooxygenase 1 (COX 1) inhibitor, a selective cyclooxygenase 2 (COX 2) inhibitor, or a fibrate, will be formulated into a pharmaceutical composition and administered to the patient as described above.

A 73 year old female complains of severe pain in her bladder. A physician determines that the pain is due to an autonomic neuropathy. The woman is treated by instillation administration of a pharmaceutical composition comprising ibuprofen as disclosed herein taken twice daily. Alternatively, the woman is treated by oral administration of a pharmaceutical composition comprising aspirin as disclosed herein taken thrice daily. Alternatively, the woman is treated by oral administration of a pharmaceutical composition comprising naproxen as disclosed herein taken twice daily. The woman's condition is monitored and after 3 days of treatment the woman indicates that she no longer feels pain in her bladder. At one and three month check-ups, the woman indicates that she still feels no pain. This reduction in autonomic neuropathy symptoms indicates successful treatment with the pharmaceutical composition disclosed herein. A similar type of oral administration of a pharmaceutical composition disclosed herein will be used to treat a patient suffering from severe pain associated with any other autonomic neuropathy affecting any other internal organ. In a similar manner, any of the therapeutic compounds such as, *e.g.,* a salicylate derivative NSAID, a p-amino phenol derivative NSAID, a propionic acid derivative NSAID, an acetic acid derivative NSAID, an enolic acid derivative NSAID, a fenamic acid derivative NSAID, a non-selective cyclo-oxygenase (COX) inhibitor, a selective cyclooxygenase 1 (COX 1) inhibitor, a selective cyclooxygenase 2 (COX 2) inhibitor, or a fibrate, will be formulated into a pharmaceutical composition and administered to the patient as described above.

A 42 year old male complains of severe pain whenever any pressure is applied to his left side of his face. A physician determines that the pain is due to a trigeminal neuralgia. The man is treated by topical administration of a pharmaceutical composition comprising ibuprofen as disclosed herein taken twice daily. Alternatively, the man is treated by topical administration a pharmaceutical composition comprising aspirin as disclosed herein taken thrice daily. Alternatively, the man is treated by topical administration a pharmaceutical composition comprising naproxen as disclosed herein taken twice daily. The man's condition is monitored and after 3 days of treatment the man indicates there is reduced pain in his face. At one and two month check-ups, the man indicates that he continues to have reduced pain. This reduction in trigeminal neuralgia pain symptoms indicates successful treatment with the pharmaceutical composition disclosed herein. A similar type of topical administration of a pharmaceutical composition disclosed herein will be used to treat a patient suffering from severe pain associated with any other neuralgia, including, without limitation, a glossopharyngeal neuralgia, a post-herpetic neuralgia, a carpal tunnel syndrome, a meralgia paresthetica, a sciatica and an atypical facial pain. In a similar manner, any of the therapeutic compounds such as, *e.g.,* a salicylate derivative NSAID, a p-amino phenol derivative NSAID, a propionic acid derivative NSAID, an acetic acid derivative NSAID, an enolic acid derivative NSAID, a fenamic acid derivative NSAID, a non-selective cyclo-oxygenase (COX) inhibitor, a selective cyclooxygenase 1 (COX 1) inhibitor, a selective cyclooxygenase 2 (COX 2) inhibitor, or a fibrate, will be formulated into a pharmaceutical composition and administered to the patient as described above.

A 54 year old female complains of pain in her left shoulder after suffering a heart-attack. A physician determines that the pain is due to referred pain from a myocardial ischaemia. The woman is treated by oral administration a pharmaceutical composition comprising ibuprofen as disclosed herein taken twice daily. Alternatively, the woman is treated by oral administration a pharmaceutical composition comprising aspirin as disclosed herein taken thrice daily. Alternatively, the woman is treated by oral administration a pharmaceutical composition comprising naproxen as disclosed herein taken twice daily. The woman's condition is monitored and after 3 days of treatment the woman indicates that there is a reduction in shoulder pain. At one and three month check-ups, the woman indicates that she continues to have reduced shoulder pain. This reduction in referred pain symptoms indicates successful treatment with the pharmaceutical composition disclosed herein. A similar type of oral administration of a pharmaceutical composition disclosed herein will be used to treat a patient suffering from severe pain associated with any other referred pain, including, without limitation, an intervertebral disc herniation. In a similar manner, any of the therapeutic compounds such as, *e.g.,* a salicylate derivative NSAID, a p-amino phenol derivative NSAID, a propionic acid derivative NSAID, an acetic acid derivative NSAID, an enolic acid derivative NSAID, a fenamic acid derivative NSAID, a non-selective cyclo-oxygenase (COX) inhibitor, a selective cyclooxygenase 1 (COX 1) inhibitor, a selective cyclooxygenase 2 (COX 2) inhibitor, or a fibrate, will be formulated into a pharmaceutical composition and administered to the patient as described above.

A 26 year old male complains of severe pain in the location where is amputated arm once was. A physician determines that the pain is due to phantom pain. The man is treated by oral administration a pharmaceutical composition comprising ibuprofen as disclosed herein taken twice daily. Alternatively, the man is treated by oral administration a pharmaceutical composition comprising aspirin as disclosed herein taken thrice daily. Alternatively, the man is treated by oral administration a pharmaceutical composition comprising naproxen as disclosed herein taken twice daily. The man's condition is monitored and after 3 days of treatment the man indicates that there is a reduction in pain. At one and three month check-ups, the man indicates that he continues to have reduced pain. This reduction in phantom pain symptoms indicates successful treatment with the pharmaceutical composition disclosed herein. A similar type of oral administration of a pharmaceutical composition disclosed herein will be used to treat a patient suffering from severe pain associated with any other deafferentation pain syndrome, including, without limitation, a brain injury, a spinal cord injury, a lumbar radiculopathy, a post-stroke pain, a paraplegia, avulsion of the brachial plexus or other types of lesions of peripheral nerves, a pathology of the central nervous system. In a similar manner, any of the therapeutic compounds such as, *e.g.,* a salicylate derivative NSAID, a p-amino phenol derivative NSAID, a propionic acid derivative NSAID, an acetic acid derivative NSAID, an enolic acid derivative NSAID, a fenamic acid derivative NSAID, a non-selective cyclo-oxygenase (COX) inhibitor, a selective cyclooxygenase 1 (COX 1) inhibitor, a selective cyclooxygenase 2 (COX 2) inhibitor, or a fibrate, will be formulated into a pharmaceutical composition and administered to the patient as described above.

A 58 year old male complains of a headache. The man is treated by oral administration of a pharmaceutical composition comprising ibuprofen as disclosed herein taken twice daily. Alternatively, the man is treated by oral administration of a pharmaceutical composition comprising aspirin as disclosed herein taken thrice daily. Alternatively, the man is treated by oral administration of a pharmaceutical composition comprising naproxen as disclosed herein taken twice daily. The man's condition is monitored and after 3 hours the headache pain is gone. This elimination in headache pain indicates successful treatment with the pharmaceutical composition disclosed herein. A similar type of oral administration of a pharmaceutical composition disclosed herein will be used to treat a patient suffering from severe pain associated with any other headache pain, including, without limitation, a muscular/myogenic headache, a vascular headache, a traction headache, inflammatory headache, a chronic sinusitis headache, a hormone headache, a rebound headache, an organic headache, and an ictal headache. In a similar manner, any of the therapeutic compounds such as, *e.g.,* a salicylate derivative NSAID, a p-amino phenol derivative NSAID, a propionic acid derivative NSAID, an acetic acid derivative NSAID, an enolic acid derivative NSAID, a fenamic acid derivative NSAID, a non-selective cyclo-oxygenase (COX) inhibitor, a selective cyclooxygenase 1 (COX 1) inhibitor, a selective cyclooxygenase 2 (COX 2) inhibitor, or a fibrate, will be formulated into a pharmaceutical composition and administered to the patient as described above.

A 59 year old female complains of severe headache pain. A physician determines that the pain is due to migraine. The woman is treated by oral administration of a pharmaceutical composition comprising ibuprofen as disclosed herein taken twice daily. Alternatively, the woman is treated by topical administration of a pharmaceutical composition comprising aspirin as disclosed herein taken thrice daily. Alternatively, the woman is treated by topical administration of a pharmaceutical composition comprising naproxen as disclosed herein taken twice daily. The woman's condition is monitored and after 3 days of treatment the woman indicates there is no migraine pain reoccurrence. At one and three month check-ups, the woman indicates that she continues to have reduced frequency and intensity of migraine headache pain. This reduction in migraine symptoms indicates successful treatment with the pharmaceutical composition disclosed herein. A similar type of oral administration of a pharmaceutical composition disclosed herein will be used to treat a patient suffering from severe pain associated with any other migraine headache pain, including, without limitation, a migraine without aura (common migraine), a migraine with aura (classic migraine), a menstrual migraine, a migraine equivalent (acephalic headache), a complicated migraine, an abdominal migraine and a mixed tension migraine. In a similar manner, any of the therapeutic compounds such as, *e.g.,* a salicylate derivative NSAID, a p-amino phenol derivative NSAID, a propionic acid derivative NSAID, an acetic acid derivative NSAID, an enolic acid derivative NSAID, a fenamic acid derivative NSAID, a non-selective cyclo-oxygenase (COX) inhibitor, a selective cyclooxygenase 1 (COX 1) inhibitor, a selective cyclooxygenase 2 (COX 2) inhibitor, or a fibrate, will be formulated into a pharmaceutical composition and administered to the patient as described above.

Certain embodiments of the present invention are described herein, including the best mode known to the inventors for carrying out the invention. Of course, variations on these described embodiments will become apparent to those of ordinary skill in the art upon reading the foregoing description. The inventor expects skilled artisans to employ such variations as appropriate, and the inventors intend for the present invention to be practiced otherwise than specifically described herein. Accordingly, this invention includes all modifications and equivalents of the subject matter recited in the claims appended hereto as permitted by applicable law. Moreover, any combination of the above-described embodiments in all possible variations thereof is encompassed by the invention unless otherwise indicated herein or otherwise clearly contradicted by context.

Groupings of alternative embodiments, elements, or steps of the present invention are not to be construed as limitations. Each group member may be referred to and claimed individually or in any combination with other group members disclosed herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Unless indicated to the contrary, the numerical parameters set forth in the specification and attached claims are approximations that may vary. At the very least, and not as an attempt to limit the application of the doctrine of equivalents to the scope of the claims, each numerical indication should at least be construed in light of the number of reported significant digits and by applying ordinary rounding techniques. Notwithstanding that the numerical ranges and values setting forth the broad scope of the invention are approximations, the numerical ranges and values set forth in the specific examples are reported as precisely as possible. Any numerical range or value, however, inherently contains certain errors necessarily resulting from the standard deviation found in their respective testing measurements. Recitation of numerical ranges of values herein is merely intended to serve as a shorthand method of referring individually to each separate numerical value falling within the range. Unless otherwise indicated herein, each individual value of a numerical range is incorporated into the present specification as if it were individually recited herein.

The terms "a," "an," "the" and similar referents used in the context of describing the present invention (especially in the context of the following claims) are to be construed to cover both the singular and the plural, unless otherwise indicated herein or clearly contradicted by context. All methods described herein can be performed in any suitable order unless otherwise indicated herein or otherwise clearly contradicted by context. The use of any and all examples, or exemplary language (e.g., "such as") provided herein is intended merely to better illuminate the present invention and does not pose a limitation on the scope of the invention otherwise claimed. No language in the present specification should be construed as indicating any non-claimed element essential to the practice of the invention.

Specific embodiments disclosed herein may be further limited in the claims using consisting of or consisting essentially of language. When used in the claims, whether as filed or added per amendment, the transition term "consisting of" excludes any element, step, or ingredient not specified in the claims. The transition term "consisting essentially of' limits the scope of a claim to the specified materials or steps and those that do not materially affect the basic and novel characteristic(s). Embodiments of the present invention so claimed are inherently or expressly described and enabled herein.

## Claims

1. A solid solution pharmaceutical composition comprising: a) one or more therapeutic compounds having an anti-pain activity comprising 5% to 55% by weight of the pharmaceutical composition,
wherein when the one or more therapeutic compounds is a therapeutic compound having a free acid or base with a logP of 2.2 to 3.0, one or more stabilizing agents comprising a liquid glycol polymer are present,
wherein when the one or more therapeutic compounds is a therapeutic compound salt with a logP of 2.2 or less, one or more neutralizing agents are present;
b) one or more lipids that are solid at 20°C comprising 30% to 75% by weight of the pharmaceutical composition, wherein the one or more lipids have a melting point of 40°C to 50°C, and wherein the lipids comprise a triglyceride with one saturated or unsaturated fatty acid having a carbon length of C₁₂-C₂₄, two saturated or unsaturated fatty acids each having a carbon length of C₁₂-C₂₄, or three saturated or unsaturated fatty acids each having a carbon length of C₁₂-C₂₄;
c) one or more lipids that are liquid at 20°C comprising 1% to 20% by weight of the pharmaceutical composition, wherein the solid solution pharmaceutical composition has a melting point of 30°C or higher.

2. The solid solution pharmaceutical composition according to Claim 1, wherein the one or more therapeutic compound is 20% to 30% by weight of the pharmaceutical composition.

3. The solid solution pharmaceutical composition according to Claim 1 or Claim 2, wherein the one or more lipids that are solid at 20°C is 35% to 45% by weight of the pharmaceutical composition.

4. The solid solution pharmaceutical composition according to any one of Claims 1-3, wherein the one or more lipids that are liquid at 20°C is 1% to 10% by weight of the pharmaceutical composition.

5. The solid solution pharmaceutical composition according to any one of Claims 1-4, wherein the one or more lipids that are liquid at 20°C comprises one or more partially hydrogenated fats.

6. The solid solution pharmaceutical composition according to any one of Claims 1-4, wherein the one or more lipids that are liquid at 20°C comprises one or more monoglycerides,

7. The solid solution pharmaceutical composition according to Claim 6, wherein the one or more monoglycerides comprises glycerol monolinoleate.

8. The solid solution pharmaceutical composition according to any one of Claims 1-7, wherein the solid solution pharmaceutical composition further comprises one or more stabilizing agents and/or one or more neutralizing agents.

9. The solid solution pharmaceutical composition according to Claim 8, wherein the one or more stabilizing agents comprises 1% to 5% by weight of the pharmaceutical composition.

10. The solid solution pharmaceutical composition according to Claim 9, wherein the one or more stabilizing agents comprises a liquid glycol polymer, a monohydric alcohol, isosorbide dimethyl ether, and diethylene glycol monoethyl ether (2-(2-ethoxyethoxy)ethanol).

11. The solid solution pharmaceutical composition according to Claim 8, wherein the one or more neutralizing agents comprises 5% to 55% by weight of the pharmaceutical composition.

12. The solid solution pharmaceutical composition according to Claim 11, wherein the one or more neutralizing agents comprises one or more fatty acids, sodium acetate, or triethanolamine.

13. The solid solution pharmaceutical composition according to any one of claims 1-12 for use in treating a severe pain condition.

14. The solid solution pharmaceutical composition for use according to claim 13, wherein the severe pain condition is an acute pain, a subacute pain, or a chronic pain, or wherein the severe pain condition is a nociceptive pain, a pathological pain, a referred pain, a headache or a migraine.

## Patentansprüche

1. Feste pharmazeutische Lösungszusammensetzung, umfassend: a) eine oder mehrere therapeutische Verbindungen, die eine Anti-Schmerz-Aktivität aufweisen und 5 % bis 55 % nach Gewicht der pharmazeutischen Zusammensetzung umfassen,
wobei, wenn die eine oder mehreren therapeutischen Verbindungen eine therapeutische Verbindung mit einer freien Säure oder Base mit einem logP von 2,2 bis 3,0 ist, ein oder mehrere Stabilisierungsmittel umfassend ein flüssiges Glycolpolymer vorliegen,
wobei, wenn die eine oder mehreren therapeutischen Verbindungen ein therapeutisches Verbindungssalz mit einem logP von 2,2 oder weniger ist, ein oder mehrere Neutralisierungsmittel vorliegen;
b) ein oder mehrere Lipide, die bei 20 °C fest sind, umfassend 30 % bis 75 % nach Gewicht der pharmazeutischen Zusammensetzung, wobei das eine oder mehrere Lipide einen Schmelzpunkt von 40 °C bis 50°C aufweisen und wobei die Lipide ein Triglycerid mit einer gesättigten oder ungesättigten Fettsäure mit einer Kohlenstofflänge von C₁₂-C₂₄, zwei gesättigte oder ungesättigte Fettsäuren jeweils mit einer Kohlenstofflänge von C₁₂-C₂₄ oder drei gesättigte oder ungesättigte Fettsäuren jeweils mit einer Kohlenstofflänge von C₁₂-C₂₄ umfassen;
c) ein oder mehrere Lipide, die bei 20 °C flüssig sind, umfassend 1 % bis 20 % nach Gewicht der pharmazeutischen Zusammensetzung, wobei die feste pharmazeutische Lösungszusammensetzung einen Schmelzpunkt von 30°C oder höher aufweist.

2. Feste pharmazeutische Lösungszusammensetzung nach Anspruch 1, wobei die eine oder mehreren therapeutische Verbindungen 20 % bis 30 % nach Gewicht der pharmazeutischen Zusammensetzung beträgt.

3. Feste pharmazeutische Lösungszusammensetzung nach Anspruch 1 oder Anspruch 2, wobei das eine oder mehrere Lipide, die bei 20°C fest sind, 35 % bis 45 % nach Gewicht der pharmazeutischen Zusammensetzung beträgt.

4. Feste pharmazeutische Lösungszusammensetzung nach einem der Ansprüche 1-3, wobei das eine oder mehrere Lipide, die bei 20°C flüssig sind, 1 % bis 10 % nach Gewicht der pharmazeutischen Zusammensetzung beträgt.

5. Feste pharmazeutische Lösungszusammensetzung nach einem der Ansprüche 1-4, wobei das eine oder mehrere Lipide, die bei 20 °C flüssig sind, ein oder mehrere teilweise hydrierte Fette umfasst.

6. Feste pharmazeutische Lösungszusammensetzung nach einem der Ansprüche 1-4, wobei das eine oder mehrere Lipide, die bei 20 °C flüssig sind, ein oder mehrere Monoglyceride umfasst.

7. Feste pharmazeutische Lösungszusammensetzung nach Anspruch 6, wobei das eine oder mehrere Monoglyceride Glycerolmonolinoleat umfasst.

8. Feste pharmazeutische Lösungszusammensetzung nach einem der Ansprüche 1-7, wobei die feste pharmazeutische Lösungszusammensetzung ferner ein oder mehrere Stabilisierungsmittel und/oder ein oder mehrere Neutralisierungsmittel umfasst.

9. Feste pharmazeutische Lösungszusammensetzung nach Anspruch 8, wobei das eine oder mehrere Stabilisierungsmittel 1 % bis 5 % nach Gewicht der pharmazeutischen Zusammensetzung umfasst.

10. Feste pharmazeutische Lösungszusammensetzung nach Anspruch 9, wobei das eine oder mehrere Stabilisierungsmittel ein flüssiges Glycolpolymer, einen einwertigen Alkohol, Isosorbiddimethylether und Diethylenglycolmonoethylether (2-(2-Ethoxyethoxy)ethanol) umfasst.

11. Feste pharmazeutische Lösungszusammensetzung nach Anspruch 8, wobei das eine oder mehrere Neutralisierungsmittel 5 % bis 55 % nach Gewicht der pharmazeutischen Zusammensetzung umfasst.

12. Feste pharmazeutische Lösungszusammensetzung nach Anspruch 11, wobei das eine oder mehrere Neutralisierungsmittel eine oder mehrere Fettsäuren, Natriumacetat oder Triethanolamin umfasst.

13. Feste pharmazeutische Lösungszusammensetzung nach einem der Ansprüche 1-12 zur Verwendung bei der Behandlung eines schweren Schmerzzustands.

14. Feste pharmazeutische Lösungszusammensetzung zur Verwendung nach Anspruch 13, wobei der schwere Schmerzzustand ein akuter Schmerz, ein subakuter Schmerz oder ein chronischer Schmerz ist oder wobei der schwere Schmerzzustand ein nozizeptiver Schmerz, ein pathologischer Schmerz, ein fortgeleiteter Schmerz, ein Kopfschmerz oder eine Migräne ist.

## Revendications

1. Composition pharmaceutique de solution solide comprenant : a) un ou plusieurs composés thérapeutiques présentant une activité anti-douleur comprenant 5 % à 55 % en poids de la composition pharmaceutique, lorsque lesdits un ou plusieurs composés thérapeutiques sont un composé thérapeutique ayant un acide ou une base libre avec un logP de 2,2 à 3,0, un ou plusieurs agents stabilisants comprenant un polymère de glycol liquide étant présents ; lorsque lesdits un ou plusieurs composés thérapeutiques représentent un sel de composé thérapeutique avec un logP de 2,2 ou moins, un ou plusieurs agents neutralisants étant présents ;
b) un ou plusieurs lipides solides à 20°C comprenant 30 % à 75 % en poids de la composition pharmaceutique, lesdits un ou plusieurs lipides ayant un point de fusion de 40°C à 50°C, et lesdits lipides comprenant un triglycéride avec un acide gras saturé ou insaturé ayant une longueur carbonée de C₁₂ à C₂₄, deux acides gras saturés ou insaturés ayant chacun une longueur carbonée de C₁₂ à C₂₄, ou trois acides gras saturés ou insaturés ayant chacun une longueur carbonée de C₁₂ à C₂₄;
c) un ou plusieurs lipides liquides à 20°C comprenant 1 % à 20 % en poids de la composition pharmaceutique, ladite composition pharmaceutique de solution solide ayant un point de fusion de 30°C ou plus.

2. Composition pharmaceutique de solution solide selon la revendication 1, lesdits un ou plusieurs composés thérapeutiques représentant 20 % à 30 % en poids de la composition pharmaceutique.

3. Composition pharmaceutique de solution solide selon la revendication 1 ou la revendication 2, lesdits un ou plusieurs lipides solides à 20°C représentant 35 % à 45 % en poids de la composition pharmaceutique.

4. Composition pharmaceutique de solution solide selon l'une quelconque des revendications 1 à 3, lesdits un ou plusieurs lipides liquides à 20°C représentant 1 % à 10 % en poids de la composition pharmaceutique.

5. Composition pharmaceutique de solution solide selon l'une quelconque des revendications 1 à 4, lesdits un ou plusieurs lipides liquides à 20°C comprenant une ou plusieurs matières grasses partiellement hydrogénées.

6. Composition pharmaceutique de solution solide selon l'une quelconque des revendications 1 à 4, lesdits un ou plusieurs lipides liquides à 20°C comprenant un ou plusieurs monoglycérides.

7. Composition pharmaceutique de solution solide selon la revendication 6, lesdits un ou plusieurs monoglycérides comprenant le monolinoléate de glycérol.

8. Composition pharmaceutique de solution solide selon l'une quelconque des revendications 1 à 7, ladite composition pharmaceutique de solution solide comprenant en outre un ou plusieurs agents stabilisants et/ou un ou plusieurs agents neutralisants.

9. Composition pharmaceutique de solution solide selon la revendication 8, lesdits un ou plusieurs agents stabilisants représentant 1 % à 5 % en poids de la composition pharmaceutique.

10. Composition pharmaceutique de solution solide selon la revendication 9, lesdits un ou plusieurs agents stabilisants comprenant un polymère de glycol liquide, un alcool monohydrique, l'éther diméthylique d'isosorbide et l'éther monoéthylique de diéthylène glycol (2-(2-éthoxyéthoxy)éthanol).

11. Composition pharmaceutique de solution solide selon la revendication 8, lesdits un ou plusieurs agents neutralisants représentant 5 % à 55 % en poids de la composition pharmaceutique.

12. Composition pharmaceutique de solution solide selon la revendication 11, lesdits un ou plusieurs agents neutralisants comprenant un ou plusieurs acides gras, l'acétate de sodium ou la triéthanolamine.

13. Composition pharmaceutique de solution solide selon l'une quelconque des revendications 1 à 12, pour utilisation dans le traitement d'un état de douleur grave.

14. Composition pharmaceutique de solution solide pour utilisation selon la revendication 13, l'état de douleur grave étant une douleur aiguë, une douleur subaiguë ou une douleur chronique, ou ledit état de douleur grave étant une douleur nociceptive, une douleur pathologique, une douleur projetée, un mal de tête ou une migraine.
